# EUROPEAN PATENT SPECIFICATION

(11) **EP 4 039 812 B1**
(45) Date of publication and mention of the grant of the patent: **11.06.2025**
(21) Application number: 22156322.4
(22) Date of filing: 28.02.2018
(51) Int. Cl.: C12N 15/86, A61K 48/00, A61P 25/00, A61K 38/00, C07K 14/47

(54) **REGENERATING FUNCTIONAL NEURONS FOR TREATMENT OF NEURAL INJURY CAUSED BY DISRUPTION OF BLOOD FLOW**
REGENERIERENDE FUNKTIONELLE NEURONEN ZUR BEHANDLUNG VON DURCH STÖRUNGEN DER DURCHBLUTUNG VERURSACHTEN NEURONALEN VERLETZUNGEN
RÉGÉNÉRATION DE NEURONES FONCTIONNELS POUR LE TRAITEMENT DES LÉSIONS NEURALES CAUSÉES PAR LA PERTURBATION DU FLUX SANGUIN

(30) Priority: 28.02.2017 US 201762464469 P; 13.06.2017 US 201762518914 P
(43) Date of publication of application: 10.08.2022
(62) Divisional of application: 18760296.6
(73) Proprietor: The Penn State Research Foundation, University Park, PA 16802 (US)
(72) Inventor: CHEN, Gong, State College, 16803 (US)
(74) Representative: Greaves Brewster LLP

(56) References cited:
- WO-A1-2019/152857
- WANG ET AL: "DEVELOPING HIPPOCAMPAL DELIVERY OF AAV-NEUROD1 AS A NOVEL THERAPY FOR ALZHEIMER'S DISEASE", THESIS, May 2016 (2016-05-01), Pennsylvania University, XP055629104, Retrieved from the Internet <URL:https://etda.libraries.psu.edu/files/final_submissions/11608> [retrieved on 20191007]
- ZIYUAN GUO ET AL: "In Vivo Direct Reprogramming of Reactive Glial Cells into Functional Neurons after Brain Injury and in an Alzheimer's Disease Model", CELL STEM CELL, vol. 14, no. 2, February 2014 (2014-02-01), AMSTERDAM, NL, pages 188 - 202, XP055387984, ISSN: 1934-5909, DOI: 10.1016/j.stem.2013.12.001
- ANONYMOUS: "Team:Lethbridge/project - 2014.igem.org", 2 July 2015 (2015-07-02), XP055652357, Retrieved from the Internet <URL:https://web.archive.org/web/20150702194117/http://2014.igem.org/Team:Lethbridge/project> [retrieved on 20191212]
- SRIVASTAVA DEEPAK ET AL: "In Vivo Cellular Reprogramming: The Next Generation", CELL, ELSEVIER, AMSTERDAM, NL, vol. 166, no. 6, 8 September 2016 (2016-09-08), pages 1386 - 1396, XP029718850, ISSN: 0092-8674, DOI: 10.1016/J.CELL.2016.08.055
- T. TAKANO ET AL: "Astrocytes and Ischemic Injury", STROKE, vol. 40, no. 3, Supplement 1, March 2009 (2009-03-01), US, pages S8 - S12, XP055299267, ISSN: 0039-2499, DOI: 10.1161/STROKEAHA.108.533166

## Description

This application claims priority to U.S. Provisional Patent Application Serial No. 62/464,469, filed February 28, 2017; and U.S. Provisional Patent Application Serial No. 62/518,914, filed June 13, 2017.

### FIELD

The claimed subject matter relates to an adeno-associated virus expression vector comprising a human GFAP promoter sequence operably linked to a nucleic acid encoding NeuroD1 for use in a method decreasing neuroinflammation after a stroke in a subject in need thereof.

### BACKGROUND

The central nervous system (CNS) in mammals is largely unable to regenerate itself following injury. Neurons and other CNS cells are often killed or injured as a result of a disease or injury which interrupts blood flow in the region where the cells are located. Furthermore, pathological changes in the vicinity of the blood flow interruption are responsible for numerous adverse effects, such as destruction or injury of neurons, destruction or injury of glial cells, destruction or injury of blood vessels, and destruction or injury of supporting cells, in the region of the CNS affected by the disruption of normal blood flow. There is a continuing need for compositions and methods for treating the effects of disruption of normal blood flow in the CNS of an individual subject. Wang et al., Thesis, May 2016 (2016-05), XP055629104, Pennsylvania University (https://etda.libraries.psu.edu/files/final_submissions/11608) discloses developing hippocampal delivery of AAV-NeuroD1 as a novel therapy for Alzheimer's disease but does not disclose an AAV vector expressing NeuroD1 under the control of human GFAP for use in the treatment of neuroinflammation associated with stroke. Ziyuan Guo et al., Cell Stem Cell, Vol. 14, No. 2, February 2014, pages 188-202 discloses *in vivo* direct reprogramming of reactive glial cells into functional neurons after brain injury and in an Alzheimer's disease model, but does not disclose an AAV vector or the treatment of neuroinflammation after a stroke.

### SUMMARY

The claimed subject matter is set out in the appended claims. The references below to the methods of treatment by therapy or surgery or in vivo diagnosis methods are to be interpreted as references to compounds, pharmaceutical compositions and medicaments of the present disclosure for use in those methods.

Provided is a recombinant adeno-associated virus (AAV) expression vector comprising a human GFAP promoter sequence operably linked to a nucleic acid encoding NeuroD1 for use in a method for decreasing neuroinflammation after a stroke in a subject in need thereof, wherein the decrease in neuroinflammation comprises a downregulation in at least one pro-inflammatory factor selected from the group consisting of interferon y, TNFα, interleukin 1β, and interleukin 6, and wherein the AAV expression vector is administered to the central nervous system of the subject.

According to the claimed subject matter, the nucleic acid sequence encoding NeuroD1 is operably linked to a human GFAP promoter sequence.

Methods are provided according to aspects of the present invention which include administering a therapeutically effective dose of exogenous NeuroD1 as set out above and in the appended claims, wherein expressing exogenous NeuroD1 includes delivering an effective "flip-excision" recombinant expression vector combination of 1) a recombinant adeno-associated virus expression vector comprising a nucleic acid encoding NeuroD1 and 2) a recombinant adeno-associated virus expression vector comprising a nucleic acid encoding a site-specific recombinase to the area. Optionally, NeuroD1 is the only exogenously expressed transcription factor delivered to the area.

Methods are provided according to aspects of the present invention which include administering 1-500 µl of a pharmaceutically acceptable carrier containing adeno-associated virus particles comprising a nucleic acid encoding NeuroD1 as set out above and in the appended claims at a concentration of 10¹⁰-10¹⁴ adeno-associated virus particles/ml carrier to the subject, in the area where normal blood flow has been disrupted, at a controlled flow rate of 0.1-5 µl/min.

According to aspects of the present invention, the exogenous NeuroD1 as set out above and in the appended claims is administered at a time following disruption of normal blood flow in the CNS when reactive astrocytes are present.

According to aspects of the present invention, the exogenous NeuroD1 as set out above and in the appended claims is administered at a time following disruption of normal blood flow in the CNS when a glial scar is present.

According to aspects of the present invention, the disruption of normal blood flow in the CNS is due to a disorder selected from the group consisting of ischemic stroke and hemorrhagic stroke.

Methods are provided according to aspects of the present invention which include administering a therapeutically effective dose of exogenous NeuroD1 as set out above and in the appended claims, wherein administering the therapeutically effective dose of NeuroD1 includes administering a recombinant expression vector, the expression vector including a nucleic acid sequence encoding NeuroD1 protein, wherein the nucleic acid sequence encoding NeuroD1 protein includes a nucleic acid sequence selected from the group consisting of: a nucleic acid sequence encoding SEQ ID NO:2 or a functional fragment thereof; a nucleic acid sequence encoding SEQ ID NO:4 or a functional fragment thereof; SEQ ID NO: 1 or a functional fragment thereof; SEQ ID NO:3 or a functional fragment thereof; and a nucleic acid sequence encoding a protein which has 70%, 71%, 72%, 73%, 74%, 75%, 76%, 77%, 78%, 79%, 81%, 82%, 83%, 84%, 85%, 86%,87%, 88%, 89%, 90%, 91%, 92%, 93%, 94%, 95%, 96%, 97%, 98%, 99%, or greater, identity to SEQ ID NO: 2 or SEQ ID NO: 4, or a functional fragment thereof.

Optionally, administering the therapeutically effective dose of NeuroD1 as set out above and in the appended claims includes stereotactic injection in or near a glial scar.

Optionally, methods according to the present invention further include assessing the effectiveness of the treatment in the subject. Optionally, methods according to the present invention further include assessing the effectiveness of the treatment in the subject, wherein assessing the effectiveness of the treatment includes an assay selected from: electrophysiology assay, blood flow assay, tissue structure assay, function assay and a combination of any two or more thereof.

Optionally, methods according to the present invention further include assessing the effectiveness of the treatment in the subject, wherein assessing the effectiveness of the treatment includes an electroencephalogram of the subject.

Optionally, methods according to the present invention further include assessing the effectiveness of the treatment in the subject, wherein assessing the effectiveness of the treatment includes an assay of blood flow selected from the group consisting of: Near Infrared Spectroscopy and fMRI.

Optionally, methods according to the present invention further include assessing the effectiveness of the treatment in the subject, wherein assessing the effectiveness of the treatment includes an assay of tissue structure selected from the group consisting of: MRI, CAT scan, PET scan and ultrasound.

Optionally, methods according to the present invention further include assessing the effectiveness of the treatment in the subject, wherein assessing the effectiveness of the treatment includes a behavioral assay.

Optionally, assessing the effectiveness of the treatment in the subject includes an assay performed prior to administration of the therapeutically effective dose of exogenous NeuroD1. Thus for example, according to aspects of the present invention, methods are provided which include: 1) performing a first assay selected from: an electrophysiology assay, blood flow assay, tissue structure assay, function assay, or a combination of any two or more thereof, on the subject, wherein the first assay is performed prior to administration of a therapeutically effective dose of exogenous NeuroD1; 2) administering a therapeutically effective dose of exogenous NeuroD1 as set out above and in the appended claims and 3) performing a second assay selected from: an electrophysiology assay, blood flow assay, tissue structure assay, function assay, or a combination of any two or more thereof, on the subject, wherein the second assay is performed after administration of a therapeutically effective dose of exogenous NeuroD1.

NeuroD1-mediated astrocyte-to-neuron conversion produced according to methods of the present invention regenerates 100-600 NeuN+ new neurons/mm² in the stroke areas, which is 20-200 times more efficient than the internal neuroregeneration capability.

Accompanying neuroregeneration, NeuroD1-treatment according to aspects of the present invention also decreases reactive astrocytes, reduces neuroinflammation, enhances neuronal survival, repairs blood vessels, and restores blood-brain-barrier (BBB) after stroke. Furthermore, NeuroD1-converted neurons according to aspects of the present invention not only form local neural circuits but also integrate into global brain network, achieving functional rescue of motor deficits induced by ischemic stroke. Thus, NeuroD1-mediated *in vivo* cell conversion according to methods of treatment of the present invention provides unprecedented neuroregenerative efficiency for the treatment of neurological disorders.

Methods of treatment according to aspects of the present invention demonstrate that NeuroD1-mediated *in vivo* glia-to-neuron conversion can functionally rescue motor deficits induced by ischemic stroke. In addition to neuroregeneration after glia conversion, many injured neurons are preserved after reduction of reactive astrocytes and decrease of neuroinflammation. NeuroD1-treatment of the present invention also repairs damaged blood vessels and restores BBB integrity in the stroke areas, leading to a landscape change from neuroinhibitory toward a neuropermissive environment. Methods of treatment according to aspects of the present invention regenerate and preserve over 50-80% of the total neurons after injury resulting from disruption of normal blood flow in the CNS.

### High Efficiency of Neuroregeneration Through In vivo Cell Conversion

Brain function relies upon a delicate balance between neurons and their surrounding glial cells. After severe ischemic injury, neurons die either immediately or gradually due to the secondary injury, but their neighboring glial cells can be activated and start to proliferate, resulting in glial scar tissue that eventually inhibits neuroregeneration. The NeuroD 1-mediated *in vivo* cell conversion technology described herein restores neuronal functions in the injured areas resulting from disruption of normal blood flow in the CNS by directly converting reactive glial cells into functional neurons. The *in vivo* glia-to-neuron conversion technology of the present invention described herein has a number of advantages compared to "classic" administration of exogenous stem cells for engraftment and attempt to treat stroke.

One advantage is the use endogenous glial cells instead of external cells for neuroregeneration, avoiding immunorejection associated with cell transplantation. Using endogenous glial cells that are nearby the lost neurons for regeneration is perhaps the most economical way to restore neuron functions in a local circuit. Another advantage is that the conversion of dividing reactive glial cells into non-dividing neurons not only reduces the number of reactive glial cells but also decreases the potential tumor risk associated with dividing glial cells. In contrast, "classic" stem cell therapy characterized by administration of exogenous stem cells is intrinsically linked to tumor risk. Thus, no exogenous stem cells are administered in methods according to aspects of the present invention.

In addition, the NeuroD1-mediated glia-to-neuron conversion therapy described herein can regenerate a large number of new neurons in areas characterized by disruption of normal blood flow in the CNS, such as stroke areas, averaging 400 NeuN+ cells/mm², which is about 100 times of the internal neurogenesis capability in the adult mouse cortex or striatum after ischemic stroke.

Such remarkable neuroregeneration efficacy is also unmatchable by the typically low efficiency of neuroregeneration after exogenous stem cell engraftment. Without being bound by theory, efficient neuroregeneration is critical for functional repair because small numbers of new neurons either fail to survive in an injury environment or are insufficient to rebuild neural circuits.

In contrast to modulating endogenous neural stem cells, the *in vivo* cell conversion approach described herein makes use of reactive glial cells that are closely associated with neural injury throughout the nervous system for in situ regeneration and repair. The *in vivo* cell conversion approach described herein also differs significantly from many other approaches currently in development for stroke treatment, which have largely focused on short-term treatment immediately after ischemic injury, such as removing blood clots, promoting blood flow, anti-inflammation, anti-oxidant, or reducing excitotoxicity. While these approaches are necessary for short-term treatment, their long-term effectiveness will be limited if a large number of neurons are lost or functionally impaired after stroke.

Complementary to these short-term approaches, the *in vivo* cell conversion approach described herein offers a long-term solution by regenerating a large number of functional neurons by converting local glial cells and rebuilding local neural circuits that have been disrupted by ischemic injury. Notably, the *in vivo* cell conversion approach described herein has a broad time window of days or weeks or months, rather than hours, after ischemic stroke for neuroregeneration and tissue repair. Thus, optionally, a short-term treatment is administered immediately after ischemic injury according to aspects of the present invention, such as removing blood clots, promoting blood flow, anti-inflammation, anti-oxidant, or reducing excitotoxicity, in addition to administering a therapeutically effective dose of exogenous NeuroD1 as set out above and in the appended claims.

Converting reactive glial cells into functional neurons according to methods of treatment of the present invention is different from killing reactive glial cells. Reactive glial cells are a positive defense response against neural injury and killing reactive glial cells can worsen the infarct areas. On the other hand, it is well known that reactive glial cells release cytokines and inflammatory factors, including CSPG, LCN2, TNFα, IL-1β, etc., which can inhibit axonal regeneration and neural regrowth.

Instead of killing reactive glial cells, the NeuroD1-mediated *in vivo* cell conversion technology described herein turns reactive astrocytes into functional neurons, reducing the number of reactive astrocytes and the cytokines and inflammatory factors secreted by reactive glial cells. Notably, in the astrocyte-to-neuron conversion therapy described herein, astrocytes in the injury areas are replenished by newly generated astrocytes. While killing reactive astrocytes after injury results in detrimental effects, converting reactive astrocytes into neurons according to methods of treatment of the present invention yields neural repair.

### BRIEF DESCRIPTION OF THE DRAWINGS

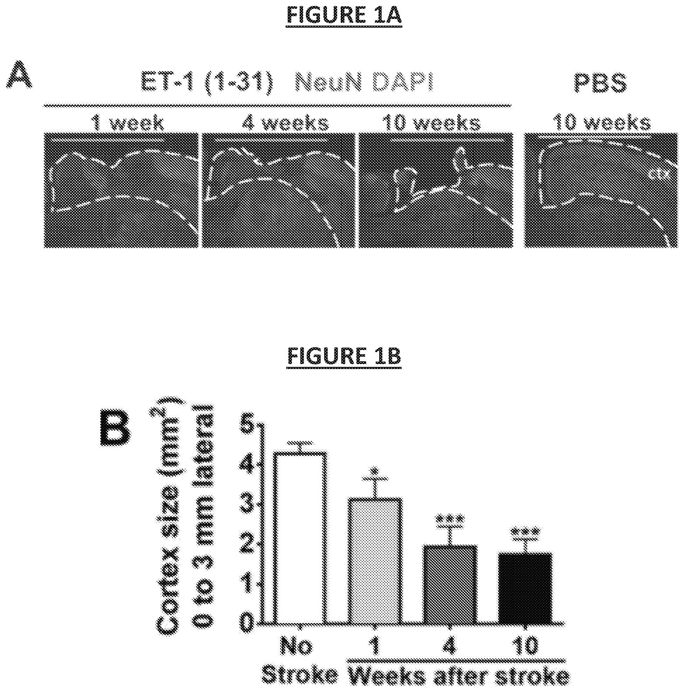

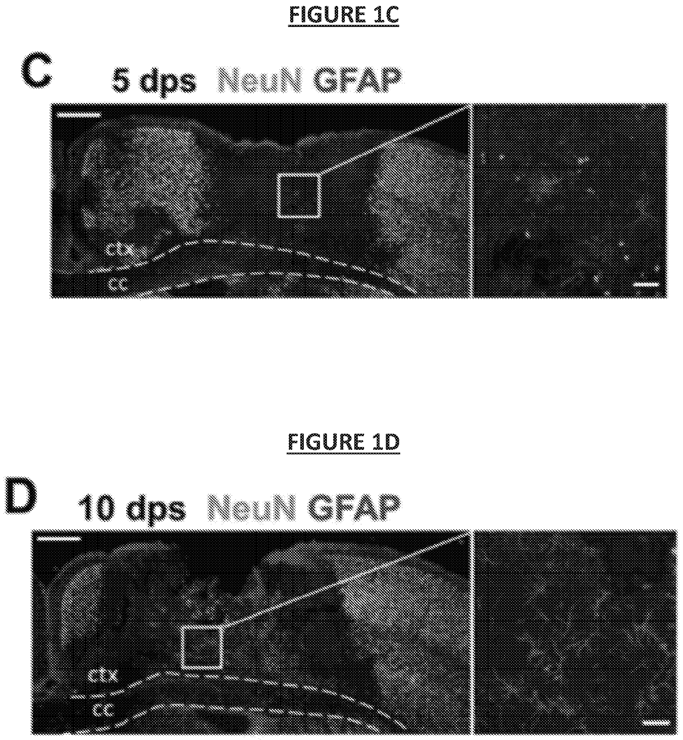

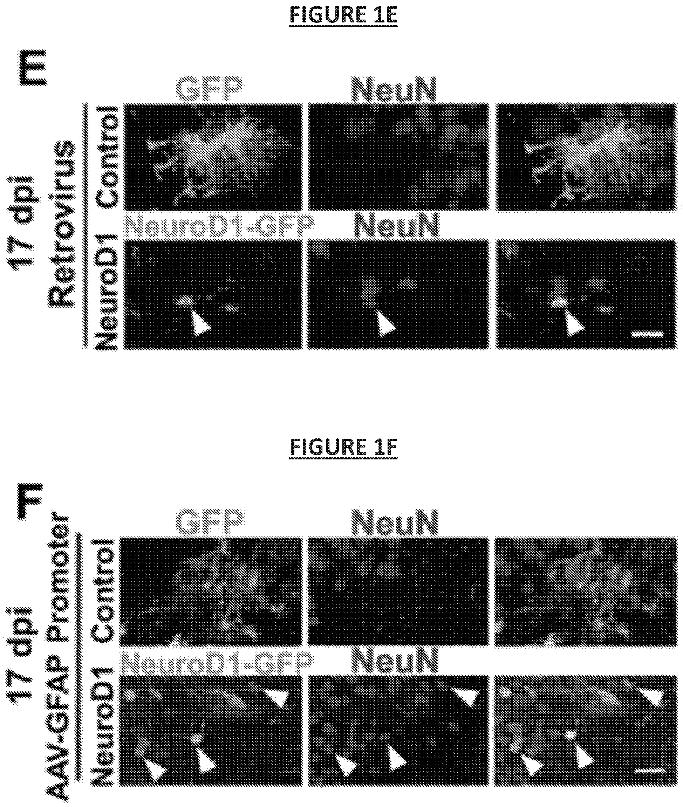

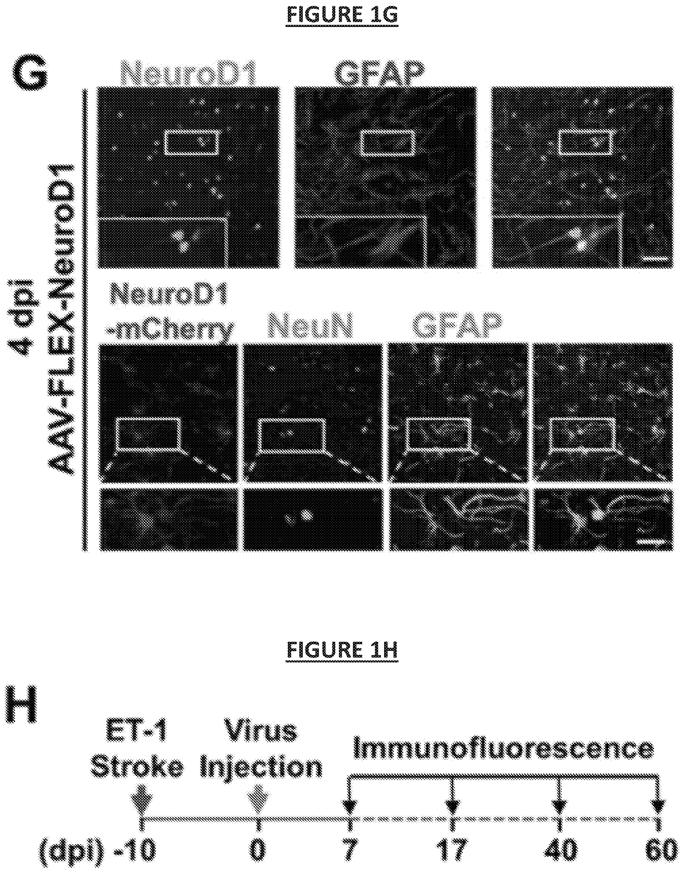

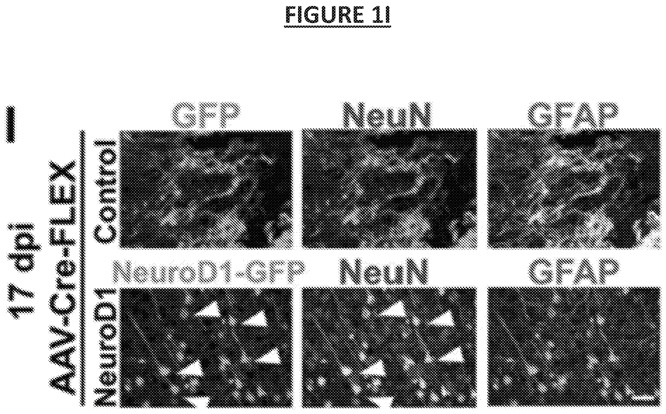

Figures 1A, 1B, 1C, 1D, 1E, 1F, 1G, 1H, and 1I show establishment of a focal stroke model and NeuroD1-mediated glia-to-neuron conversion.
Figure 1A shows that injection of endothelin-1 (1-31) into mouse motor cortex led to a gradual tissue loss starting from one week after the ischemic injury. Injection of PBS served as sham controls. Dashed lines indicate cortical areas. Scale bar: (A) 3 mm;
Figure 1B is a graph showing results of quantitative analysis of the cortical size from the midline to 3 mm lateral area at 1, 4 and 10 weeks after stroke (n = 3 animals for each time point).
Figures 1C and Figure 1D show results of immunostaining of NeuN (neuronal marker) and GFAP (astrocyte marker) at 5 days (Figures 1C) and 10 days post stroke (dps) (Figures 1D), respectively. Note that the NeuN signal was significantly impaired in the motor cortex, and the GFAP signal was weak at 5 dps but increased significantly at 10 dps. Dashed lines indicate corpus callosum. Scale bar: Left panel of Figures 1C and Figure 1D, 200 µm, Right inset panel of Figures 1C and Figure 1D 40 µm.
Figure 1E shows results of injection of retroviruses carrying CAG::GFP as control or CAG::NeuroD1-IRES-GFP at 10 dps and immunostaining at 17 days post viral injection (dpi). Expressing GFP alone only labeled glial cells, whereas NeuroD1-GFP expressing cells showed immunopositive signal for NeuN. Scale bar: 20 µm.
Figure 1F shows results of injection of adeno-associated virus (AAV9) carrying hGFAP::NeuroD1-P2A-GFP or hGFAP::GFP as control at 10 dps and immunostained at 17 dpi. GFP control group showed mainly glial cells without NeuN signal, whereas NeuroD1 group showed many NeuN-immunopositive neurons. Scale bar: 40 µm.
Figure 1G shows that at 4 dpi, injection of AAV9 hGFAP::Cre and CAG::FLEX-NeuroD1-P2A-mCherry resulted in significant NeuroD1 expression in GFAP-labeled astrocytes (top row). Interestingly, some NeuroD1-mCherry labeled cells showed both NeuN and GFAP signal (bottom row), suggesting a transition stage from astrocytes to neurons. Scale bar: (G) 40 µm (inset 20 µm).
Figure 1H is a schematic illustration of experimental design for stroke induction, AAV injection, and immunostaining procedures. Viral injection in this scheme indicates the Cre-FLEX system carrying NeuroD1 or GFP (mCherry) control in the rest experiments in mice.
Figure 1I shows that at 17 dpi, the GFP control group showed many GFAP+ reactive glial cells (top row, GFAP), whereas the majority of NeuroD1-GFP labeled cells became NeuN-positive neurons (bottom row). Scale bar: (I) 40 µm.
Figures 2A and 2B show establishment of a focal stroke model and AAV Cre-FLEX system.

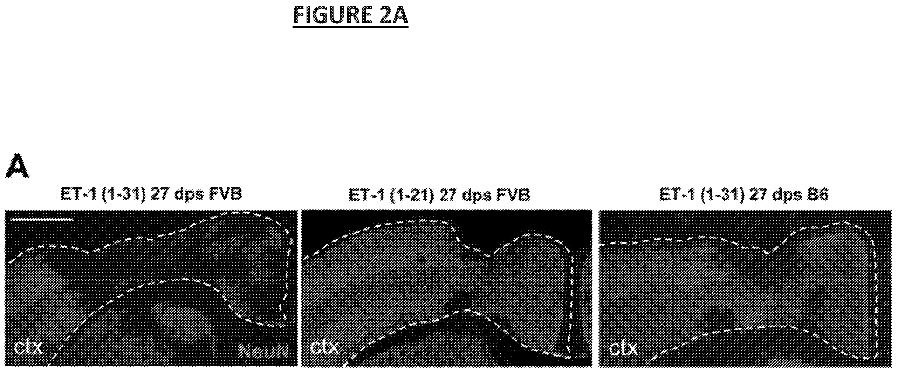

Figure 2A shows representative images illustrating severe ischemic injury induced by ET-1 (1-31) in FVB mice (left), but mild injury induced by ET-1 (1-21) in FVB mice or ET-1 (1-31) in B6 mice. NeuN immunostaining at 27 dps revealed that ET-1 (1-31) induced more NeuN loss and more severe cortical atrophy in FVB animals compared to the other two conditions. Scale bar: (A) 400 µm.

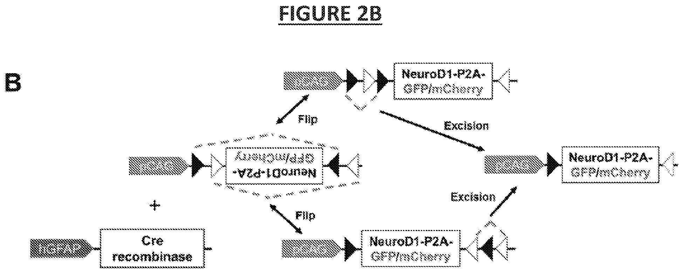

Figure 2B) Schematic illustration of the working mechanism of a Cre-FLEX system according to aspects of the present invention.

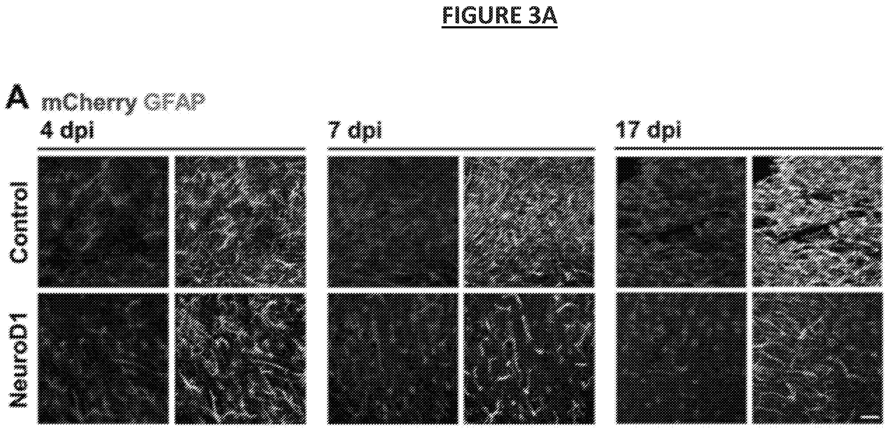

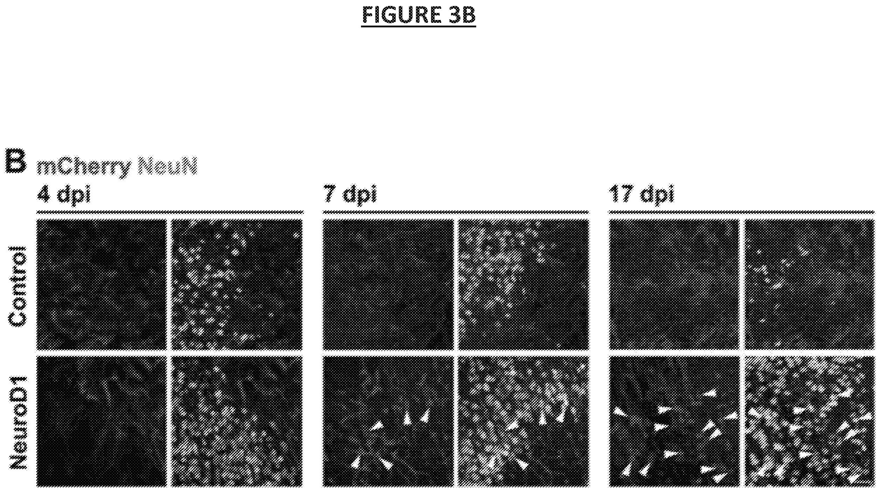

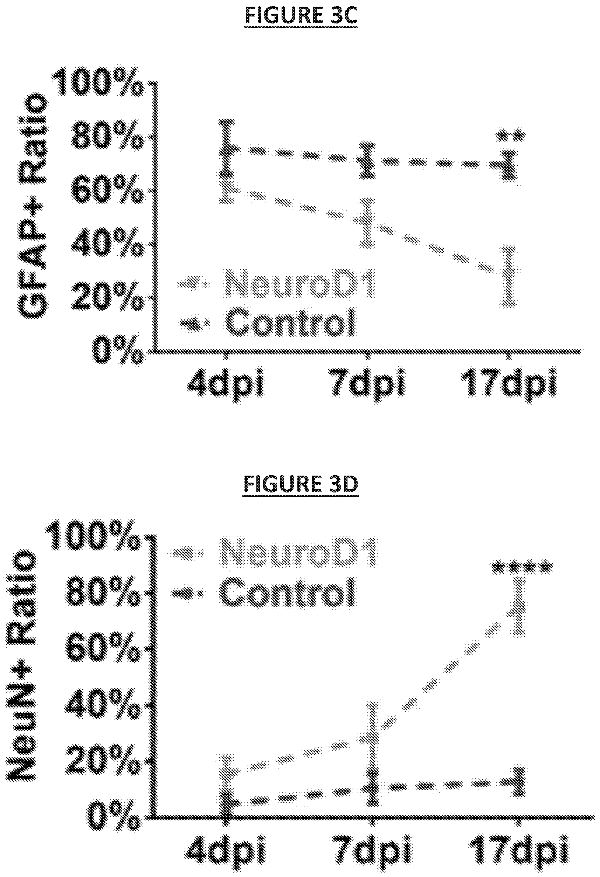

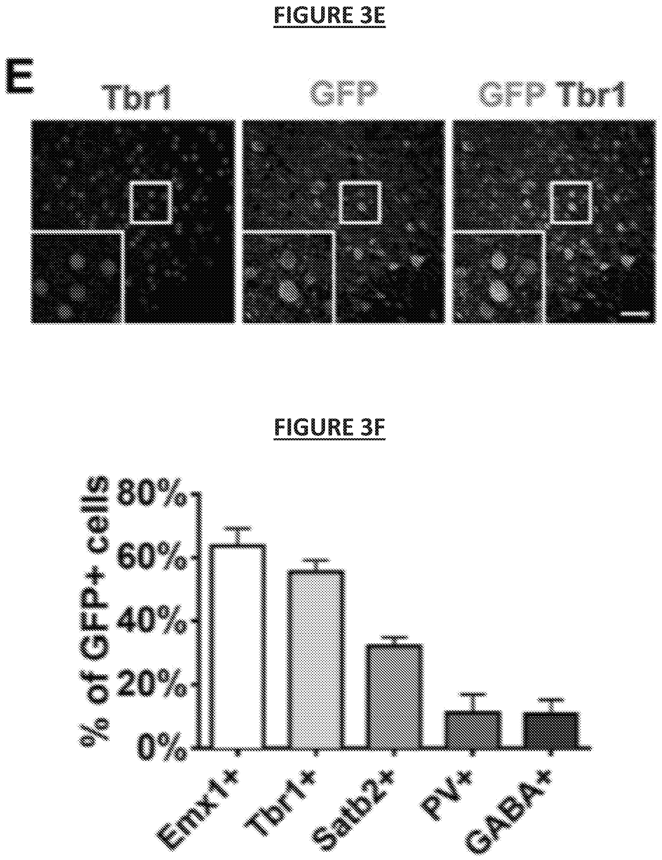

Figures 3A, 3B, 3C, 3D, 3E, and 3F show that NeuroD1 efficiently converts reactive astrocytes into cortical neurons.
Figure 3A shows identification of astrocytes with GFAP immunostaining at 4, 7 or 17 days post viral injection (dpi) in both control and NeuroD1 groups. Scale bar: (A) 40 µm.
Figure 3B shows identification of neurons with NeuN immunostaining at 4, 7 or 17 dpi. Arrowheads indicate some of the NeuroD1-converted neurons. Scale bar: (B) 40 µm.
Figure 3C is a graph showing quantification of GFAP+ cells among all viral infected cells. Note a significant decrease of astrocytes in NeuroD1 group. ** P < 0.01, Two-way ANOVA followed by Sidak's multiple comparison test. n = 3 mice per group. 3 images were randomly taken in cortical areas with viral infection. Data are represented as mean ± s.e.m.
Figure 3D is a graph showing quantification of NeuN+ cells among all viral infected cells. Note a significant increase of neurons in NeuroD1 group. **** P < 0.0001, Two-way ANOVA followed by Sidak's multiple comparison test. n = 3 mice per group. 3 images were randomly taken in cortical areas with viral infection. Data are represented as mean ± s.e.m.
Figure 3E shows representative images illustrating NeuroD1-converted neurons (GFP+) expressing cortical marker (Tbr1) at 60 dpi. Note that Tbr1+ cells include both converted and non-converted cells. Scale bar: (E) 40 µm.
Figure 3F is a graph showing characterization of the neuronal identity afterNeuroD1-mediated in vivo cell conversion. Many NeuroD1-converted neurons were immunopositive for cortical neuronal markers but only 10% were GABAergic neurons. n = 3 mice each group. Data are represented as mean ± s.e.m.

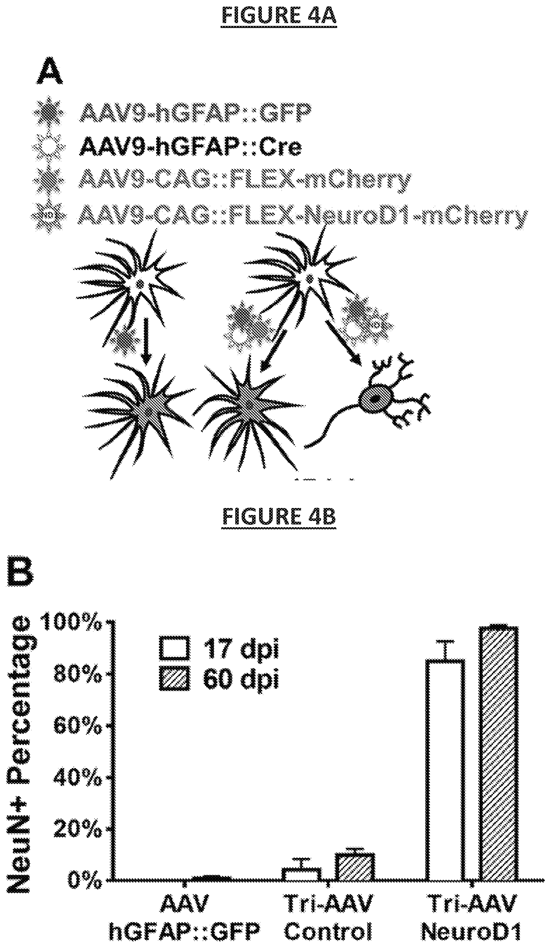

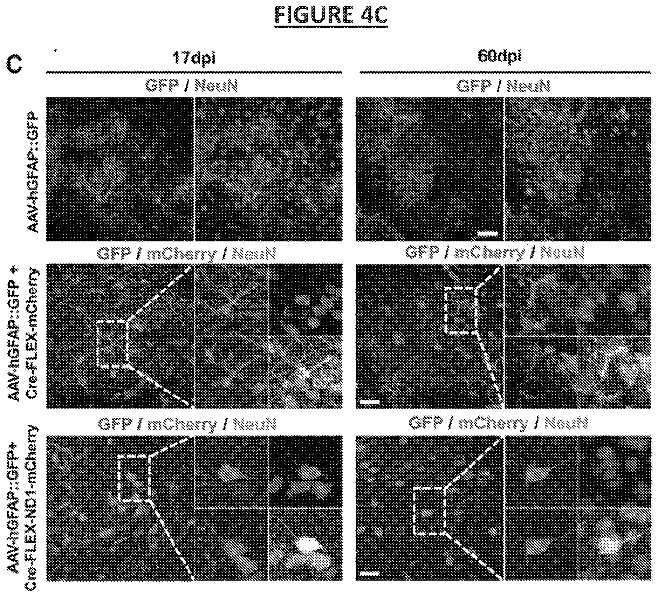

Figures 4A, 4B, and 4C show a Tri-AAV system to trace NeuroD1-converted neurons.
Figure 4A is a schematic diagram illustrating the mechanism of Tri-AAV tracing system: when only AAV-hGFAP::GFP is injected, only astrocytes will be labeled green. When AAV-hGFAP::GFP is injected together with the Cre-FLEX-mCherry control system, astrocytes will express both GFP and mCherry and show yellow color. When AAV-hGFAP::GFP is injected together with the Cre-FLEX-NeuroD1-mCherry conversion system, astrocytes will be converted into neurons and express both GFP and mCherry, appearing yellow but NeuN+.
Figure 4B is a graph showing quantification of conversion efficiency by Tri-AAV tracing system at both 17 dpi and 60 dpi. For hGFAP::GFP group, percentage of GFP+/NeuN+ cells among all GFP+ cells was quantified. For hGFAP::GFP plus Cre-FLEX-mCherry or Cre-FLEX-NeuroD1-mCherry group, percentage of GFP+/mCherry+/NeuN+ cells among all GFP+/mCherry+ was quantified; (n = 2 animals and >150 cells per group).
Figure 4C shows representative images showing different levels of colocalization of GFP, mCherry and NeuN signal in three groups at 17 and 60 dpi. Scale bar: (C) 40 µm.

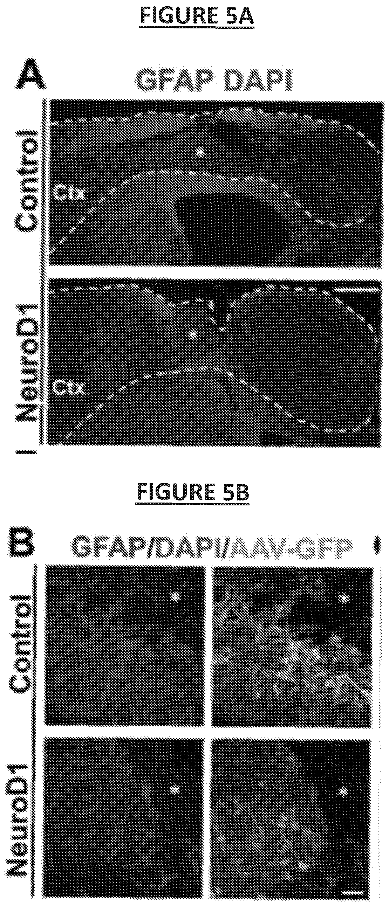

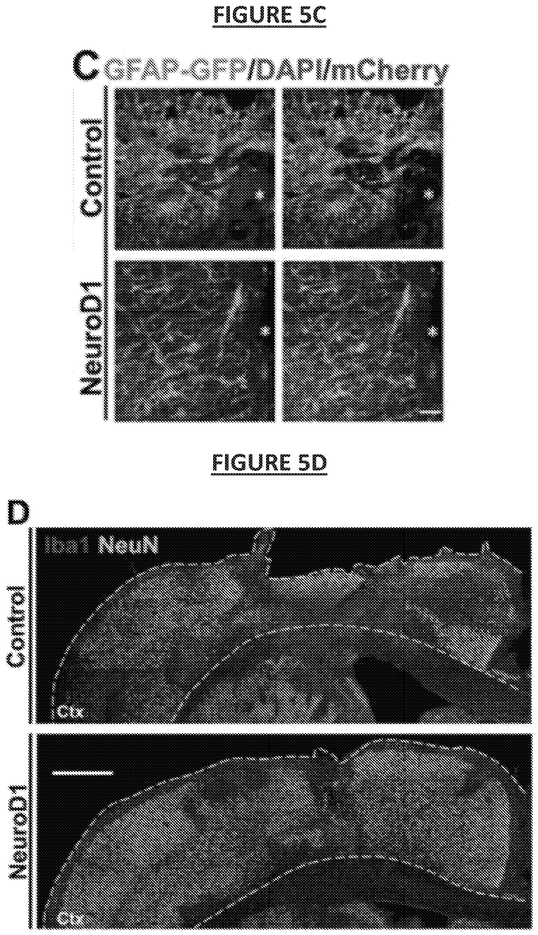

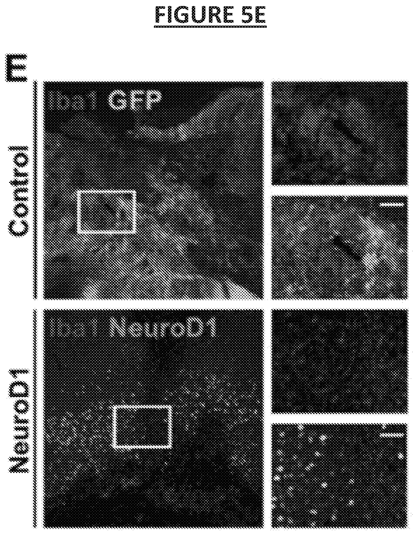

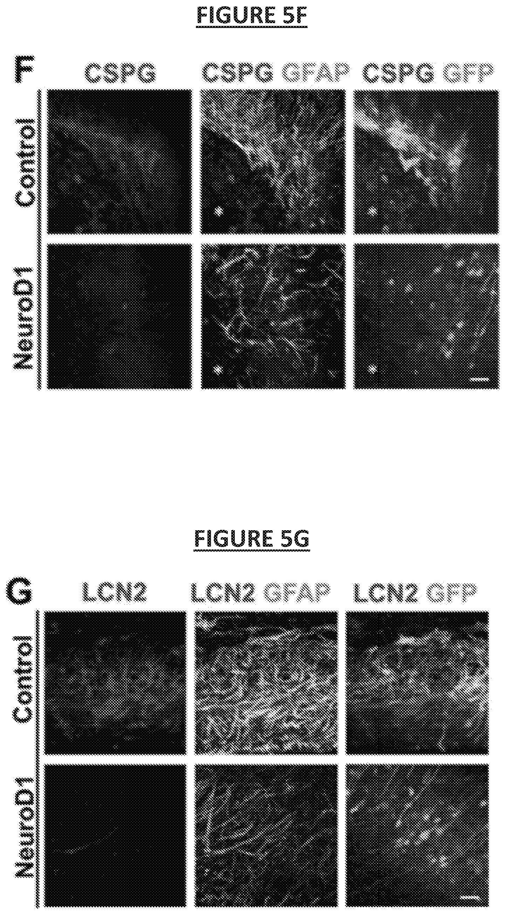

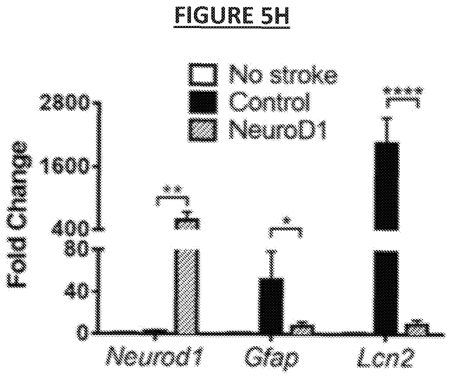

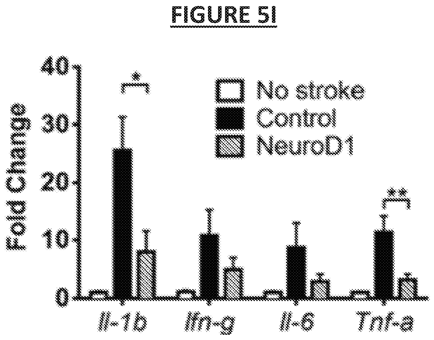

Figures 5A, 5B, 5C, 5D, 5E, 5F, 5G, 5H, and 5I show that NeuroD1 treatment reduces neuroinflammation in the stroke areas.
Figure 5A shows representative low magnification images illustrating GFAP-labeled reactive astrocytes in the stroke cortex in control and NeuroD1 group at 17 dpi. Dashed lines indicate cortex areas. Stroke injury core is labeled by asterisk (□).Scale bar: (A) 400 µm.
Figure 5B shows high magnification Images illustrating GFAP-labeled reactive astrocytes (red) in the peri-infarct areas following stroke in the GFP control group (top row) and NeuroD1 group (bottom row). Note that the astrocytes in the NeuroD1 group appeared to be less reactive in morphology compared to the control group. The injury core is labeled by asterisk. Scale bar: (B) 40 µm.
Figure 5C shows images from transgenic GFAP-GFP mice further illustrating that a significant number of astrocytes persisted in the NeuroD1-converted areas (bottom row), suggesting that astrocytes are not depleted after cell conversion. Scale bar: (C) 40 µm.
Figure 5D shows low magnification images showing microglia marker Iba1 in control group and NeuroD1 group at 17dpi. Note a significant reduction of Iba1 signal in NeuroD1 group. Scale bar: (D) 400 µm.
Figure 5E shows high magnification images showing reactive microglia (Iba1) with amoeboid shape in the GFP control group (top), but more ramified morphology in the NeuroD1 group (bottom). Scale bar: (E) 40 µm.
Figure 5F shows immunofluorescent images showing a significant reduction of CSPG, an important neuroinhibitory factor released by reactive glial cells, in the NeuroD1 group compared to the control group in the peri-infarct area at 17dpi. Scale bar: (F) 40 µm.
Figure 5G shows immunofluorescent images showing a significant reduction of LCN2, an inflammatory factor secreted by reactive glial cells, in the NeuroD1 group compared to the control group at 17 dpi. Scale bar: (G) 40 µm.
Figure 5H is a graph showing results of real-time quantitative PCR (RT-PCR) verified the NeuroD1 expression in NeuroD1-infected tissues, but revealed a significant decrease of the reactive astrocyte markers GFAP and LCN2 in the NeuroD1 group. * P < 0.05, ** P < 0.01, **** P < 0.0001, One-way ANOVA followed by Tukey's multiple comparison test. n = 4 mice for each group. Data are represented as mean ± s.e.m.
Figure 5I is a graph showing that RT-PCR revealed a significant increase of pro-inflammatory factors IL-1β, IFNγ, IL-6 and TNFα after stroke, but a significant decrease after NeuroD1 treatment. * P < 0.05, ** P < 0.01, One-way ANOVA followed by Tukey's multiple comparison test. n = 4 mice for each group. Data are represented as mean ± s.e.m.

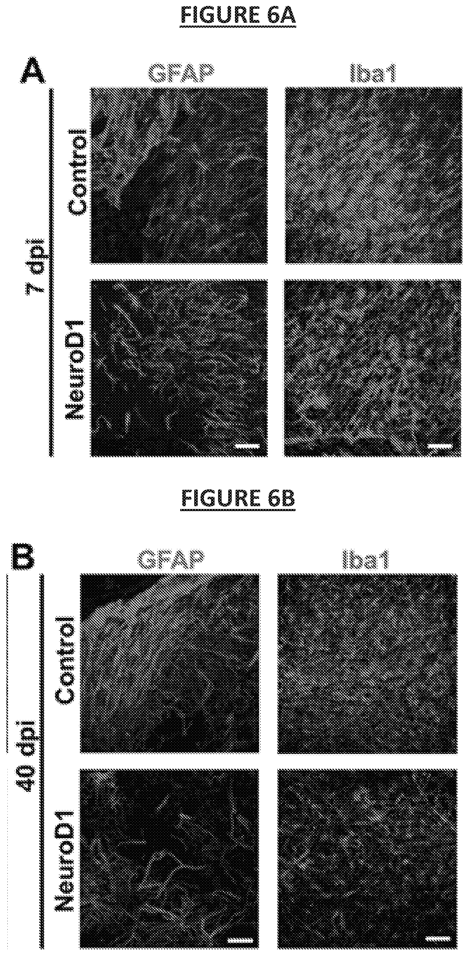

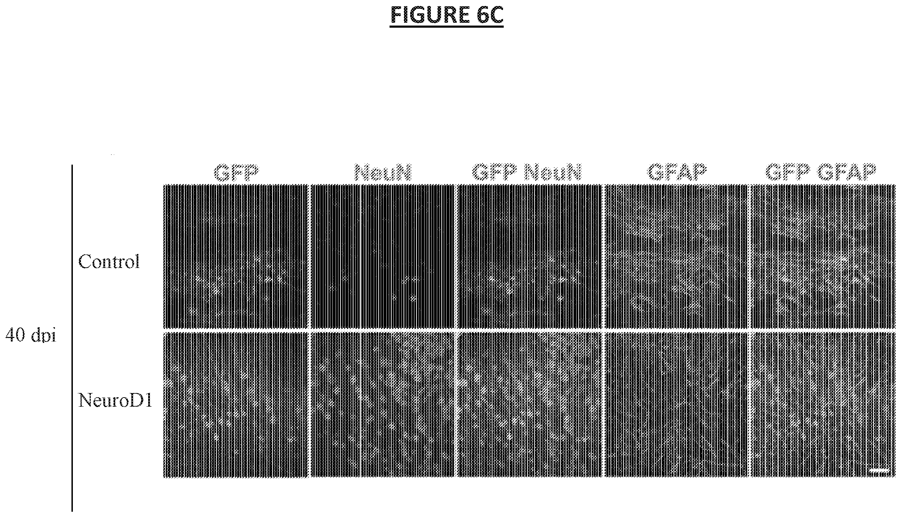

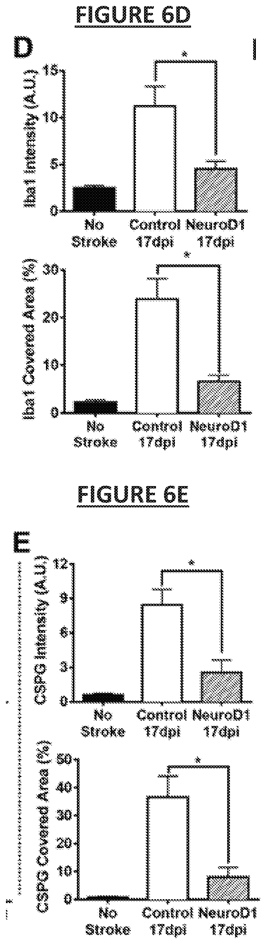

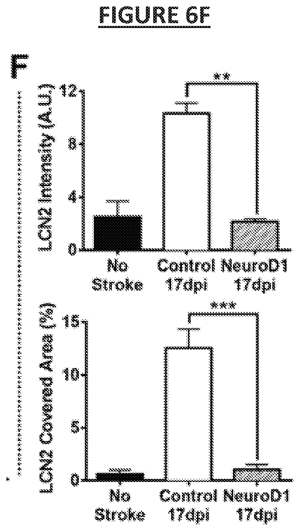

Figures 6A, 6B, 6C, 6D, 6E, and 6F show reduction of reactive glial cells and inflammation after NeuroD1 treatment.
Figures 6A and Figure 6B show representative images of astrocytes (GFAP) and microglia (Iba1) in the peri-infarct area at 7 dpi (Figure 6A) and 40 dpi (Figure 6B). Note that in NeuroD1 group at 40 dpi, astrocytes and microglia appeared less in number and also less reactive in morphology compared to the control group. Scale bars: 40 µm.
Figure 6C) Representative images of GFP (NeuroD1-GFP) co-stained with GFAP and NeuN in peri-infarct areas at 40 dpi. Note that in NeuroD1-infected areas, many astrocytes were detected and less reactive, suggesting that they were not depleted after conversion. Scale bar: 40 µm.
Figures 6D, 6E, and 6F are graphs showing quantification of the signal intensity (A.U., artificial unit) and covered area (%) for microglial marker Iba1 (Figure 6D), glial scar inhibitory factor CSPG (Figure 6E), and astrocyte inflammatory marker LCN2 (Figure 6F). * P < 0.05, ** P < 0.01, *** P < 0.001. One-way ANOVA followed by Tukey multiple comparison test, n = 3 mice per group. Data are represented as mean ± s.e.m.

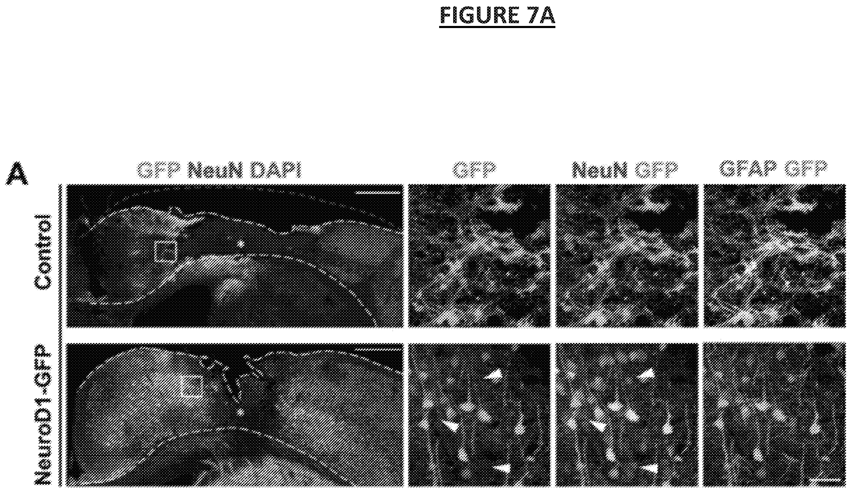

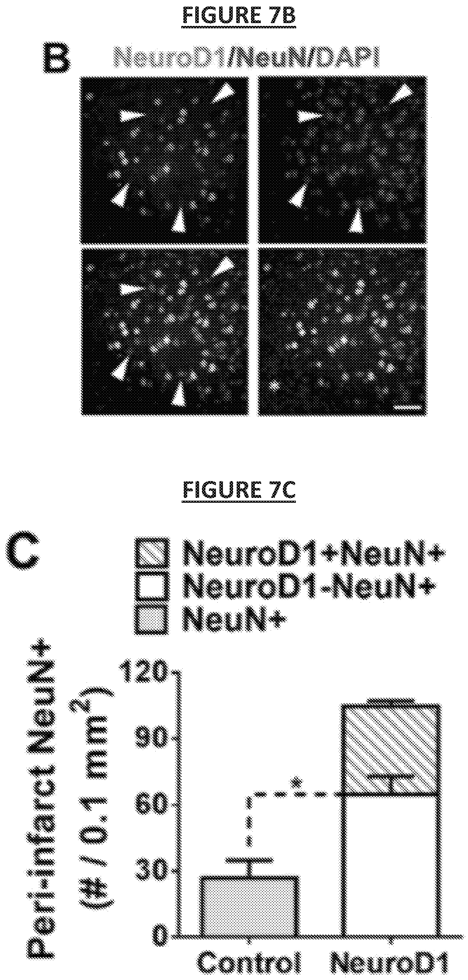

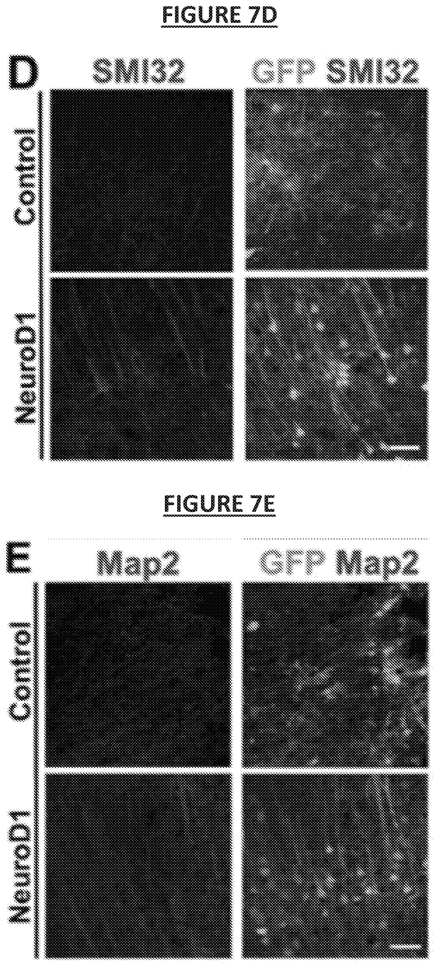

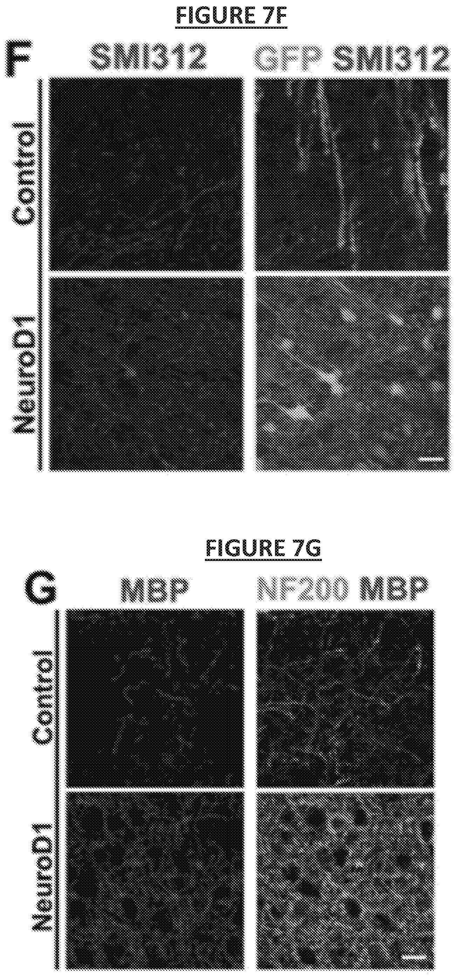

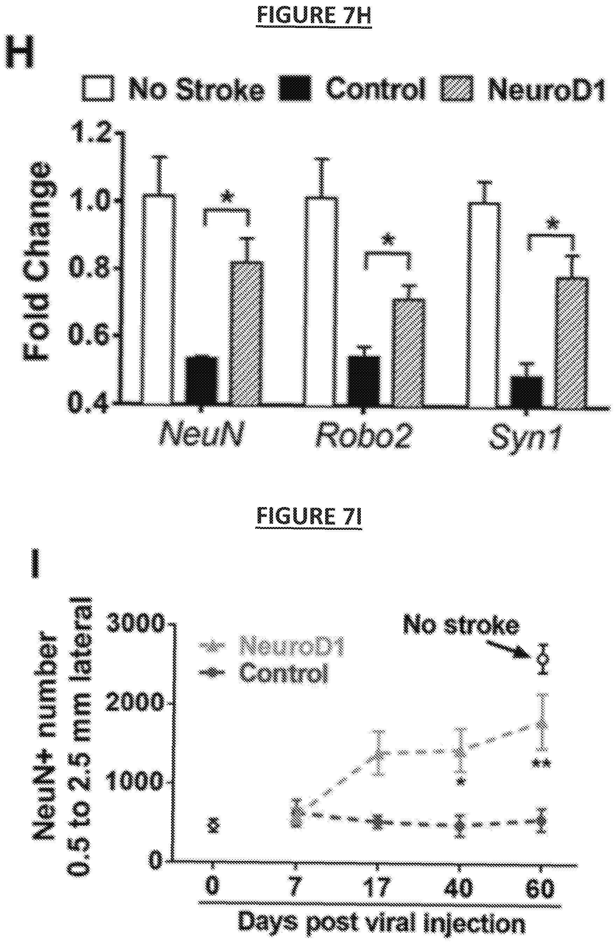

Figures 7A, 7B, 7C, 7D, 7E, 7F, 7G, 7H, and 7I show that NeuroD1 regenerates new neurons and preserves injured neurons in the stroke areas.
Figure 7A shows a comparison of the motor cortex (17 dpi) between the control (top row) and NeuroD1 group (bottom row). Low magnification panels in the left showing the overall cortical morphology after stroke, while right panels showing enlarged images in the peri-infarct areas. Note that NeuroD1 group showed not only converted neurons (GFP+) but also non-converted neurons (NeuN+ but GFP-, arrowheads). Scale bar: 500 µm for the left low magnification cortex images; 40 µm for enlarged inset images;
Figure 7B shows immunofluorescent staining of NeuroD1 and NeuN in NeuroD1 group at 17 dpi, illustrating both NeuroD1-converted and non-converted neurons intermingled together. Arrowheads point to non-converted neurons (NeuN+ but NeuroD1-). Scale bar: (B) 40 µm.
Figure 7C is a graph showing quantification of NeuN+ cells in peri-infarct area of control group and NeuroD1 group, with the later included both NeuroD1+ and NeuroD1- neurons. Note that the number of non-converted neurons in the NeuroD1 group more than doubled the number in the control group, suggesting a neuroprotective effect of NeuroD1 conversion.
Figure 7D and Figure 7E show immunofluorescent images of neuronal dendrite markers SMI32 (Figure 7D) and MAP2 (Figure 7E) showing much improved neuronal morphology in the NeuroD1 group (bottom row) compared to the control group (top row). Scale bars: 40 µm.
Figure 7F and Figure 7G show immunostaining of axonal marker SMI312 (Figure 7F), NF200 (Figure 7G) and axon myelination marker MBP (Figure 7G) demonstrating increased axons and axonal myelination in the NeuroD1 group (bottom rows) compared to the control group (top rows). Note the better colocalization of NF200 and MBP in NeuroD1 group than the control group. Scale bars: 20 µm.
Figure 7H is a graph showing results of RT-PCR which revealed a significant increase in the expression level of neuronal factors including NeuN, Robo2 and Syn1 in the stroke areas of the NeuroD1 group compared to control. * P < 0.05, One-way ANOVA followed by Tukey's multiple comparison test. n = 4 mice each group. Data are represented as mean ± s.e.m.
Figure 7I is a graph showing quantitative analysis of the total number of NeuN+ cells in the motor cortical areas between 500-2500 µm lateral from the midline. Note a significant difference between the control and NeuroD1 group by 60 dpi. n = 3 mice in each group. * P < 0.05, ** P < 0.01, Two-way ANOVA followed by Sidak's multiple comparison tests. Data are represented as mean ± s.e.m.

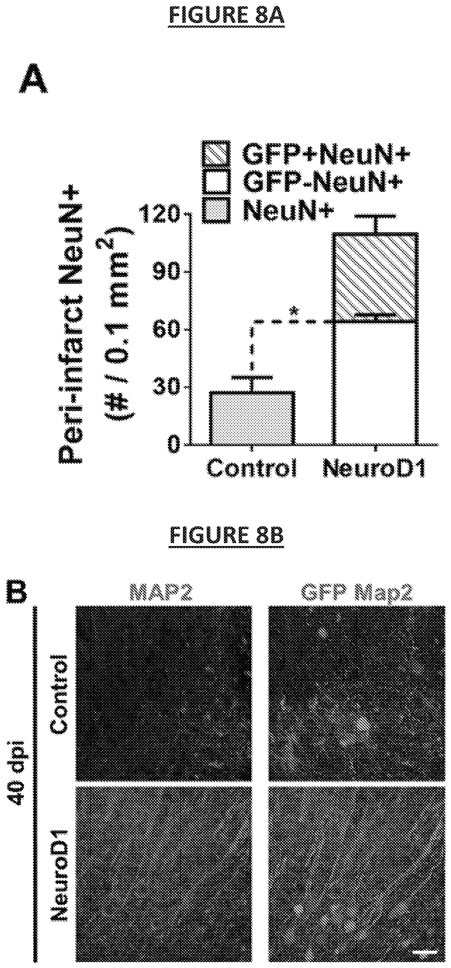

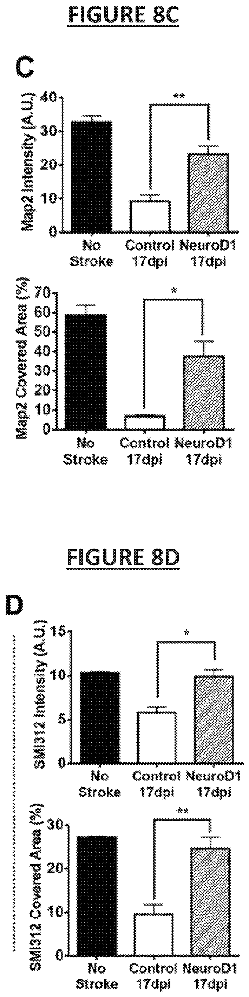

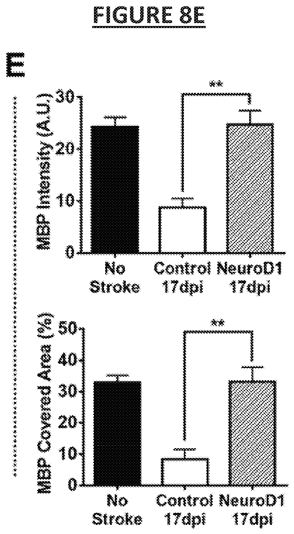

Figures 8A, 8B, 8C, 8D, and 8E show neuroregeneration and neuroprotection after NeuroD1-treatment.
Figure 8A is a graph showing quantification of NeuN positive cells in the peri-infarct areas of control group and NeuroD1 group. In NeuroD1 group, NeuN positive cells were further divided into NeuroD1-converted (GFP+/NeuN+ double positive) and non-converted neurons (GFP negative).
Figure 8B shows representative images of neuronal dendritic marker Map2 in the peri-infarct area at 40 dpi. Scale bar: 40 µm.
Figures 8C, 8D, and 8E are graphs showing quantification of the signal intensity (A.U., artificial unit) and covered area (%) for dendritic marker Map2 (Figure 8C), axon marker SMI312 (Figure 8D) and myelination marker MBP (Figure 8E). * P < 0.05, ** P < 0.01. One-way ANOVA followed by Tukey multiple comparison test, n = 3 mice per group. Data are represented as mean ± s.e.m.

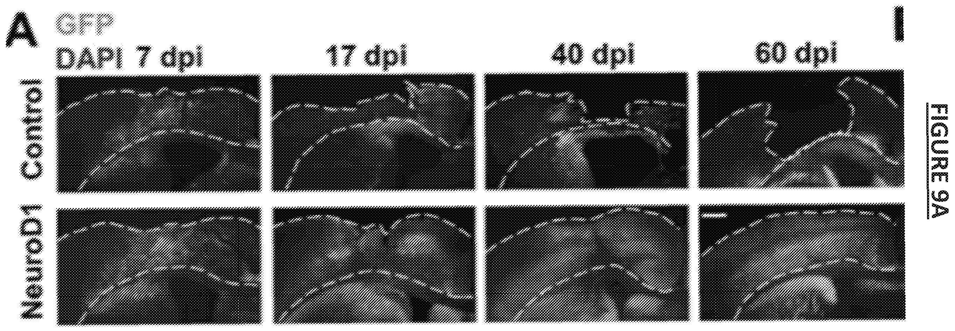

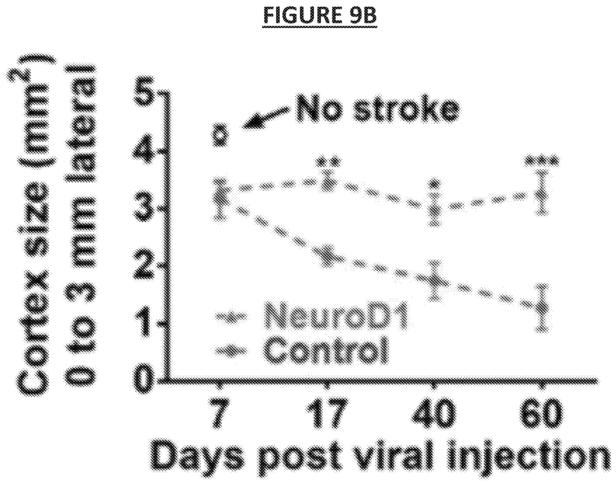

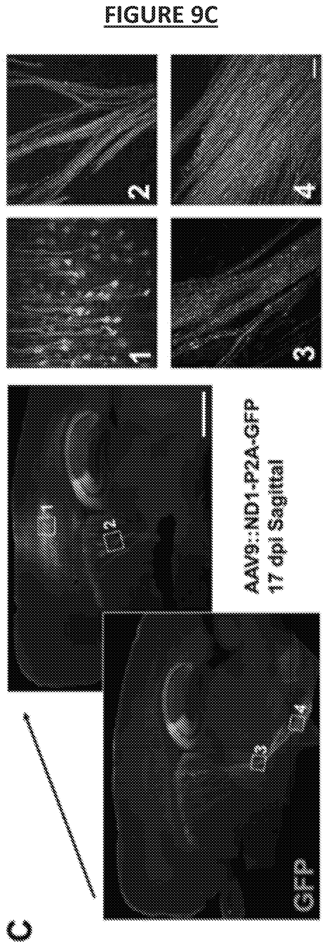

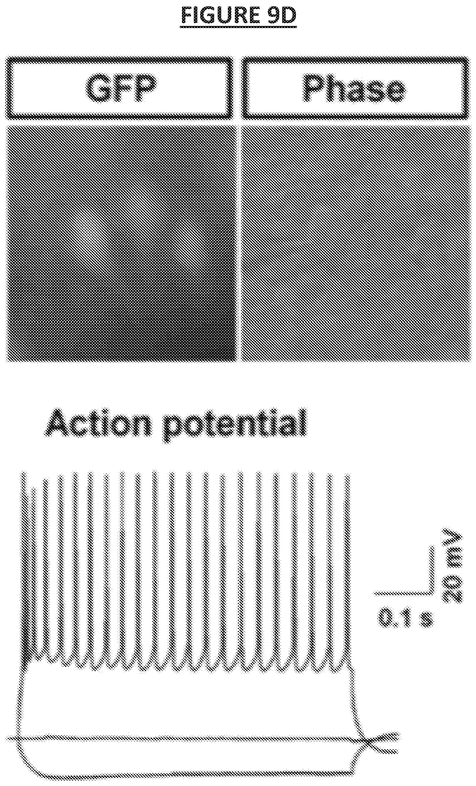

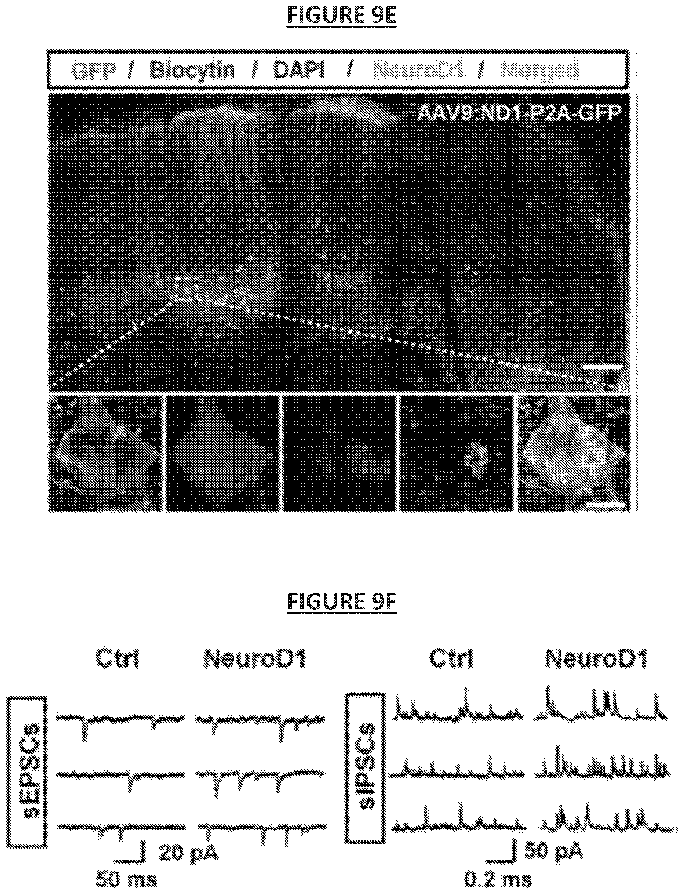

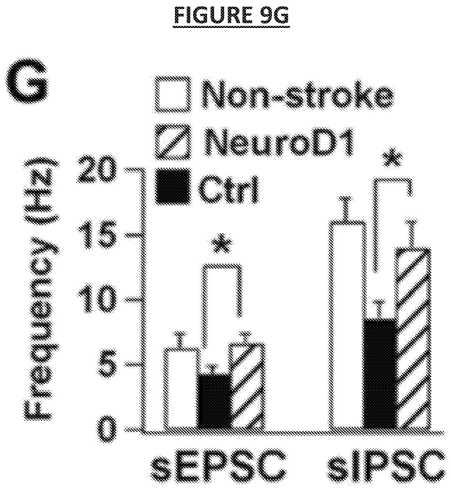

Figures 9A, 9B, 9C, 9D, 9E, 9F, and 9G show reconstitution of neural circuits by NeuroD1-mediated cell conversion in the stroke areas.
Figure 9A shows low magnification images illustrating cortical tissue loss in the GFP control group (top row), and the rescue of tissue loss by NeuroD1 treatment (bottom row). GFP labels AAV-infected cells, and DAPI labels cell nuclei. Dashed lines delineate the cortical areas. Scale bar: 400 µm.
Figure 9B is a graph showing results of quantitative analysis of the cortical areas (from midline to 3 mm lateral) in the control versus NeuroD1 group at 7, 17, 40 and 60 dpi. Note a steady tissue loss in the control group, but a clear cortical preservation in the NeuroD1 group.* P < 0.01, ** P < 0.01, *** P < 0.005, Two-way ANOVA followed by Sidak's multiple comparison test. n = 3 mice per group. Data are represented as mean ± s.e.m.
Figure 9C shows representative images of serial sagittal sections from medial (lower left) to lateral (upper) position, illustrating axonal projection from NeuroD1-converted neurons in the cortex (box 1) to striatum (box 2), thalamus (box 3), and hypothalamus (box 4). Scale bars: 1000 µm for left sagittal images; 40 µm for inset images.
Figure 9D shows fluorescent (GFP) and bright field images showing NeuroD1-infected neurons in brain slices (top). Bottom, representative traces showing repetitive action potentials evoked in NeuroD1-converted neurons (60 dpi).
Figure 9E shows results of Biocytin infusion in the recording pipette during whole-cell recording followed with post-fix immunostaining illustrating that the recorded neurons were both GFP+ and NeuroD1+, with complex apical dendrites at 60 dpi. Scale bars: 200 µm for the top low magnification image; 5 µm for inset images in bottom row.
Figure 9F shows representative traces of excitatory (sEPSCs) and inhibitory synaptic events (sIPSCs) recorded in NeuroD1-GFP labeled neurons (60 dpi).
Figure 9G is a graph showing quantification of the frequency of both sEPSCs and sIPSCs in cortical slices without stroke (white bar), or with stroke (black bar, GFP control; striped bar, NeuroD1 group). Note that after stroke, NeuroD1 group showed significantly higher frequency of both sEPSCs and sIPSCs than the control group (EPSC: control, 4.3 ± 0.6, n = 22; NeuroD1, 6.7 ± 0.8, n = 25; p = 0.023, Student's t test) (IPSC: control, 8.6 ± 1.3, n = 22; NeuroD1, 14.0 ± 2.0, n = 25; p = 0.032, Student's t test).

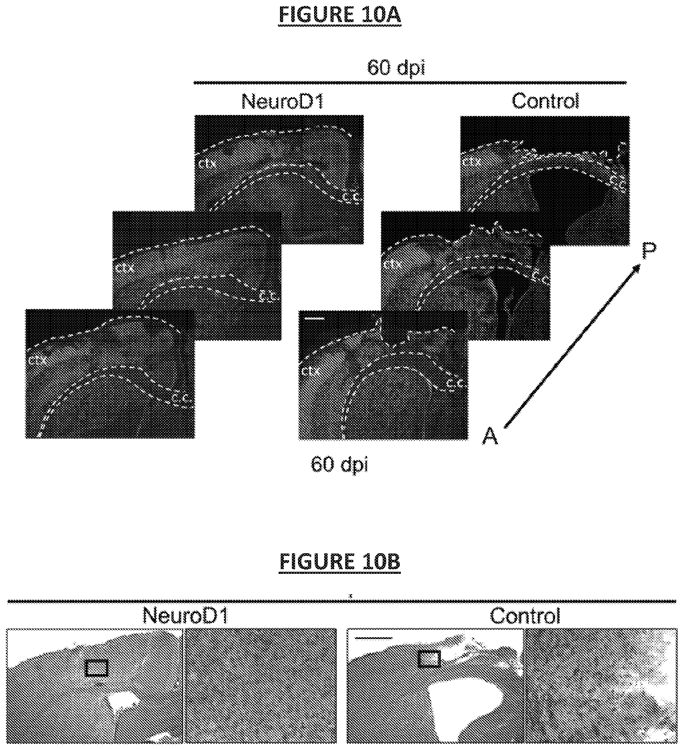

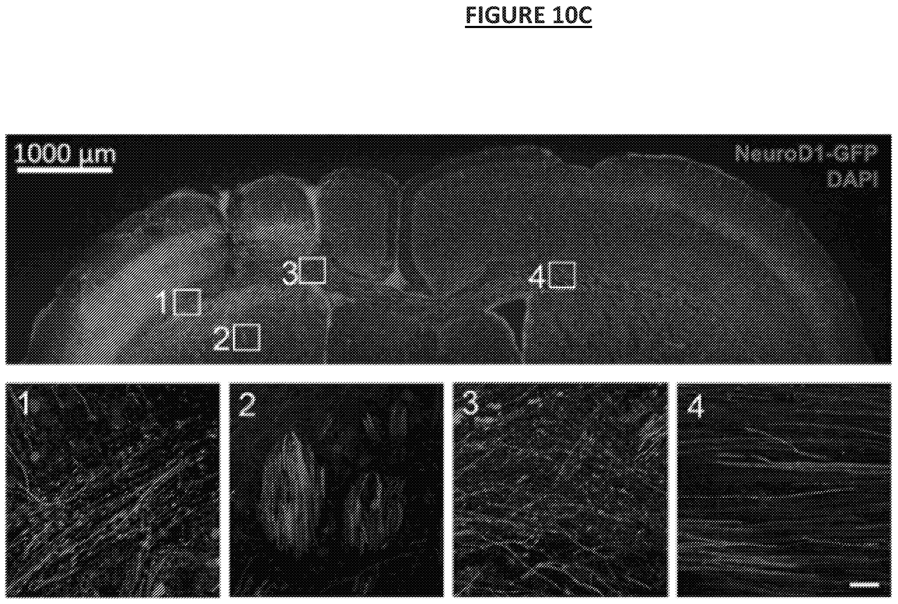

Figures 10A, 10B, and 10C show brain tissue repair and long range axonal projection after NeuroD1 treatment.
Figure 10A shows serial brain sections from anterior (A) to posterior (P) in the control group vs NeuroD1 group. Note severe tissue damage in the control group. ctx, cortex; c.c., corpus callosum. Scale bar: 400 µm.
Figure 10B shows H&E (hematoxylin and eosin) staining at 60 dpi also shows severe tissue damage in the control group. Note an enlarged lateral ventricle in the control group, which typically associates with severe brain damage. Scale bar: 400 µm.
Figure 10C shows coronal section of mouse cortex at 9 months post NeuroD1 virus injection, showing long-range axon projecting to the contralateral side through corpus callosum. Bottom row illustrates enlarged view of axon projection inside the corpus callosum (ipsilateral shown in box 1 and 3, contralateral in box 4) and subcortical projection through the striatum (box 2). Scale bars: top row: 1000 µm, bottom row: 40 µm.

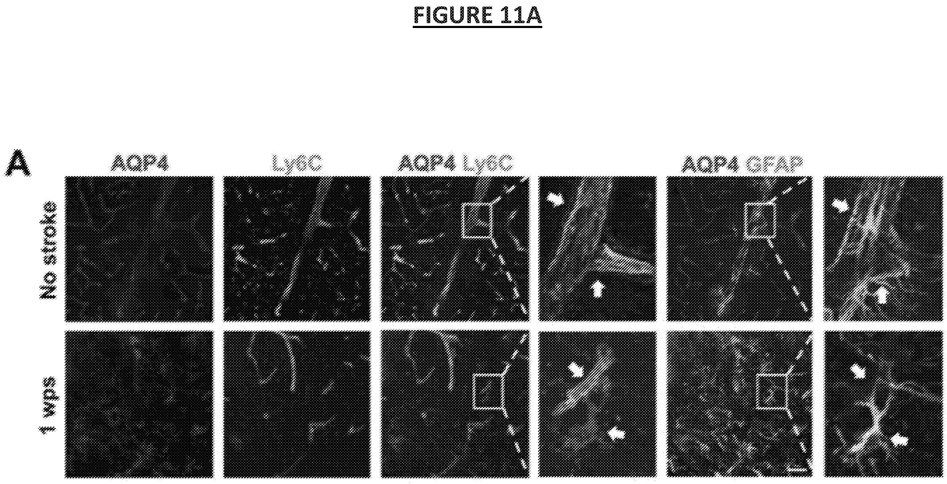

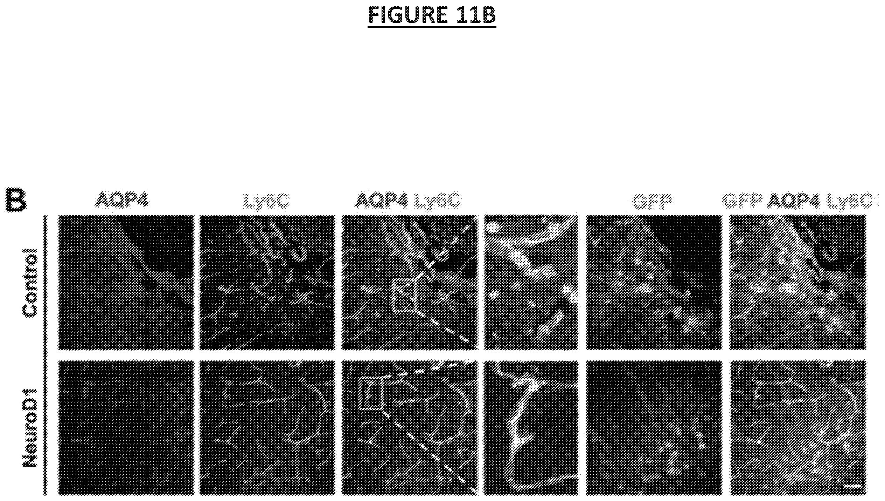

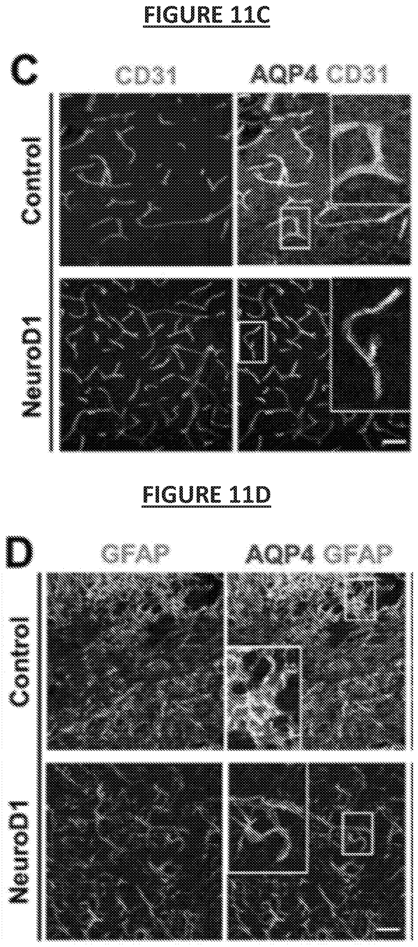

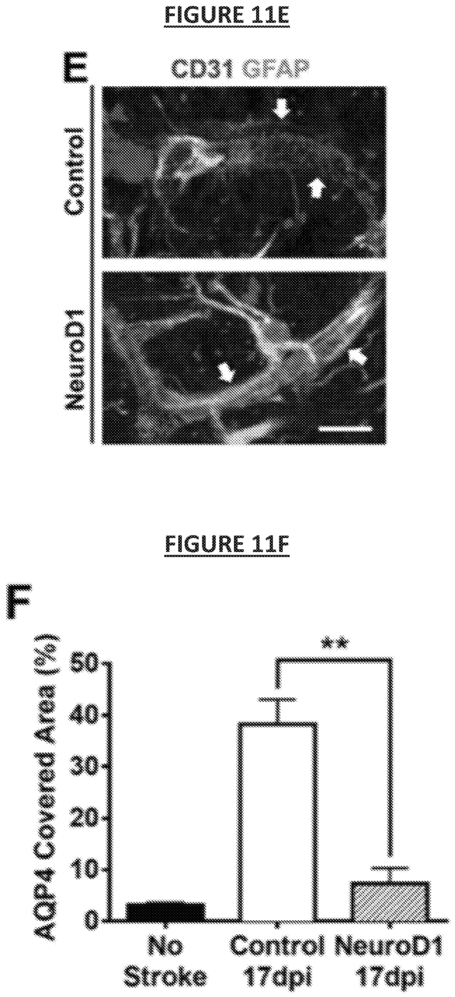

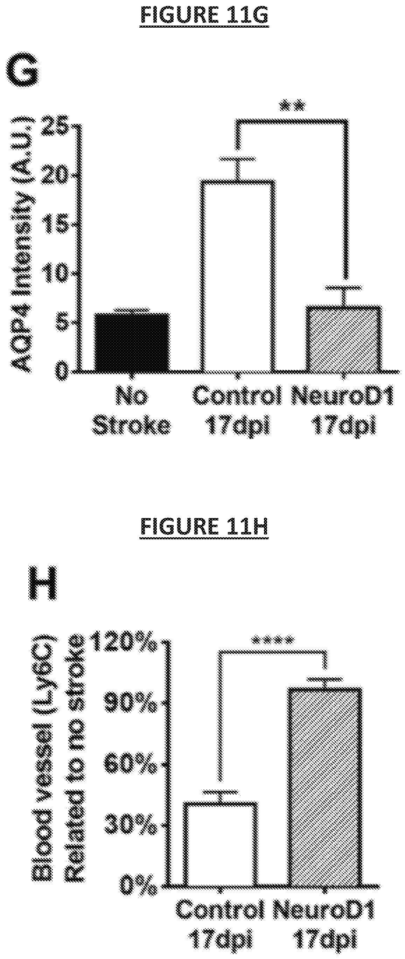

Figures 11A, 11B, 11C, 11D, 11E, 11F, 11G, and 11H show repair of blood vessels and blood-brain-barrier by NeuroD1-treatment.
Figure 11A shows disruption of blood vessels and blood-brain-barrier (BBB) induced by ischemic injury. Top row illustrates that in non-stroke brain, astrocytic endfeet (marked by a water channel aquaporin 4, AQP4, red) wrapped around blood vessels (labeled by monocyte and endothelial cell marker Ly6C, cyan) to form intact BBB. Arrows indicate colocalization of AQP4 with GFAP around blood vessel. Bottom row, 1 week post stroke (wps), AQP4 signal dissociated from blood vessels, suggesting a disruption of BBB. Scale bar: 40 µm.
Figure 11B shows comparison of blood vessels and BBB between the control and NeuroD1 groups after stroke. Top row, control group showed disrupted blood vessels and diffused AQP4 signal throughout the stroke areas. Bottom row, NeuroD1 group showed much improved blood vessels associated with AQP4 signal, suggesting repaired BBB after cell conversion (17 dpi). Scale bar: 40 µm.
Figure 11C shows immunofluorescent images of another blood vessel marker CD31 costaining with AQP4, confirming the rescue of blood vessels and BBB in the NeuroD1-treated group (17 dpi). Scale bar: 40 µm.
Figure 11D shows co-immunostaining of AQP4 and GFAP showing mislocalized AQP4 signal in reactive astrocytes in control group, compared to concentrated AQP4 signal in the endfeet of astrocytes in NeuroD1 group (17 dpi). Scale bar: 40 µm.
Figure 11E shows high power images illustrating the dissociation of astrocytic endfeet (GFAP) from the blood vessels (CD31) in the control group (top), compared to a nice re-association of astrocytic endfeet with the blood vessels in NeuroD1 group (bottom). Scale bar: 5 µm.
Figures 11F and 11G are graphs showing quantification of AQP4 signal intensity (Figure 11F) and covered area (Figure 11G) in non-stroke, stroke control group, and stroke NeuroD1 group (17 dpi). NeuroD1 group is much closer to the non-stroke group. ** P < 0.01. One-way ANOVA followed by Tukey multiple comparison test, n = 3 mice per group. Data are represented as mean ± s.e.m.
Figure 11H is a graph showing quantification of blood vessels (Ly6C signal) in peri-infarct areas of control group and NeuroD1 group. NeuroD1 group showed significantly more blood vessels than the control group. **** P < 0.0001. One-way ANOVA followed by Tukey multiple comparison test, n = 3 per mice group. Data are represented as mean ± s.e.m.

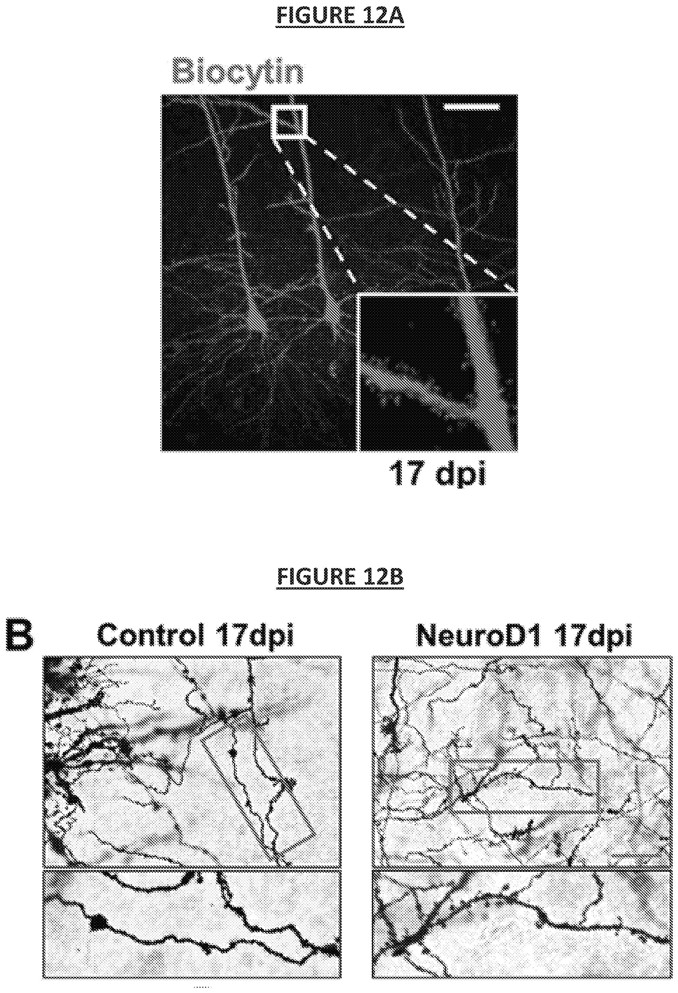

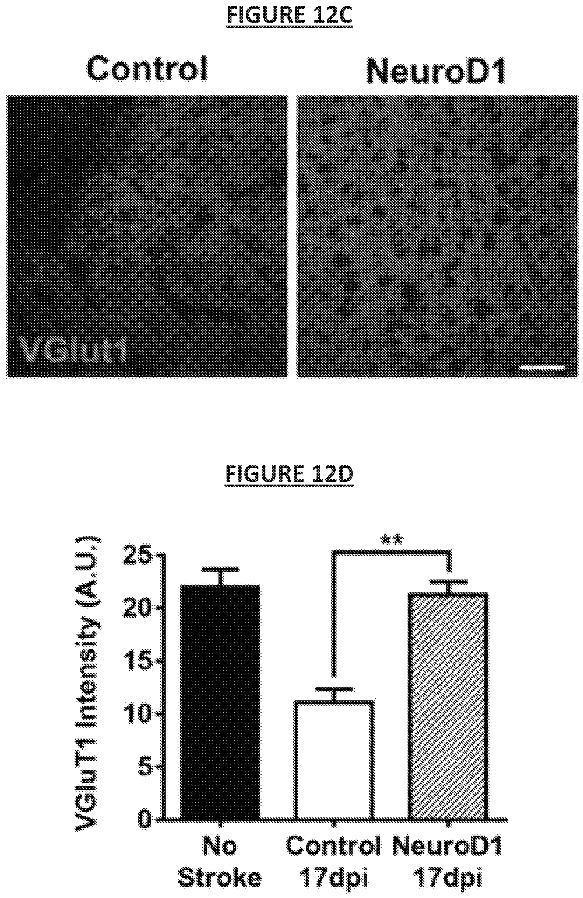

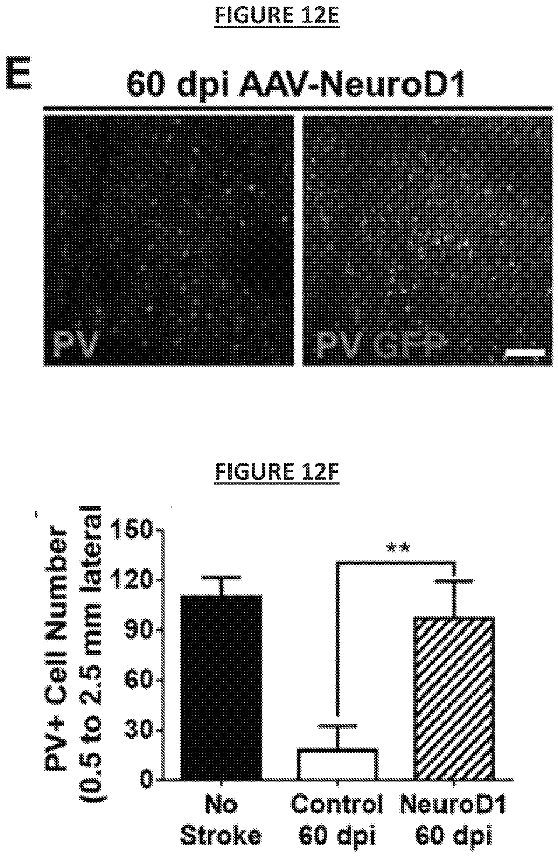

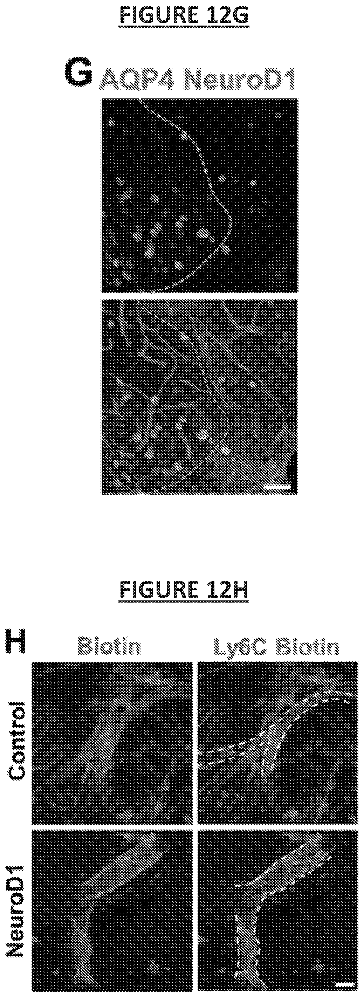

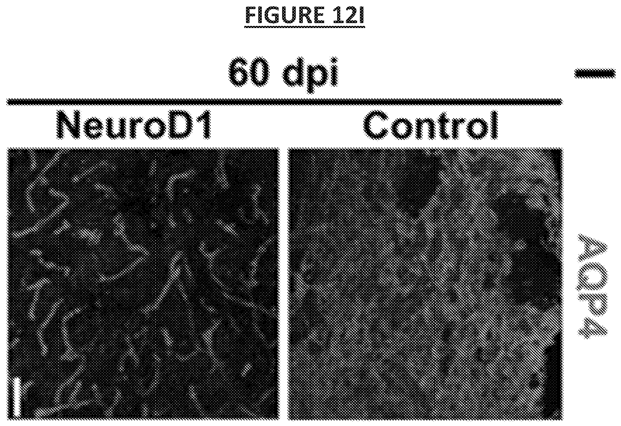

Figures 12A, 12B, 12C, 12D, 12E, 12F, 12G, 12H, and 12I show broad impact of NeuroD1-mediated cell conversion on injured tissue, including synapse recovery and re-balance of excitation-inhibition.
Figure 12A shows images of biocytin, infused during patch-clamp recording and followed with post-fix staining, showing high density of spines along the dendrites in NeuroD1-infected neurons. Scale bar: 50 µm.
Figure 12B shows representative images of Golgi impregnation staining at 17 dpi showed an increase of neuronal processes and dendritic spines in the peri-infarct area of NeuroD1 group compared to controls. Scale bar: 20 µm.
Figure 12C shows representative images of VGluT1 immunofluorescent staining at 17 dpi and Figure 12D is a graph showing quantification of the signal intensity in control and NeuroD1 group. ** P < 0.01, One-way ANOVA followed by Tukey multiple comparison test, n = 3 animals per group. Data are represented as mean ± s.e.m. Scale bar: 40 µm.
Figure 12E shows representative images PV-labeled interneurons in NeuroD1-converted areas and Figure 12F is a graph showing quantification of PV-labeled interneurons in NeuroD1-converted areas. Note that PV neurons were intermingled together with NeuroD1-converted neurons (NeuroD1-GFP) at 60 dpi. Many more PV interneurons were preserved in NeuroD1 group than in the control group. ** P < 0.01. One-way ANOVA followed by Tukey multiple comparison test, n = 3 animals per group. Data are represented as mean ± s.e.m. Scale bar: 100 µm.
Figure 12G is an illustrative image showing that in the NeuroD1 group, the AQP4 signal exhibited clear association with blood vessels in the areas with high density of NeuroD1-expressing cells but less in the neighboring areas with low density of NeuroD1-expressing cells (distinguished by dashed line). Scale bar: 40 µm.
Figure 12H shows representative images of biotin perfusion analysis demonstrating that in the injury areas of control group, biotin leaked outside of the blood vessels (delineated by dashed lines, labeled by Ly6C), while biotin remained largely inside the blood vessels in the injury areas of NeuroD1 group. Scale bar: 5 µm.
Figure 12I shows representative images of astrocytic endfeet marker AQP4 in the peri-infarct areas at 60 dpi. Note a significant re-association of AQP4 with blood vessels in NeuroD1 group. Scale bar: 40 µm.

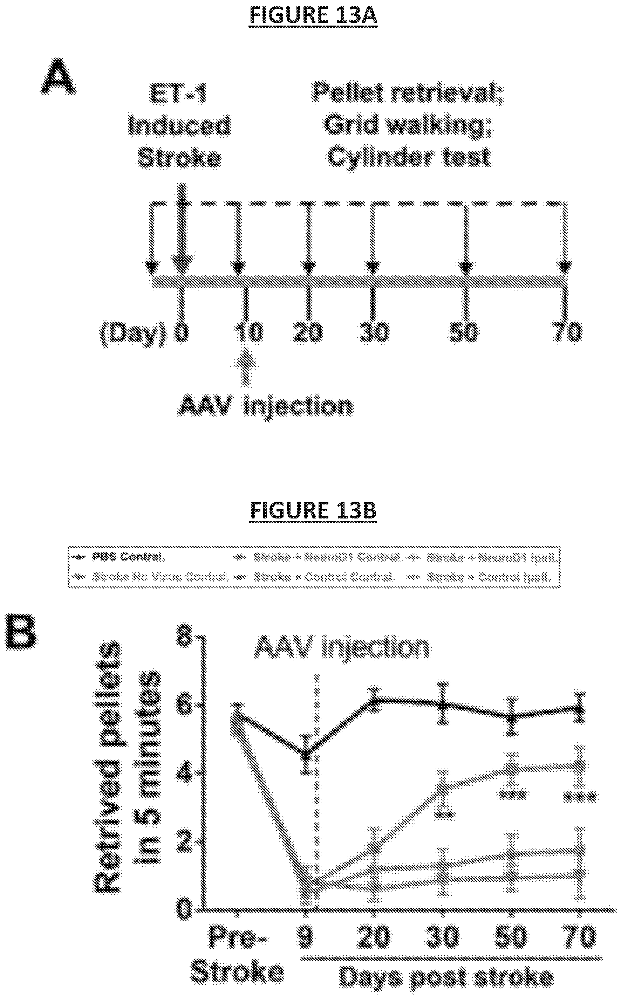

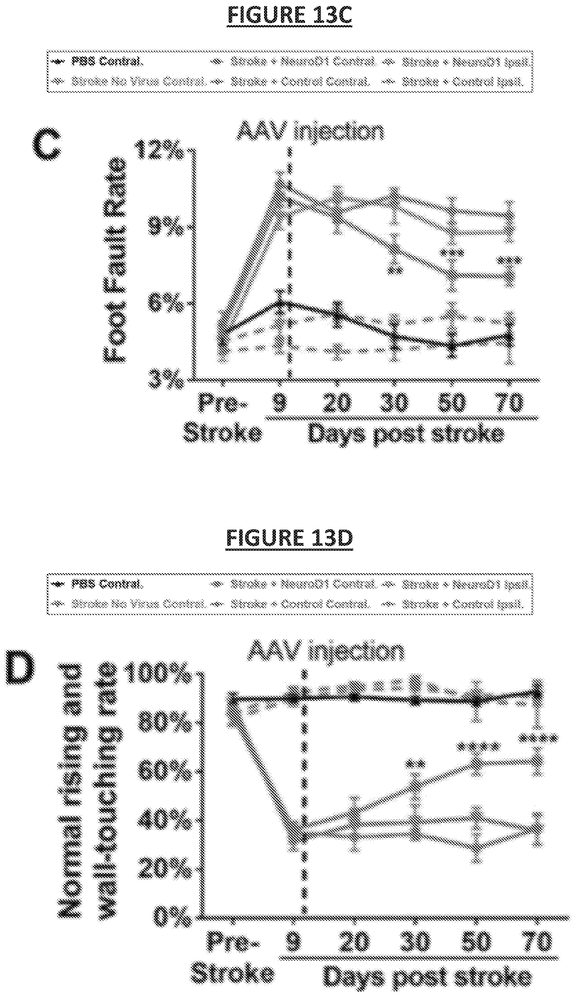

Figures 13A, 13B, 13C, and 13D show behavioral improvement after NeuroD1-treatment.
Figure 13A is a schematic illustration of experimental design for mouse forelimb motor functional tests. At 9 days post stroke (9 dps) and one day prior to viral injection, behavioral tests were performed to assess the motor functions of mice injected with ET-1 (1-31) or PBS as sham controls. AAV were injected at 10 dps, and behavioral tests were performed at the indicated time points (20, 30, 50, and 70 dps) to assess functional recovery.
Figure 13B is a graph showing results of a pellet retrieval test. NeuroD1 group showed accelerated recovery after stroke compared to the control group. Ischemic injury at the motor cortex severely impaired the food pellet retrieval capability, from 5-6 pellets/5 min pre-stroke down to 1 pellet/5 min at 9 dps. After NeuroD1 treatment, pellet retrieval ability showed steady recovery compared to the control group. Note that for food pellet retrieval test, only the motor cortex contralateral to the dominant side of forelimb was stroke injured and tested. Two-way ANOVA followed by Tukey multiple comparison test. n = 12 for ET-1 + control AAV group; n = 11 for NeuroD1 AAV; n = 6 for ET-1 plus no virus group; and n = 6 for PBS group. ** P < 0.01, *** P < 0.001. Data are represented as mean ± s.e.m.
Figure 13C is a graph showing results of a grid walking test. NeuroD1 group showed lower foot fault rate compared to the control group. Stroke injury significantly increased the foot fault rate. NeuroD1 treatment decreased the foot fault rate. Two-way ANOVA followed by Tukey multiple comparison test. n = 9 for ET-1 + control AAV contralateral group; n = 11 for ET-1 + NeuroD1 AAV contralateral group; n = 5 for ET-1 + NeuroD1 ipsilateral group; and n = 5 for ET-1 + control ipsilateral group; n = 6 for ET-1 plus no virus contralateral group; and n = 6 PBS contralateral group. ** P < 0.01, *** P < 0.001. Data are represented as mean ± s.e.m.
Figure 13D is a graph showing results of a cylinder test. NeuroD1-treated mice showed considerable recovery of rising and touching the sidewall function of forelimbs compared to the control mice. Stroke insult impaired the mouse forelimb function in terms of rising and touching the wall of a cylinder. Two-way ANOVA followed by Tukey multiple comparison test. n = 9 for stroke plus control AAV contralateral group; n = 11 for stroke plus NeuroD1 AAV contralateral group; n = 9 for stroke plus NeuroD1 ipsilateral group; n = 7 for stroke plus control ipsilateral group; n = 6 for stroke plus no virus contralateral group, n = 5 for PBS contralateral group. ** P < 0.01, **** P < 0.0001. Data are represented as mean ± s.e.m.

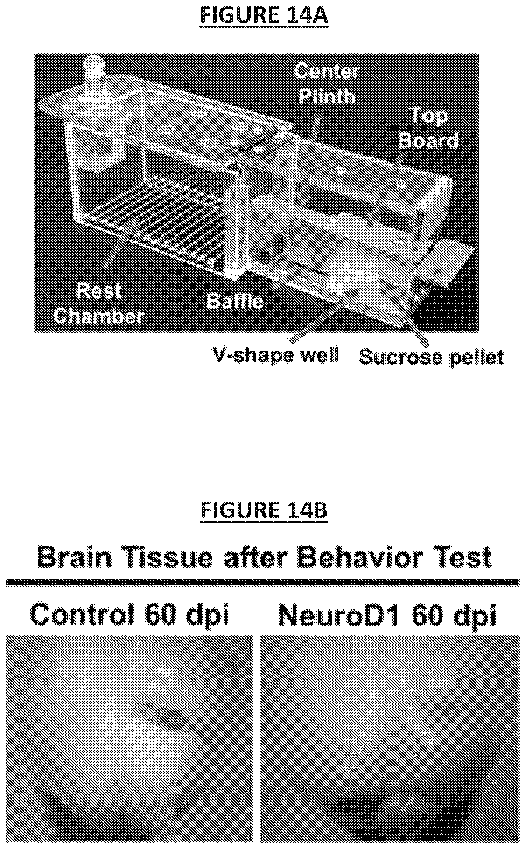

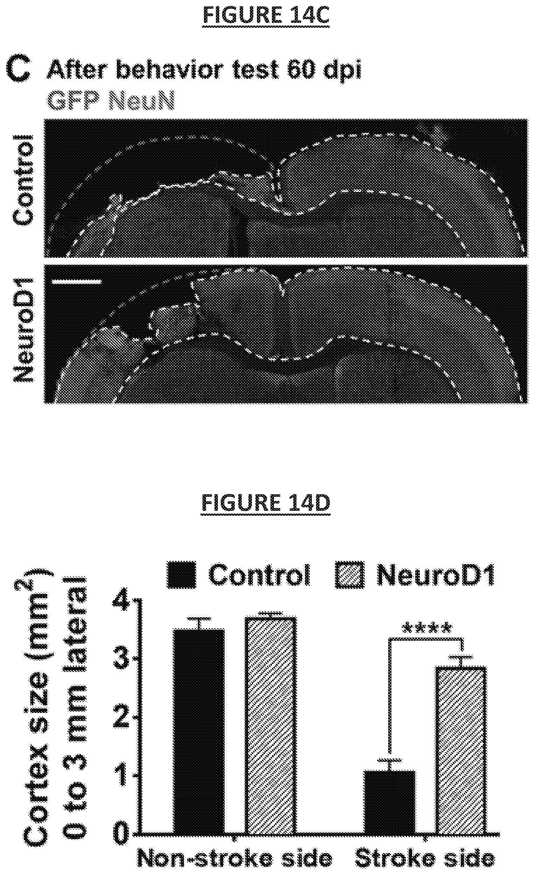

Figures 14A, 14B, 14C, and 14D show assessment of tissue damage after behavioral tests.
Figure 14A is a picture of the pellet retrieval device modified from staircase. The mouse in the rest chamber was trained to reach the sucrose pellet in the V-shape well after 18 hr food deprivation. It is clear from this design that the mice had to spend a great effort with motor coordination and strength to successfully retrieve the food pellet.
Figure 14B shows representative pictures of mouse brains after finishing behavioral tests (2 months post viral injection). The control group showed severe cortical tissue loss on stroke side after ET-1 (1-31)-induced focal stroke, whereas NeuroD1-treatment showed a significant rescue of the tissue loss.
Figure 14C shows representative images of the mouse cortex in coronal sections illustrating the severe tissue loss after ET-1 (1-31) induced ischemic stroke. Note that two ET-1 (1-31) injections were made in both motor cortex and sensory cortex on one side to induce more severe injury for long-lasting behavioral tests. Therefore, the tissue loss is also more severe than the single injection performed for most of the cell conversion studies. Nevertheless, the NeuroD1 repairing effect is still significant. Scale bar: 1000 µm.
Figure 14D is a graph showing quantification of cortical tissue damage after behavioral tests. In NeuroD1 group with two sites of ET-1 (1-31) injection, the stroke side also showed tissue damage compared to its contralateral non-stroke side (P = 0.012), but it was much better preserved than the control group (**** P < 0.0001). Two-way ANOVA followed by Sidak multiple comparison test, n = 6 mice per group. Data are represented as mean ± s.e.m.

### DETAILED DESCRIPTION

Unexpectedly, expression of a neural transcription factor NeuroD1 in reactive astrocytes treats the effects of disruption of normal blood flow in the CNS in an individual subject in need thereof. Thus, provided by the present invention are methods as set out in the appended claims.

Administration of a therapeutically effective amount of NeuroD1 to a subject affected by disruption of normal blood flow in the CNS mediates: the generation of new glutamatergic neurons by conversion of reactive astrocytes to glutamatergic neurons; reduction of the number of reactive astrocytes; survival of injured neurons including GABAergic and glutamatergic neurons; the generation of new non-reactive astrocytes; the reduction of reactivity of non-converted reactive astrocytes; and reintegration of blood vessels into the injured region.

Administration of a therapeutically effective amount of NeuroD1 to a subject affected by disruption of normal blood flow in the CNS mediates: reduced inflammation at the injury site; reduced neuroinhibition at the injury site; re-establishment of normal microglial morphology at the injury site; re-establishment of neural circuits at the injury site, increased blood vessels at the injury site; re-establishment of blood-brain-barrier at the injury site; re-establishment of normal tissue structure at the injury site; and improvement of motor deficits due to the disruption of normal blood flow.

The subject in need of treatment may be human or non-human mammalian, but can be non-mammalian as well.

Surprisingly, administration of a therapeutically effective amount of NeuroD1 to ameliorate the effects of disruption of normal blood flow in the CNS in an individual subject in need thereof has greater beneficial effects when administered to reactive astrocytes than to quiescent astrocytes, typically 3 to 60 days, 5 to 45 days or 8 to 30 days following the onset of disruption of normal blood flow in the CNS of the subject, although certain benefit can be achieved earlier or later, including 2 days to 1 year or later following the onset of interruption of blood flow in the CNS of the subject.

Administration of NeuroD1 to a subject may be used to treat injury arising from disruption of normal blood flow to the CNS, as caused by ischemia, thrombosis, embolism, hemorrhage, concussion, tumor, infection, inflammation, traumatic brain and spinal cord injury, or chronic disease mediated restriction of blood vessels, which results in neuron cell death and activation of reactive astrocytes. Administration of NeuroD1 to a subject may be used to treat injury arising from disruption of normal blood flow to the CNS including, but not limited to, ischemic or hemorrhagic stroke, brain aneurysm, tumor, infection, inflammation, concussion, traumatic brain injury, traumatic spinal injury, ischemic or hemorrhagic myelopathy (spinal cord infarction), global ischemia as caused by cardiac arrest or severe hypotension (shock), hypoxicischemic encephalopathy as caused by hypoxia, hypoglycemia, or anemia, CNS embolism as caused by infective endocarditis or atrial myxoma, fibrocartilaginous embolic myelopathy, CNS thrombosis as caused by pediatric leukemia, cerebral venous sinus thrombosis as caused by nephrotic syndrome (kidney disease), chronic inflammatory disease, pregnancy, use of estrogen-based contraceptives, meningitis, and dehydration.

Methods include administration of a therapeutically effective amount of NeuroD1 to the subject in the region of disruption of normal blood flow in the CNS according to aspects of the present invention. Methods include administration of a therapeutically effective amount of NeuroD1 to the subject at or near the site of disruption of normal blood flow in the CNS according to aspects of the present invention. Methods include administration of a therapeutically effective amount of NeuroD1 to the subject in or near a glial scar caused by disruption of normal blood flow in the CNS according to aspects of the present invention.

NeuroD1 treatment can be administered to the region of injury as diagnosed by MRI. Electrophysiology can assess functional changes in neural firing as caused by neural cell death or injury. Non-invasive methods to assay neural damage include EEG. Disruption of blood flow to a point of injury may be non-invasively assayed via Near Infrared Spectroscopy and fMRI. Blood flow within the region may either be increased, as seen in aneurysms, or decreased, as seen in ischemia. Injury to the CNS caused by disruption of blood flow additionally causes short-term and long-term changes to tissue structure that can be used to diagnose point of injury. In the short term, injury will cause localized swelling. In the long term, cell death will cause points of tissue loss. Non-invasive methods to assay structural changes caused by tissue death include MRI, PET scan, CAT scan, or ultrasound. These methods may be used singularly or in any combination to pinpoint the focus of injury.

In particular aspects according to the present invention, NeuroD1 is administered as set out above and in the appended claims at the periphery of the injury site where a glial scar will develop if the subject is untreated or where a glial scar is already present. Glial scar location may be as determined by assaying tissue structure or function. As described above, non-invasive methods to assay structural changes caused by tissue death include MRI, CAT scan, or ultrasound. Functional assay may include EEG recording.

In particular aspects according to the present invention, NeuroD1 is administered as an expression vector containing a DNA sequence encoding NeuroD1 as set out above and in the appended claims.

According to aspects of the present invention, a viral vector including a nucleic acid encoding NeuroD1 as set out above and in the appended claims is delivered by injection into the central nerve tissue of a subject, such as by intracerebral injection, spinal cord injection and/or injection into the cerebrospinal fluid. An alternative viral delivery is intrathecal injection.

According to aspects of the present invention, a viral vector including a nucleic acid encoding NeuroD1 as set out above and in the appended claims is delivered by stereotactic injection into the brain of a subject.

The term "expression vector" refers to a recombinant vehicle for introducing a nucleic acid encoding NeuroD1 into a host cell *in vitro* or *in vivo* where the nucleic acid is expressed to produce NeuroD1. In particular embodiments, an expression vector including SEQ ID NO: 1 or 3 or a substantially identical nucleic acid sequence is expressed to produce NeuroD1 in cells containing the expression vector. The term "recombinant" is used to indicate a nucleic acid construct in which two or more nucleic acids are linked and which are not found linked in nature. Expression vectors include, but are not limited to plasmids, viruses, BACs and YACs. Particular viral expression vectors illustratively include those derived from adenovirus, adeno-associated virus, retrovirus, and lentivirus.

Method are provided according to aspects of the present invention which include providing a viral vector comprising a nucleic acid encoding NeuroD1 as set out above and in the appended claims; and delivering the viral vector to the central nervous system of the subject, whereby the viral vector infects glial cells of the central nervous system, respectively, producing infected glial cells and whereby exogenous NeuroD1 is expressed in the infected glial cells at a therapeutically effective level, wherein the expression of NeuroD1 in the infected cells results in a greater number of neurons in the subject compared to an untreated subject having the same neurological condition, whereby the neurological condition is treated. In addition to the generation of new neurons, the number of reactive glial cells will also be reduced, resulting in less neuroinhibitory factors released, less neuroinflammation, more blood vessels that are also evenly distributed, thereby making local environment more permissive to neuronal growth or axon penetration, hence alleviating neurological conditions.

Adeno-associated vectors are particularly useful in methods according to aspects of the present invention and will infect both dividing and non-dividing cells, at an injection site. Adeno-associated viruses (AAV) are ubiquitous, noncytopathic, replication-incompetent members of ssDNA animal virus of parvoviridae family. According to aspects of the present invention, any of various recombinant adeno-associated viruses, such as serotypes 1-9, can be used.

A "FLEX" switch approach is used to express NeuroD1 in infected cells according to aspects of the present invention. The terms "FLEX" and "flip-excision" are used interchangeably to indicate a method in which two pairs of heterotypic, antiparallel loxP-type recombination sites are disposed on either side of an inverted NeuroD1 coding sequence which first undergo an inversion of the coding sequence followed by excision of two sites, leading to one of each orthogonal recombination site oppositely oriented and incapable of further recombination, achieving stable inversion, see for example Schnutgen et al., Nature Biotechnology 21:562-565, 2003; and Atasoy et al, J. Neurosci., 28:7025-7030, 2008. Since the site-specific recombinase under control of a glial cell-specific promoter will be strongly expressed in glial cells, including reactive astrocytes, NeuroD1 will also be expressed in glial cells, including reactive astrocytes. Then, when the stop codon in front of NeuroD1 is removed from recombination, the constitutive or neuron-specific promoter will drive high expression of NeuroD1, allowing reactive astrocytes to be converted into functional neurons.

Site-specific recombinases and their recognition sites include, for example, Cre recombinase along with recognition sites loxP and lox2272 sites, or FLP-FRT recombination, or their combinations.

In order to achieve optimal infection, a concentration of 10¹⁰-10¹⁴ adeno-associated virus particles/ml, 1-500 µl of volume, should be injected at a controlled flow rate of 0.1-5 µl/min.

According to aspects of the present invention, an adeno-associated virus vector including a nucleic acid encoding NeuroD1 as set out above and in the appended claims wherein the DNA sequence encoding NeuroD1 is inverted and in the wrong orientation for expression of NeuroD1 and further includes sites for recombinase activity by a site specific recombinase, until the site-specific recombinase inverts the inverted DNA sequence encoding NeuroD1, thereby allowing expression of NeuroD1, is delivered by stereotactic injection into the brain of a subject along with an adeno-associated virus encoding a site specific recombinase.

According to aspects of the present invention, an adeno-associated virus vector including a nucleic acid encoding NeuroD1 as set out above and in the appended claims wherein the DNA sequence encoding NeuroD1 is inverted and in the wrong orientation for expression of NeuroD1 and further includes sites for recombinase activity by a site specific recombinase, until the site-specific recombinase inverts the inverted DNA sequence encoding NeuroD1, thereby allowing expression of NeuroD1, is delivered by stereotactic injection into the brain of a subject along with an adeno-associated virus encoding a site specific recombinase in the region of or at the site of disruption of normal blood flow in the CNS according to aspects of the present invention. Optionally, the site of stereotactic injection is in or near a glial scar caused by disruption of normal blood flow in the CNS.

According to aspects of the present invention, the site-specific recombinase is Cre recombinase and the sites for recombinase activity are recognition sites loxP and lox2272 sites.

According to aspects of the present invention, NeuroD1 treatment of a subject as set out above and in the appended claims is monitored during or after treatment to monitor progress and/or final outcome of the treatment. Post-Treatment Assay for successful neuronal cell integration and restoration of tissue microenvironment is diagnosed by restoration or near-restoration of normal electrophysiology, blood flow, tissue structure, and function. Non-invasive methods to assay neural function include EEG. Blood flow may be non-invasively assayed via Near Infrared Spectroscopy and fMRI. Non-invasive methods to assay tissue structure include MRI, CAT scan, PET scan, or ultrasound. Behavioral assays may be used to non-invasively assay for restoration of CNS function. The behavioral assay should be matched to the loss of function caused by original CNS injury. For example, if injury caused paralysis, the patient's mobility and limb dexterity should be tested. If injury caused loss or slowing of speech, patient's ability to communicate via spoken word should be assayed. Restoration of normal behavior post NeuroD1 treatment indicates successful creation and integration of effective neuronal circuits. These methods may be used singularly or in any combination to assay for neural function and tissue health. Assays to evaluate treatment may be performed at any point, such as 1 day, 2 days, 3 days, one week, 2 weeks, 3 weeks, one month, or later, after NeuroD1 treatment. Such assays may be performed prior to NeuroD1 treatment in order to establish a baseline comparison if desired.

Scientific and technical terms used herein are intended to have the meanings commonly understood by those of ordinary skill in the art. Such terms are found defined and used in context in various standard references illustratively including J. Sambrook and D.W. Russell, Molecular Cloning: A Laboratory Manual, Cold Spring Harbor Laboratory Press; 3rd Ed., 2001; F.M. Asubel, Ed., Short Protocols in Molecular Biology, Current Protocols; 5th Ed., 2002; B. Alberts et al., Molecular Biology of the Cell, 4th Ed., Garland, 2002; D.L. Nelson and M.M. Cox, Lehninger Principles of Biochemistry, 4th Ed., W.H. Freeman & Company, 2004; Engelke, D.R., RNA Interference (RNAi): Nuts and Bolts of RNAi Technology, DNA Press LLC, Eagleville, PA, 2003; Herdewijn, p. (Ed.), Oligonucleotide Synthesis: Methods and Applications, Methods in Molecular Biology, Humana Press, 2004; A. Nagy, M. Gertsenstein, K. Vintersten, R. Behringer, Manipulating the Mouse Embryo: A Laboratory Manual, Cold Spring Harbor Laboratory Press,3rd Ed.; December 15, 2002,ISBN-10:0879695919; Kursad Turksen (Ed.), Embryonic Stem Cells: Methods and Protocols in Methods in Molecular Biology, 2002; 185, Human Press: Current Protocols in Stem Cell Biology, ISBN:9780470151808.

The singular terms "a," "an," and "the" are not intended to be limiting and include plural referents unless explicitly stated otherwise or the context clearly indicates otherwise.

The term "NeuroD1 protein" refers to a bHLH proneural transcription factor involved in embryonic brain development and in adult neurogenesis, see Cho, J.H. et al., Mol, Neurobiol., 30:35-47, 2004; Kuwabara, T. et al., Nature Neurosci., 12:1097-1105, 2009; and Gao, Z. et al., Nature Neurosci., 12:1090-1092, 2009. NeuroD1 is expressed late in development, mainly in the nervous system and is involved in neuronal differentiation, maturation and survival.

The term "NeuroD1 protein" encompasses human NeuroD1 protein, identified here as SEQ ID NO: 2 and mouse NeuroD1 protein, identified here as SEQ ID NO: 4. In addition to the NeuroD1 protein of SEQ ID NO: 2 and SEQ ID NO: 4, the term "NeuroD1 protein" encompasses variants of NeuroD1 protein, such as variants of SEQ ID NO: 2 and SEQ ID NO: 4, which may be included in methods of the present invention. As used herein, the term "variant" refers to naturally occurring genetic variations and recombinantly prepared variations, each of which contain one or more changes in its amino acid sequence compared to a reference NeuroD1 protein, such as SEQ ID NO: 2 or SEQ ID NO: 4. Such changes include those in which one or more amino acid residues have been modified by amino acid substitution, addition or deletion. The term "variant" encompasses orthologs of human NeuroD1, including for example mammalian and bird NeuroD1, such as, but not limited to NeuroD1 orthologs from a non-human primate, cat, dog, sheep, goat, horse, cow, pig, bird, poultry animal and rodent such as but not limited to mouse and rat. In a non-limiting example, mouse NeuroD1, exemplified herein as amino acid sequence SEQ ID NO: 4 is an ortholog of human NeuroD1.

Preferred variants have at least 75%, 80%, 85%, 90%, 91%, 92%, 93%, 94%, 95%, 96%, 97%, 98%, or 99% identity to SEQ ID NO: 2 or SEQ ID NO: 4.

Mutations can be introduced using standard molecular biology techniques, such as site-directed mutagenesis and PCR-mediated mutagenesis. One of skill in the art will recognize that one or more amino acid mutations can be introduced without altering the functional properties of the NeuroD1 protein. For example, one or more amino acid substitutions, additions, or deletions can be made without altering the functional properties of the NeuroD1 protein of SEQ ID NO: 2 or 4.

Conservative amino acid substitutions can be made in a NeuroD1 protein to produce a NeuroD1 protein variant. Conservative amino acid substitutions are art recognized substitutions of one amino acid for another amino acid having similar characteristics. For example, each amino acid may be described as having one or more of the following characteristics: electropositive, electronegative, aliphatic, aromatic, polar, hydrophobic and hydrophilic. A conservative substitution is a substitution of one amino acid having a specified structural or functional characteristic for another amino acid having the same characteristic. Acidic amino acids include aspartate, glutamate; basic amino acids include histidine, lysine, arginine; aliphatic amino acids include isoleucine, leucine and valine; aromatic amino acids include phenylalanine, glycine, tyrosine and tryptophan; polar amino acids include aspartate, glutamate, histidine, lysine, asparagine, glutamine, arginine, serine, threonine and tyrosine; and hydrophobic amino acids include alanine, cysteine, phenylalanine, glycine, isoleucine, leucine, methionine, proline, valine and tryptophan; and conservative substitutions include substitution among amino acids within each group. Amino acids may also be described in terms of relative size, alanine, cysteine, aspartate, glycine, asparagine, proline, threonine, serine, valine, all typically considered to be small.

NeuroD1 variants can include synthetic amino acid analogs, amino acid derivatives and/or non-standard amino acids, illustratively including, without limitation, alpha-aminobutyric acid, citrulline, canavanine, cyanoalanine, diaminobutyric acid, diaminopimelic acid, dihydroxy-phenylalanine, djenkolic acid, homoarginine, hydroxyproline, norleucine, norvaline, 3-phosphoserine, homoserine, 5-hydroxytryptophan, 1-methylhistidine, 3-methylhistidine, and ornithine.

To determine the percent identity of two amino acid sequences or of two nucleic acid sequences, the sequences are aligned for optimal comparison purposes (e.g., gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (*i.e.,* % identity=number of identical overlapping positions/total number of positions X 100%). In one embodiment, the two sequences are the same length.

The determination of percent identity between two sequences can also be accomplished using a mathematical algorithm. A preferred, non-limiting example of a mathematical algorithm utilized for the comparison of two sequences is the algorithm of Karlin and Altschul, 1990, PNAS 87:2264 2268, modified as in Karlin and Altschul, 1993, PNAS. 90:5873 5877. Such an algorithm is incorporated into the NBLAST and XBLAST programs of Altschul et al., 1990, J. Mol. Biol. 215:403. BLAST nucleotide searches are performed with the NBLAST nucleotide program parameters set, e.g., for score=100, wordlength=12 to obtain nucleotide sequences homologous to a nucleic acid molecules of the present invention.

BLAST protein searches are performed with the XBLAST program parameters set, e.g., to score 50, wordlength=3 to obtain amino acid sequences homologous to a protein molecule of the present invention. To obtain gapped alignments for comparison purposes, Gapped BLAST are utilized as described in Altschul et al., 1997, Nucleic Acids Res. 25:3389 3402. Alternatively, PSI BLAST is used to perform an iterated search which detects distant relationships between molecules. When utilizing BLAST, Gapped BLAST, and PSI Blast programs, the default parameters of the respective programs (*e.g.,* of XBLAST and NBLAST) are used (see, *e.g.,* the NCBI website).

Another preferred, non-limiting example of a mathematical algorithm utilized for the comparison of sequences is the algorithm of Myers and Miller, 1988, CABIOS 4:11 17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 is used.

The percent identity between two sequences is determined using techniques similar to those described above, with or without allowing gaps. In calculating percent identity, typically only exact matches are counted.

The term "NeuroD1 protein" encompasses fragments of the NeuroD1 protein, such as fragments of SEQ ID NOs. 2 and 4 and variants thereof, operable in methods and compositions of the present invention.

NeuroD1 proteins and nucleic acids may be isolated from natural sources, such as the brain of an organism or cells of a cell line which expresses NeuroD1. Alternatively, NeuroD1 protein or nucleic acid may be generated recombinantly, such as by expression using an expression construct, *in vitro* or *in vivo.* NeuroD1 proteins and nucleic acids may also be synthesized by well-known methods.

NeuroD1 included in methods and compositions of the present invention is preferably produced using recombinant nucleic acid technology. Recombinant NeuroD1 production includes introducing a recombinant expression vector encompassing a DNA sequence encoding NeuroD1 into a host cell.

A nucleic acid sequence encoding NeuroD1 introduced into a host cell to produce NeuroD1 according to embodiments of the invention encodes SEQ ID NO: 2, SEQ ID NO: 4, or a variant thereof.

According to aspects of the present invention, the nucleic acid sequence identified herein as SEQ ID NO: 1 encodes SEQ ID NO: 2 and is included in an expression vector and expressed to produce NeuroD1. According to aspects of the present invention, the nucleic acid sequence identified herein as SEQ ID NO: 3 encodes SEQ ID NO: 4 and is included in an expression vector and expressed to produce NeuroD1.

It is appreciated that due to the degenerate nature of the genetic code, nucleic acid sequences substantially identical to SEQ ID NOs. 1 and 3 encode NeuroD1 and variants of NeuroD1, and that such alternate nucleic acids may be included in an expression vector and expressed to produce NeuroD1 and variants of NeuroD1. One of skill in the art will appreciate that a fragment of a nucleic acid encoding NeuroD1 protein can be used to produce a fragment of a NeuroD1 protein.

An expression vector contains a nucleic acid that includes segment encoding a polypeptide of interest operably linked to one or more regulatory elements that provide for transcription of the segment encoding the polypeptide of interest. The term "operably linked" as used herein refers to a nucleic acid in functional relationship with a second nucleic acid. The term "operably linked" encompasses functional connection of two or more nucleic acid molecules, such as a nucleic acid to be transcribed and a regulatory element. The term "regulatory element" as used herein refers to a nucleotide sequence which controls some aspect of the expression of an operably linked nucleic acid. Exemplary regulatory elements include an enhancer, such as, but not limited to: woodchuck hepatitis virus posttranscriptional regulatory element (WPRE); an internal ribosome entry site (IRES) or a 2A domain; an intron; an origin of replication; a polyadenylation signal (pA); a promoter; a transcription termination sequence; and an upstream regulatory domain, which contribute to the replication, transcription, post-transcriptional processing of an operably linked nucleic acid sequence. Those of ordinary skill in the art are capable of selecting and using these and other regulatory elements in an expression vector with no more than routine experimentation.

The term "promoter" as used herein refers to a DNA sequence operably linked to a nucleic acid sequence to be transcribed such as a nucleic acid sequence encoding NeuroD1. A promoter is generally positioned upstream of a nucleic acid sequence to be transcribed and provides a site for specific binding by RNA polymerase and other transcription factors. In specific embodiments, a promoter is generally positioned upstream of the nucleic acid sequence transcribed to produce the desired molecule, and provides a site for specific binding by RNA polymerase and other transcription factors.

As will be recognized by the skilled artisan, the 5' non-coding region of a gene can be isolated and used in its entirety as a promoter to drive expression of an operably linked nucleic acid. Alternatively, a portion of the 5' non-coding region can be isolated and used to drive expression of an operably linked nucleic acid. In general, about 500-6000 bp of the 5' non-coding region of a gene is used to drive expression of the operably linked nucleic acid. Optionally, a portion of the 5' non-coding region of a gene containing a minimal amount of the 5' non-coding region needed to drive expression of the operably linked nucleic acid is used. Assays to determine the ability of a designated portion of the 5' non-coding region of a gene to drive expression of the operably linked nucleic acid are well-known in the art.

The nucleic acid encoding NeuroD1 is operably linked to a human GFAP promoter sequence. Human GFAP promoter is shown herein as SEQ ID NO:6.

A nucleic acid sequence which is substantially identical to SEQ ID NO: 1 or SEQ ID NO:3 is characterized as having a complementary nucleic acid sequence capable of hybridizing to SEQ ID NO: 1 or SEQ ID NO:3 under high stringency hybridization conditions.

In addition to one or more nucleic acids encoding NeuroD1, one or more nucleic acid sequences encoding additional proteins can be included in an expression vector. For example, such additional proteins include non-NeuroD1 proteins such as reporters, including, but not limited to, beta-galactosidase, green fluorescent protein and antibiotic resistance reporters.

In particular embodiments, the recombinant expression vector encodes at least NeuroD1 of SEQ ID NO:2, a protein having at least 95% identity to SEQ ID NO:2, or a protein encoded by a nucleic acid sequence substantially identical to SEQ ID NO: 1.

In particular embodiments, the recombinant expression vector as set out above and in the appended claims encodes at least NeuroD1 of SEQ ID NO:4, a protein having at least 95% identity to SEQ ID NO:4, or a protein encoded by a nucleic acid sequence substantially identical to SEQ ID NO:2.

SEQ ID NO:9 is an example of a nucleic acid including CAG promoter operably linked to a nucleic acid encoding NeuroD1, and further including a nucleic acid sequence encoding EGFP and an enhancer, WPRE. An IRES separates the nucleic acid encoding NeuroD1 and the nucleic acid encoding EGFP. SEQ ID NO:9 is inserted into an expression vector for expression of NeuroD1 and the reporter gene EGFP. Optionally, the IRES and nucleic acid encoding EGFP are removed and the remaining CAG promoter and operably linked nucleic acid encoding NeuroD1 is inserted into an expression vector for expression of NeuroD1. The WPRE or another enhancer is optionally included.

Optionally, a reporter gene is included in a recombinant expression vector encoding NeuroD1. A reporter gene may be included to produce a peptide or protein that serves as a surrogate marker for expression of NeuroD1 from the recombinant expression vector. The term "reporter gene" as used herein refers to gene that is easily detectable when expressed, for example by chemiluminescence, fluorescence, colorimetric reactions, antibody binding, inducible markers and/or ligand binding assays. Exemplary reporter genes include, but are not limited to, green fluorescent protein (GFP), enhanced green fluorescent protein (eGFP), yellow fluorescent protein (YFP), enhanced yellow fluorescent protein (eYFP), cyan fluorescent protein (CFP), enhanced cyan fluorescent protein (eCFP), blue fluorescent protein (BFP), enhanced blue fluorescent protein (eBFP), MmGFP (Zernicka-Goetz et al., Development, 124:1133-1137, 1997, dsRed, luciferase and beta-galactosidase (lacZ).

The process of introducing genetic material into a recipient host cell, such as for transient or stable expression of a desired protein encoded by the genetic material in the host cell is referred to as "transfection." Transfection techniques are well-known in the art and include, but are not limited to, electroporation, particle accelerated transformation also known as "gene gun" technology, liposome-mediated transfection, calcium phosphate or calcium chloride coprecipitation-mediated transfection, DEAE-dextran-mediated transfection, microinjection, polyethylene glycol mediated transfection, heat shock mediated transfection and virus-mediated transfection. As noted herein, virus-mediated transfection may be accomplished using a viral vector such as those derived from adenovirus, adeno-associated virus and lentivirus.

Optionally, a host cell is transfected ex-vivo and then re-introduced into a host organism. For example, cells or tissues may be removed from a subject, transfected with an expression vector encoding NeuroD1 and then returned to the subject.

Introduction of a recombinant expression vector including a nucleic acid encoding NeuroD1, or a functional fragment thereof, into a host glial cell *in vitro* or *in vivo* for expression of exogenous NeuroD1 in the host glial cell to convert the glial cell to a neuron is accomplished by any of various transfection methodologies.

Expression of exogenous NeuroD1 in the host glial cell to convert the glial cell to a neuron is optionally achieved by introduction of mRNA encoding NeuroD1, or a functional fragment thereof, to the host glial cell *in vitro* or *in vivo.*

Expression of exogenous NeuroD1 in the host glial cell to convert the glial cell to a neuron is optionally achieved by introduction of NeuroD1 protein to the host glial cell *in vitro* or *in vivo.* Details of these and other techniques are known in the art, for example, as described in J. Sambrook and D.W. Russell, Molecular Cloning: A Laboratory Manual, Cold Spring Harbor Laboratory Press; 3rd Ed., 2001; F.M. Ausubel, Ed., Short Protocols in Molecular Biology, Current Protocols; 5th Ed., 2002; and Engelke, D.R., RNA Interference (RNAi): Nuts and Bolts of RNAi Technology, DNA Press LLC, Eagleville, PA, 2003.

An expression vector including a nucleic acid encoding NeuroD1 or a functional fragment thereof, mRNA encoding NeuroD1 or a functional fragment thereof, and/or NeuroD1 protein, full-length or a functional fragment thereof, is optionally associated with a carrier for introduction into a host cell *in vitro* or *in vivo.*

In particular aspects, the carrier is a particulate carrier such as lipid particles including liposomes, micelles, unilamellar or mulitlamellar vesicles; polymer particles such as hydrogel particles, polyglycolic acid particles or polylactic acid particles; inorganic particles such as calcium phosphate particles such as described in for example U.S. Patent No. 5,648,097; and inorganic/organic particulate carriers such as described for example in U.S. Patent No. 6,630,486.

A particulate carrier can be selected from among a lipid particle; a polymer particle; an inorganic particle; and an inorganic/organic particle. A mixture of particle types can also be included as a particulate pharmaceutically acceptable carrier.

A particulate carrier is typically formulated such that particles have an average particle size in the range of about 1 nm - 10 microns. In particular aspects, a particulate carrier is formulated such that particles have an average particle size in the range of about 1 nm - 100 nm.

Further description of liposomes and methods relating to their preparation and use may be found in Liposomes: A Practical Approach (The Practical Approach Series, 264), V. P. Torchilin and V. Weissig (Eds.), Oxford University Press; 2nd ed., 2003. Further aspects of nanoparticles are described in S.M. Moghimi et al., FASEB J. 2005, 19, 311-30.

Expression of NeuroD1 using a recombinant expression vector is accomplished by introduction of the expression vector into a eukaryotic or prokaryotic host cell expression system such as an insect cell, mammalian cell, yeast cell, bacterial cell or any other single or multicellular organism recognized in the art. Host cells are optionally primary cells or immortalized derivative cells. Immortalized cells are those which can be maintained in-vitro for at least 5 replication passages.

Host cells containing the recombinant expression vector are maintained under conditions wherein NeuroD1 is produced. Host cells may be cultured and maintained using known cell culture techniques such as described in Celis, Julio, ed., 1994, Cell Biology Laboratory Handbook, Academic Press, N.Y. Various culturing conditions for these cells, including media formulations with regard to specific nutrients, oxygen, tension, carbon dioxide and reduced serum levels, can be selected and optimized by one of skill in the art.

According to aspects of the present invention, a recombinant expression vector including a nucleic acid encoding NeuroD1 as set above and in the appended claims is introduced into glial cells of a subject. Expression of exogenous NeuroD1 in the glial cells "converts" the glial cells into neurons.

According to aspects of the present invention, a recombinant expression vector including a nucleic acid encoding NeuroD1 or a functional fragment thereof as set above and in the appended claims is introduced into astrocytes of a subject. Expression of exogenous NeuroD1 in the glial cells "converts" the astrocytes into neurons.

According to aspects of the present invention, a recombinant expression vector including a nucleic acid encoding NeuroD1 or a functional fragment thereof as set above and in the appended claims is introduced into reactive astrocytes of a subject. Expression of exogenous NeuroD1 or a functional fragment thereof in the reactive astrocytes "converts" the reactive astrocytes into neurons.

According to aspects of the present invention, a recombinant expression vector including a nucleic acid encoding NeuroD1 or a functional fragment thereof as set above and in the appended claims is introduced into NG2 cells of a subject. Expression of exogenous NeuroD1 or a functional fragment thereof in the NG2 cells "converts" the NG2 cells into neurons.

Detection of expression of exogenous NeuroD1 following introduction of a recombinant expression vector including a nucleic acid encoding the exogenous NeuroD1 or a functional fragment thereof is accomplished using any of various standard methodologies including, but not limited to, immunoassays to detect NeuroD1, nucleic acid assays to detect NeuroD1 nucleic acids and detection of a reporter gene co-expressed with the exogenous NeuroD1.

The terms "converts" and "converted" are used herein to describe the effect of expression of NeuroD1 or a functional fragment thereof resulting in a change of a glial cell, astrocyte or reactive astrocyte phenotype to a neuronal phenotype. Similarly, the phrases "NeuroD1 converted neurons" and "converted neurons" are used herein to designate a cell including exogenous NeuroD1 protein or a functional fragment thereof which has consequent neuronal phenotype.

The term "phenotype" refers to well-known detectable characteristics of the cells referred to herein. The neuronal phenotype can be, but is not limited to, one or more of: neuronal morphology, expression of one or more neuronal markers, electrophysiological characteristics of neurons, synapse formation and release of neurotransmitter. For example, neuronal phenotype encompasses but is not limited to: characteristic morphological aspects of a neuron such as presence of dendrites, an axon and dendritic spines; characteristic neuronal protein expression and distribution, such as presence of synaptic proteins in synaptic puncta, presence of MAP2 in dendrites; and characteristic electrophysiological signs such as spontaneous and evoked synaptic events.

In a further example, glial phenotype such as astrocyte phenotype and reactive astrocyte phenotypes encompasses but is not limited to: characteristic morphological aspects of astrocytes and reactive astrocytes such as a generally "star-shaped" morphology; and characteristic astrocyte and reactive astrocyte protein expression, such as presence of glial fibrillary acidic protein (GFAP).

The term "nucleic acid" refers to RNA or DNA molecules having more than one nucleotide in any form including single-stranded, double-stranded, oligonucleotide or polynucleotide. The term "nucleotide sequence" refers to the ordering of nucleotides in an oligonucleotide or polynucleotide in a single-stranded form of nucleic acid.

The term "NeuroD1 nucleic acid" refers to an isolated NeuroD1 nucleic acid molecule and encompasses isolated NeuroD1 nucleic acids having a sequence that has at least 70%, 75%, 80%, 85%, 90%, 91%, 92%, 93%, 94%, 95%, 96%, 97%, 98%, or 99% identity to the DNA sequence set forth in SEQ ID NO:1 or SEQ ID NO:3, or the complement thereof, or a fragment thereof, or an isolated DNA molecule having a sequence that hybridizes under high stringency hybridization conditions to the nucleic acid set forth as SEQ ID NO:1 or SEQ ID NO:3, a complement thereof or a fragment thereof.

The nucleic acid of SEQ ID NO:3 is an example of an isolated DNA molecule having a sequence that hybridizes under high stringency hybridization conditions to the nucleic acid set forth in SEQ ID NO:1. A fragment of a NeuroD1 nucleic acid is any fragment of a NeuroD1 nucleic acid that is operable including a NeuroD1 nucleic acid.

A nucleic acid probe or primer able to hybridize to a target NeuroD1 mRNA or cDNA can be used for detecting and/or quantifying mRNA or cDNA encoding NeuroD1 protein. A nucleic acid probe can be an oligonucleotide of at least 10, 15, 30, 50 or 100 nucleotides in length and sufficient to specifically hybridize under stringent conditions to NeuroD1 mRNA or cDNA or complementary sequence thereof. A nucleic acid primer can be an oligonucleotide of at least 10, 15 or 20 nucleotides in length and sufficient to specifically hybridize under stringent conditions to the mRNA or cDNA, or complementary sequence thereof.

The terms "complement" and "complementary" refers to Watson-Crick base pairing between nucleotides and specifically refers to nucleotides hydrogen bonded to one another with thymine or uracil residues linked to adenine residues by two hydrogen bonds and cytosine and guanine residues linked by three hydrogen bonds. In general, a nucleic acid includes a nucleotide sequence described as having a "percent complementarity" to a specified second nucleotide sequence. For example, a nucleotide sequence may have 80%, 90%, or 100% complementarity to a specified second nucleotide sequence, indicating that 8 of 10, 9 of 10 or 10 of 10 nucleotides of a sequence are complementary to the specified second nucleotide sequence. For instance, the nucleotide sequence 3'-TCGA-5' is 100% complementary to the nucleotide sequence 5'-AGCT-3'. Further, the nucleotide sequence 3'-TCGA- is 100% complementary to a region of the nucleotide sequence 5'-TTAGCTGG-3'.

The terms "hybridization" and "hybridizes" refer to pairing and binding of complementary nucleic acids. Hybridization occurs to varying extents between two nucleic acids depending on factors such as the degree of complementarity of the nucleic acids, the melting temperature, Tm, of the nucleic acids and the stringency of hybridization conditions, as is well known in the art. The term "stringency of hybridization conditions" refers to conditions of temperature, ionic strength, and composition of a hybridization medium with respect to particular common additives such as formamide and Denhardt's solution.

Determination of particular hybridization conditions relating to a specified nucleic acid is routine and is well known in the art, for instance, as described in J. Sambrook and D.W. Russell, Molecular Cloning: A Laboratory Manual, Cold Spring Harbor Laboratory Press; 3rd Ed., 2001; and F.M. Ausubel, Ed., Short Protocols in Molecular Biology, Current Protocols; 5th Ed., 2002. High stringency hybridization conditions are those which only allow hybridization of substantially complementary nucleic acids. Typically, nucleic acids having about 85-100% complementarity are considered highly complementary and hybridize under high stringency conditions. Intermediate stringency conditions are exemplified by conditions under which nucleic acids having intermediate complementarity, about 50-84% complementarity, as well as those having a high degree of complementarity, hybridize. In contrast, low stringency hybridization conditions are those in which nucleic acids having a low degree of complementarity hybridize.

The terms "specific hybridization" and "specifically hybridizes" refer to hybridization of a particular nucleic acid to a target nucleic acid without substantial hybridization to nucleic acids other than the target nucleic acid in a sample.

Stringency of hybridization and washing conditions depends on several factors, including the Tm of the probe and target and ionic strength of the hybridization and wash conditions, as is well-known to the skilled artisan. Hybridization and conditions to achieve a desired hybridization stringency are described, for example, in Sambrook et al., Molecular Cloning: A Laboratory Manual, Cold Spring Harbor Laboratory Press, 2001; and Ausubel, F. et al., (Eds.), Short Protocols in Molecular Biology, Wiley, 2002.

An example of high stringency hybridization conditions is hybridization of nucleic acids over about 100 nucleotides in length in a solution containing 6X SSC, 5X Denhardt's solution, 30% formamide, and 100 micrograms/ml denatured salmon sperm at 37°C overnight followed by washing in a solution of 0.1X SSC and 0.1% SDS at 60°C for 15 minutes. SSC is 0.15M NaCl/0.015M Na citrate. Denhardt's solution is 0.02% bovine serum albumin/0.02% FICOLL/0.02% polyvinylpyrrolidone. Under highly stringent conditions, SEQ ID NO:1 and SEQ ID NO:3 will hybridize to the complement of substantially identical targets and not to unrelated sequences.

Methods are provided according to aspects of the present invention which include delivering a therapeutically effective amount of NeuroD1 as set out above and in the appended claims to glial cells of the central nervous system, the therapeutically effective amount of NeuroD1 in the glial cells results in a greater number of neurons in the subject compared to an untreated subject having the same neurological condition, whereby the neurological condition is treated.

The conversion of reactive glial cells into neurons also reduces neuroinflammation and neuroinhibitory factors associated with reactive glial cells, thereby making the glial scar tissue more permissive to neuronal growth so that neurological condition is alleviated.

The term "neurological condition" as used herein refers to any condition of the central and/or peripheral nervous system of a subject which is alleviated, ameliorated or prevented by additional neurons. Injuries or diseases which result in loss or inhibition of neurons and/or loss or inhibition of neuronal function are neurological conditions for treatment by methods according to aspects of the present invention.

The term "therapeutically effective amount" as used herein is intended to mean an amount of an inventive composition which is effective to alleviate, ameliorate or prevent a symptom or sign of a neurological condition to be treated. In particular embodiments, a therapeutically effective amount is an amount which has a beneficial effect in a subject having signs and/or symptoms of a neurological condition.

The terms "treat," "treatment," "treating" and "NeuroD1 treatment" or grammatical equivalents as used herein refer to alleviating, inhibiting or ameliorating a neurological condition, symptoms or signs of a neurological condition, and preventing symptoms or signs of a neurological condition, and include, but are not limited to therapeutic and/or prophylactic treatments.

Signs and symptoms of neurological conditions are well-known in the art along with methods of detection and assessment of such signs and symptoms.

Combinations of therapies for a neurological condition of a subject are administered according to aspects of the present invention.

According to particular aspects an additional pharmaceutical agent or therapeutic treatment administered to a subject to treats the effects of disruption of normal blood flow in the CNS in an individual subject in need thereof include treatments such as, but not limited to, removing a blood clot, promoting blood flow, administration of one or more anti-inflammation agents, administration of one or more anti-oxidant agents, and administration of one or more agents effective to reduce excitotoxicity

The term "subject" refers to humans and also to non-human mammals such as, but not limited to, non-human primates, cats, dogs, sheep, goats, horses, cows, pigs and rodents, such as but not limited to, mice and rats; as well as to non-mammalian animals such as, but not limited to, birds, poultry, reptiles, amphibians.

Embodiments of inventive compositions and methods are illustrated in the following examples. These examples are provided for illustrative purposes and are not considered limitations on the scope of inventive compositions and methods.

### EXAMPLES

### Materials and Methods

### Mouse Model of Stroke and Virus Injection

Wild type (WT) FVB/NJ and GFAP-GFP transgenic mice were employed for the majority of the experiments described in this study. GFAP-GFP transgenic mice were purchased from the Jackson Laboratory [FVB/N-Tg (GFAPGFP)14Mes/J], described in Zhuo, L. et al., 1997, Dev Biol 187, 36-42, and mated with FVB/NJ mice (Jackson Laboratory). Endothelin-1 (1-31) was injected into the motor cortex of the adult WT FVB/NJ or GFAPA-GFP transgenic mice (28-40 g, 5-10 months old) to produce focal ischemic injury as described in Horie, N. et al., 2008, J. Neurosci. Methods 173, 286-290; and Roome, R.B. et al., 2014, J. Neurosci. Methods, 233, 34-44.

Under anesthesia by intra-peritoneal injection of ketamine/xylazine (100 mg/kg ketamine; 12 mg/kg xylazine), mice were placed in a stereotaxic apparatus with the skull and bregma exposed by a midline incision. A small hole of ~1mm was drilled in the skull at the coordinates of the forelimb motor cortex (relative to the bregma): +0.2 mm anterior-posterior (AP), ±1.5 mm medial-lateral (ML). ET-1 (1-31) (Peptide international, Inc., PED-4360-s) was dissolved at 2 µg/µl in phosphate-buffered saline (PBS; OSM ~320, pH ~7.3).

A total volume of 0.5 µl (1 µg) was injected into each site starting from -1.6 mm dorsal-ventral (DV). The injection was performed by infusion pump throughout 10 min and the injection needle was withdrawn slowly at 0.1 mm/min. After injection, the needle was kept at 1.1 mm DV for additional 3 min before fully withdrawn.

Viral injection followed a similar procedure, with the exception being that viral injection was typically performed around 10 days after ET-1 injection through the same hole drilled for ET-1 injection.

5-10 months old mice were used. Mice were housed in a 12 hr light/dark cycle and supplied with sufficient food and water. Both male and female mice were used, except only male mice were used in the behavioral experiments.

### AAV Vector Construction

The hGFAP promoter was obtained from pDRIVE-hGFAP plasmid (InvivoGen, Inc.) and inserted into pAAV-MCS (Cell Biolab) between MluI and SacII to replace the CMV promoter. The Cre gene was obtained by PCR from hGFAP-Cre (Addgene plasmid #40591) and inserted into pAAV MCS between EcoRI and Sal1 sites to generate pAAV-hGFAP::Cre vector.

To construct pAAV-FLEX-mCherry-P2A-mCherry (or pAAV-FLEX-GFP-P2A-GFP) and pAAV-FLEX-NeuroD1-P2A-mCherry (or pAAV-FLEX-NeuroD1-P2A-GFP) vectors, the cDNAs coding NeuroD1, mCherry or GFP were obtained by PCR using the retroviral constructs described in Guo, Z. et al., 2014, Cell Stem Cell 14, 188-202.

The NeuroD1 gene were fused with P2A-mCherry or P2A-GFP and subcloned into the pAAV-FLEX-GFP vector (Addgene plasmid # 28304) between Kpn1 and Xho1 sites. Plasmid constructs were sequenced for verification.

### AAV Virus Production

Recombinant AAV9 was produced in 293AAV cells (Cell Biolabs). Briefly, polyethylenimine (PEI, linear, MW 25,000) was used for transfection of triple plasmids: the pAAV expression vector; pAAV9-RC (Cell Biolab); and pHelper (Cell Biolab).

72 hrs post transfection, cells were scraped off the plates in their medium and centrifuged, frozen and thawed four times by placing them alternately in dry ice/ethanol and a 37 °C water bath. AAV crude lysate was purified by centrifugation at 54,000 rpm for 1 hr in discontinuous iodixanol gradients with a Beckman SW55Ti rotor.

The virus-containing layer was extracted and viruses were concentrated by Millipore Amicon Ultra Centrifugal Filters. Virus titers were 1.2 x 10¹² GC/ml for hGFAP::Cre, , hGFAP::NeuroD1-GFP and hGFAP::GFP, 1.4 x 10¹² GC/ml for CAG::FLEX-NeuroD1-P2A-GFP and CAG::FLEX-NeuroD1-P2A-mCherry, and 1.6 x 10¹² GC/ml for CAG::FLEX-mCherry-P2A-mCherry and CAG::FLEX-GFP-P2A-GFP, determined by QuickTiter^{™} AAV Quantitation Kit (Cell Biolabs).

### Retrovirus Production

The pCAG-NeuroD1-IRES-GFP and pCAG-GFP were described in Guo, Z. et al., 2014, Cell Stem Cell 14, 188-202. To package retroviral particles, gpg helper-free human embryonic kidney (HEK) cells were transfected with the target plasmid together with vesicular stomatitis virus glycoprotein (VSV-G) vector to produce the retroviruses expressing NeuroD1 or GFP. The titer of retroviral particles was about 10⁷ particles/ml, determined after transduction of HEK cells.

### Immunohistochemistry

Immunohistochemistry of floating frozen sections of mouse brain was performed as described in Guo, Z. et al., 2014, Cell Stem Cell 14, 188-202. Animals were anesthetized with 2.5% Avertin and transcardially perfused with artificial cerebral spinal fluid (ACSF) to wash off the blood in the brain tissue. Then, brains were dissected out, trimmed, and put in 4% paraformaldehyde (PFA) for post-fixation at 4 °C overnight. After fixation, brain tissues were cut at 40 µm sections by a vibratome (Leica).

Brain sections were permeabilized in 2% Triton X-100 in PBS for 1 hr, followed by incubation in blocking buffer (2.5 % normal goat serum, 2.5 % normal donkey serum, and 0.1% Triton X-100 in PBS) for 1 hr. The primary antibodies were added into the blocking buffer with brain sections and incubated overnight at 4 °C.

After washing off primary antibodies in PBS, brain sections were incubated with secondary antibodies conjugated with different fluorophores (1:800, Jackson ImmunoResearch) for 1 hr at room temperature, washed in Triton-PBS, and then mounted onto a glass slide with an anti-fading mounting solution containing DAPI (Invitrogen). Images were acquired with confocal microscopes (Olympus FV1000 or Zeiss LSM800) and a Keyence microscope.

To test for antibody specificity, the primary antibody was withdrawn and only a secondary antibody was used for immunostaining as a side-by-side control for all the primary antibodies. No specific signal was detected in these controls.

### BBB permeability test

Deeply anesthetized mice were perfused with artificial cerebral spinal fluid as described above, followed by 15 ml 0.5 mg/ml Sulfo-NHS-LC-Biotin in PBS. For immunohistochemistry, brain sections were incubated with Texas Red Streptavidin (Vector SA-5006), 1:800 diluted in PBS + 0.3 % triton + 2.5 % normal goat or donkey serum at room temperature for 1 hr, followed by normal mounting procedures.

### Electrophysiology

Brain slice recordings were performed similar to methods described in Guo, Z. et al., 2014, Cell Stem Cell 14, 188-202; and Wu, Z. et al., 2014, Nat. Commun. 5, 4159. 2-3 months after AAV injection, the mice were anaesthetized with 2.5% avertin, and then perfused with NMDG-based cutting solution (in mM): 93 NMDG, 93 HCl, 2.5 KCl, 1.25 NaH₂PO₄, 30 NaHCO₃, 20 HEPES, 15 glucose, 12 N-Acetyl-L-cysteine, 5 sodium ascorbate, 2 thiourea, 3 sodium pyruvate, 7 MgSO₄, 0.5 CaCl₂, pH 7.3-7.4, 300 mOsmo, bubbled with 95% O₂ / 5% CO₂.

Coronal sections of 300 µm thickness were cut around AAV-injected cortical areas with a vibratome (VT1200S, Leica, Germany) at room temperature. Slices were collected and incubated at 33.0 ± 1.0 °C in oxygenated NMDG cutting solution for 10-15 minutes. Slices were then transferred to holding solutions with continuous 95% O₂ / 5% CO₂ bubbling (in mM): 92 NaCl, 2.5 KCl, 1.25 NaH₂PO₄, 30 NaHCO₃, 20 HEPES, 15 glucose, 12 N-Acetyl-L-cysteine, 5 sodium ascorbate, 2 Thiourea, 3 sodium pyruvate, 2 MgSO₄, 2 CaCl₂.

After recovery at least 0.5 h at room temperature in the holding solution, a single slice was transferred to the recording chamber continuously perfused with standard aCSF (artificial cerebral spinal fluid) saturated by 95% O₂ / 5% CO₂ at 33.0 ± 1.0 °C. The standard aCSF contained (in mM): 124 NaCl, 2.5 KCl, 1.25 NaH₂PO₄, 26 NaHCO₃, 10 glucose, 1.3 MgSO₄, 2.5 CaCl₂.

To detect action potential firing in NeuroD1-GFP-infected neurons, whole-cell recordings were performed with pipette solution containing (in mM): 135 K-Gluconate, 10 KCl, 5 Na-phosphocreatine, 10 HEPES, 2 EGTA, 4 MgATP and 0.3 Na₂ GTP, pH 7.3 adjusted with KOH, 280-290 mOsm. Depolarizing currents were injected to elicit action potentials under current-clamp model.

To record spontaneous excitatory postsynaptic currents (sEPSCs) and spontaneous inhibitory postsynaptic currents (sIPSCs), pipette solution contained (in mM): 120 Cs-Methanesulfonate, 10 KCl, 10 Na-phosphocreatine, 10 HEPES, 5 QX-314, 1 EGTA, 4 MgATP and 0.3 Na₂GTP, pH 7.3 adjusted with KOH, 280-290 mOsm. To labeled recorded neurons, 0.5% biocytin was added to the pipette solution.

The cell membrane potentials were held at -70 mV (the reversal potential of GABAA receptors) for sEPSC recording, and 0 mV (the reversal potential of ionotropic glutamate receptors) for sIPSC recording, respectively. Data were collected with a MultiClamp 700A amplifier and analyzed with pCLAMP10 software (Molecular Devices).

### Mouse Behavioral Tests

### Food Pellet Retrieval Test

For all behavioral experiments in mice, ET-1 (1-31) was injected into two points of the forelimb motor cortex in order to induce severe motor deficits. The coordinates of the two points are: (i) +0.2 mm AP, +/- 1.35 mm ML; (ii) +0.38 mm AP, +/- 2.45 mm ML (+ or - ML was based on the opposite side of the dominant forelimb in pre-stroke pellet retrieval training).

The food pellet retrieval chamber was modified from the staircase equipment for mice described in Baird, A.L. et al., 2001, Brain Res. Bull. 54, 243-250; and Roome, R.B. et al., 2014, J. Neurosci. Methods, 233, 34-44. In brief, the staircase was changed to an arm with a single V-shape well and a small baffle at the end to prevent food pellet dropped outside the well during the test. The well is 33 mm long and the lowest part of the well is 16 mm from the bottom of top board, as illustrated in the Figure 14A.

Before pellet retrieval test, mice were food-deprived for 18 hrs to increase their motivation. During initial food deprivation training, mice were given 30 sucrose pellets (14 mg, TestDiet, Inc) in home cages for familiarization. For the first part of training, the top board of the center plinth was removed to reduce difficulty and encourage animals to try reaching the food. Five pellets were placed in the well either on the right side or left side to determine the dominant forelimb. Mice were given 5 min and tested twice on each side. The dominant side was determined at this stage by counting how many pellets were retrieved. Most animals could get ~5 pellets on one side and sometimes both sides. If the animals failed to get any pellet, they were excluded from further tests.

For the second part of training and all the following testing, the top board was placed back onto the center plinth and 8 pellets were placed in the well at the dominant side. Animals were tested 3 consecutive times every day, each time being given 5 min for pellet retrieval. The animals would be used for further surgery and testing if the average retrieval was more than 4 pellets on three consecutive training days.

After training, ischemic stroke was induced in the contralateral motor cortex controlling the dominant forelimb. At 9 days after stroke and one day before viral injection, a pellet retrieval test was performed to determine their functional levels. If the number of pellets retrieved were less than half of their pre-stroke level, these animals would be used for viral injection the next day (10 days post stroke).

After viral injection, pellet retrieval tests were performed at 20, 30, 50, and 70 dps to assess their functional recovery. For each time point, pellet retrieval tests were repeated on two consecutive days, with day 1 as training and day 2 as the actual test. Animals were coded and a second experimenter who was blind to the identity of the animals performed the test and pellet counting.

### Grid Walking Test

The grid-walking test was similar to that described in Baskin, Y.K. et al., 2003, J. Neurosci. Methods 129, 87-93; and Clarkson, A.N. et al., 2010, Nature 468, 305-309.

A 1 cm² grid wire mesh of 24 cm long and 20 cm wide, elevated to 24 cm high was used. A video camera was placed below the grid, facing upwards with a 45° angle. Slow motion video footages were recorded to assess the animals' foot faults. An individual mouse was placed on top of the grid floor and allowed to freely walk for 5 min. The animals were coded and video footage was analyzed offline by a second researcher who was blind to the animal identity.

The total number of foot faults for dominant limb and the total number of non-foot-fault steps were counted. Foot fault percentage was calculated as: the number of foot faults / total number of steps × 100.

### Cylinder Test

The cylinder test was based on methods described in Roome, R.B. et al., 2014, J. Neurosci. Methods, 233, 34-44. This test involved video recording of mice rising up and touching the sidewall of a transparent cylinder with forelimbs (10 cm diameter, 15 cm tall). Each animal was placed into the cylinder on a transparent board and video-recorded with a camera from below. The experiment was stopped after the mouse attempted to rise and touch the sidewall for >30 times, which normally lasted ~3-4 minutes.

The videos were analyzed offline by an experimenter blinded to the animal's identity. 30 total attempts of rising and touching the wall were analyzed. A normal paw touch is defined as the animal placing both paws on the wall while rising, and using both paws to push against the wall while descending. An abnormal touch involves only using one of the paws to touch the wall, or one paw dragging along the wall after touching.

Dragging behavior is defined as one paw, typically the injured one, sliding along the sidewall without holding steady against the wall. A non-touching behavior is defined as the mouse not using its injured paw but only using the non-injured paw while rising and descending. Non-touching is considered a more severe injury phenotype since the mouse totally abandons the injured paw. The normal rising and touching behavior was quantified as: normal paw touches / total attempts × 100.

### Quantitative Real-Time PCR

The cortical tissues around the injury core (approximately 2 mm x 2 mm square) were taken after perfusion and flash-frozen in liquid nitrogen. The RNA extraction was conducted using Macherey-Nagel NucleoSpin RNA kit and the RNA concentration was measured by NanoDrop. The cDNAs were synthesized using Quanta Biosciences qScript cDNA supermix.

The reaction mixture was incubated at 25 °C for 5 min, 42 °C for 30 min, 85 °C for 5 min and held at 4 °C. The mixture was then diluted 5-fold with RNase/DNase-free water and 2.5 µl was taken for qRT-PCR. The primers for qRT-PCR were designed using Applied Biosystems Primer Express software.

The qRT-PCR was conducted using Quanta Biosciences PerfeCTa SYBR Green Supermix, ROX. GAPDH was used as an internal control and non-stroke cortical tissue from healthy mice was used as control. Comparative Ct method was used for calculation of fold change and Prism 6 was used for statistical analysis and bar graphs.

### Golgi Impregnation Staining

The slice Golgi kit (Bieoenno Tech, LLC) was used for Golgi staining. In brief, mice were perfused with aCSF, followed by fixative from the kit. The brains were removed and impregnated for five days, sectioned by vibratome at 200 µm, stained and mounted based on the kit protocol, coverslipped with DPX mountant. Brightfield images at 4x and 100x were taken using a Keyence BZ-9000 Fluorescent microscope.

### Quantification And Statistical Analysis

### Cortical area size analysis

Cortical areas were quantified using the images taken with a 4x lens in a Keyence BZ-9000 Fluorescent microscope. Three slices around the injection point (+0.2 AP) with most obvious stroke injury were chosen for image-taking. DAPI and NeuN signals were used to identify the cortical upper boundary and the lower interface with cingulate cortex. Cortical areas from the midline to 3 mm lateral were measured by ImageJ.

### GFAP+ and NeuN+ ratio for conversion efficiency analysis

40x confocal images of mCherry/GFP co-stained with GFAP or NeuN were used for quantification at 4, 7 and 17 dpi. Four images were taken per animal at virus-infected areas near the stroke infarct for each animal. For GFAP ratio, cells that were double-positive for mCherry/GFP and GFAP were counted using Zeiss confocal software Zen. The percentage of double positive cells among all the mCherry or GFP positive cells (all the viral infected cells) was calculated. Similar method was applied to NeuN ratio.

### Cortical neuronal marker for converted cell identity analysis

40x confocal images of GFP (NeuroD1-GFP) co-stained with cortical neuron marker (Tbr1, Emx1, Satb2, PV, GABA) were used for quantification at 60 dpi. Three images were taken at virus-infected areas showing GFP signal for each animal. Percentage of cells double-positive for GFP and each individual marker among all GFP positive cells (NeuroD1-GFP infected cells) were calculated separately.

### Glial and neuronal marker intensity and covered area analysis

Single layer confocal images of glial markers (LCN2, CSPG, AQP4 and Iba1) and neuronal process marker (Map2, SMI312, VGluT1 and MBP) were used for quantification at 17 dpi. 40x lens was used for most markers except for SMI312, for which 63x lens was used. Three images were taken at virus-infected areas close to the stroke infarct (within 500 µm from stroke core boarder) and at the middle of whole cortical thickness. One image was taken at relatively healthy region for normalization. After setting the threshold based on the background in the healthy area, ImageJ was used to quantify the intensity and covered area of the whole image area. Results from three images were used to get average data for each animal.

### Overall NeuN and PV cell number analysis

NeuN and PV cell number was counted on the images of NeuN or PV immunostaining taken by the tile function of Zeiss LSM800 confocal microscope within the cortical areas from 500 µm to 2500 µm lateral of the midline. The confocal software Zen was used for cell counting. Slices around the injection point (+0.2 AP) with most obvious stroke injury were used for image taken.

### Local NeuN number analysis

40x confocal images of NeuN immunostaining were used for quantification at 17 dpi. Three images were taken at virus-infected areas close to the stroke infarct (within 500 µm from stroke core boarder), as shown in Figure 4A. Z stack function was used with 10 sections at 1 µm interval. The confocal software Zen was used for cell counting. For NeuroD1 group, cells positive for both GFP (NeuroD1-GFP) and NeuN (converted neurons) or NeuN positive GFP negative (pre-existing neurons) were counted. For GFP control group, total number of NeuN positive cells were counted.

### Electrophysiological analysis

The sEPSCs and sIPSCs were analyzed using Mini Analysis Program (Synaptosoft, New Jersey, USA). To avoid multiple detections of large events, the analysis results were further checked visually after auto-detection. To compare sEPSCs and sIPSCs frequency between different groups, over 200 events or at least 3-min recording periods were sampled for each cell before obtaining the average value.

### Blood vessel number analysis

For Ly6C number quantification, single-layer 40x images were taken by Zeiss confocal. The Ly6C intensity was measured by ImageJ and blood vessels having intensities three times stronger than background were identified as positive signals. The number of Ly6C+ blood vessels in three images in peri-infarct areas was quantified and averaged for each mouse.

### Statistics and Blind analysis

Statistical analyses were performed using Prism 6 (GraphPad) software. All experiments shown were repeated in at least three animals, and representative data are shown. To determine the significance between groups, comparisons were made using paired two-tailed Student's t test, or repeated-measurement ANOVA test as indicated. After primarily screening for severe stroke before viral injection, animals were randomly assigned into groups with a matching deficit level. All the images for quantification were taken by one researcher and were quantified by a different researcher who was blind to the animal condition and identity. As mentioned above, mouse behavioral tests were done in a blind fashion.

### Example 1: NeuroD1 Converts Stroke-Induced Reactive Astrocytes into Neurons

In this example, it was investigated whether *in vivo* cell conversion can achieve functional brain repair following an ischemic stroke. A focal stroke model induced by the vasoconstrictive peptide endothelin-1 (ET-1) was used to produce consistent local ischemic injury in rodents (Fuxe *et al.*, 1997; Hughes *et al.*, 2003; Roome *et al.*, 2014; Windle *et al.*, 2006).

When two different ET-1 peptides, one with 21 amino acids (ET-1 (1-21)) and another with 31 amino acids (ET-1 (1-31)) were compared, it was discovered that ET-1 (1-31) produced more severe and prolonged brain damage than ET-1 (1-21) (*see,* Figure 2A). A genetic strain of mice with FVB background gave more severe stroke damage than the commonly used B6/C57 mice (*see,* Figure 2A). Notably, significant cortical tissue loss was observed with injection of ET-1 (1-31) into the motor cortex of FVB mice (*see,* Figure 1A and Figure 1B) - establishing a severe focal stroke model with consistent tissue damage over a time course of 2 months.

A suitable time window for NeuroD1 viral injection after stroke was determined. Because NeuroD1 can convert reactive astrocytes into neurons, the stroke areas were surveyed at different time points to examine when astrocytes became reactive.

Five days post stroke (dps), there was a significant loss of neuronal signal NeuN, as expected (*see,* Figure 1C). Interestingly, the GFAP signal, a commonly used reactive astrocyte marker, was also very low, suggesting that the astrocytes had not been activated at this early stage. At 10 dps, however, the GFAP signal was significantly upregulated (*see,* Figure 1D), indicating that the astrocytes became reactive around this time.

It was then determined whether these reactive astrocytes induced by ischemic stroke could be converted into neurons. Reactive glial cells infected by NeuroD1 retroviruses were successfully converted into NeuN-positive neurons (*see,* Figure 1E; 17 days post viral injection, 27 days post stroke).

While retroviruses have the advantage of mainly targeting the dividing reactive glial cells after injury, they cannot generate a large number of neurons for neural repair because the number of dividing glial cells is rather limited. In order to generate a sufficient number of neurons for functional repair after stroke, adeno-associated virus (AAV) was used to infect both dividing and non-dividing glial cells. AAV has the advantage of high infection rate and low pathogenicity in humans, and has been approved by FDA for clinical trials in the treatment of CNS disorders.

To infect astrocytes specifically, an AAV vector (recombinant serotype AAV9) expressing NeuroD1 under the control of a human GFAP promoter (hGFAP::NeuroD1-P2A-GFP) was constructed. After two weeks of infection, many NeuroD1-infected cells were also converted into NeuN-positive neurons (*see,* Figure 1F). However, one caveat with the GFAP promoter-driven expression is that the GFAP promoter will be silenced after astrocyte-to-neuron conversion, making it difficult to distinguish converted from non-converted neurons.

To overcome this limitation, the GFAP promoter and NeuroD1 were separated into two different AAV9 vectors by using the Cre-FLEX (flip-excision) homologous recombination system (*see,* Atasoy *et al.*, 2008). A human GFAP promoter was then used to drive the expression of Cre recombinase (hGFAP::Cre), which could then act on two pairs of heterotypic, antiparallel loxP-type recombination sites flanking an inverted sequence of NeuroD1-P2A-GFP (or NeuroD1-P2A-mCherry) under the control of a CAG promoter in a separate vector (CAG: :FLEX-NeuroD1-P2A-GFP/mCherry) (*see,* Figure 2B). The advantage of this system is that after Cre-mediated inversion, NeuroD1 expression will be driven by a strong promoter, CAG, to significantly increase its expression level. This was confirmed by the observation that at 4 days post viral injection (AAV9 hGFAP::Cre and CAG::FLEX- NeuroD1-P2A-mCherry), many infected astrocytes already expressed high levels of NeuroD1 (*see,* Figure 1G, top row).

Interestingly, some GFAP-positive astrocytes were clearly captured in a transitional stage toward NeuN-positive neurons (*see,* Figure 1G, bottom row). Two weeks after viral injection, many NeuroD1-infected cells lost the GFAP signal and became NeuN-positive neurons (*see,* Figure 1I, bottom row), whereas control AAV FLEX-GFP-infected cells largely retained glial morphology (*see,* Figure 1I, top row). Thus, using the Cre-FLEX system, it is demonstrated that NeuroD1 can convert many reactive astrocytes into neurons after ischemic stroke.

Conversion efficiency as well as the type of neurons generated in stroke areas were then assessed. To examine the NeuroD1-mediated conversion efficiency, the number of astrocytes and neurons among the AAV-infected cells in the stroke areas at 4, 7, and 17 days after viral injection were examined (*see,* Figures 3A-3D). Using GFAP immunostaining to identify astrocytes it was found that among the control virus mCherry/GFP-infected cells, the majority were co-localized with GFAP from 4 to 17 dpi (*see,* Figure 3A, top row).

Among the AAV-NeuroD1-mCherry infected cells, however, the GFAP signal showed a dramatic reduction from 4 to 17 dpi (*see,* Figure 3A, bottom row), suggesting that many AAV-NeuroD1-infected cells lost astrocytic identity. In contrast to GFAP, NeuN staining revealed very few neurons among the mCherry-infected control group, but the NeuroD1-mCherry expressing cells became increasingly colocalized with NeuN (*see,* Figure 3B).

Quantitative analyses revealed that ~70% of mCherry-infected cells maintained its astrocytic identity throughout 4-17 dpi (*see,* Figure 3C), but >70% of NeuroD1-infected cells adopted neuronal identity by 17 dpi (*see,* Figure 3D).

To further investigate conversion efficiency and specificity, an Tri-AAV tracing system was developed to track astrocyte-converted neurons, which included a virus that expressed GFP in the astrocytes (AAV9 hGFAP::GFP) together with a Cre-FLEX system of the present invention (*see,* Figures 4A-4C). In mCherry control group, only 10% of GFP+ cells showed NeuN+ signal by 60 dpi; but in NeuroD1-mCherry group, 95% of GFP+ cells were also NeuN+ at 60 dpi (quantified in Figure 4B), further confirming the high conversion efficiency of NeuroD1 when expressed in astrocytes. These results suggest that a NeuroD1 AAV of the present invention can efficiently convert astrocytes into neurons in the stroke areas.

The identity of neurons converted from astrocytes in the stroke areas was then assessed. It was found that the astrocyte-converted neurons in the motor cortex were immunopositive for cortical pyramidal neuron markers including Emx1, Tbr1, and Satb2 (*see,* Figure 3E and Figure 3F). Only about 10% were immunopositive for GABAergic neuron markers including parvalbumin and GABA (*see,* Figure 3F). Thus, after ectopic expression of NeuroD1 in the stroke areas, astrocyte-converted neurons showed similar neuronal identity to their neighboring cortical neurons.

### Example 2: Reduction of Neuroinflammation After Cell Conversion

Accompanying the astrocyte-to-neuron conversion after NeuroD1 infection in the stroke areas, a significant reduction in GFAP signal in the stroke areas was observed *(see,* Figure 5A). This is expected, because >70% of NeuroD1-infected reactive astrocytes have been converted into neurons, and the GFAP signal will be decreased accordingly. However, this also raises a question whether astrocytes might be depleted in the converted areas. To answer this, when the astrocyte-to-neuron conversion was largely completed, the stroke areas close to the infarction (*) which were highly infected by NeuroD 1 AAV at 17 days post viral injection (dpi; 27 dps) were examined.

While astrocytes did show a reduction in the NeuroD1-infected areas, there were still a significant number of astrocytes remaining (*see,* Figure 5B). Surprisingly, not only was the astrocyte number reduced, but astrocyte morphology was significantly changed in the NeuroD1-converted areas. In the control group, astrocytes were hypertrophic and densely intermingled together in the areas surrounding the injury core (*see,* Figure 5B, top row); whereas in the NeuroD1 group, most of the astrocytes had elongated processes with only a few showing hypertrophic morphology (*see,* Figure 5B, bottom row; and Figure 6A-C). suggests that the remaining astrocytes became less reactive in the NeuroD1-converted areas.

Because GFAP only labeled the main processes of astrocytes, GFAP-GFP transgenic mice were used to illuminate the entire astrocyte morphology. Similar to GFAP immunostaining, the use of GFAP-GFP mice also revealed fewer hypertrophic astrocytes in the NeuroD1 group (*see,* Figure 5C). Notably, an abundant amount of GFP-labeled astrocytes in the NeuroD1-mCherry expressing areas was observed (*see,* Figure 5C, bottom row), suggesting that astrocytes are not depleted after conversion. This differs markedly from the astrocyte-killing approach. The persistence of astrocytes with *in vivo* cell conversion is likely due to the fact that astrocytes have an intrinsic capacity to divide and regenerate themselves.

When 70-95% of NeuroD1-infected reactive astrocytes have been converted into neurons in the stroke sites, it could be expected that such significant reduction of reactive astrocytes would have a major impact on the local environment given the intimate relationship between astrocytes and neurons as well as astrocytes and microglia. Indeed, microglia-mediated neuroinflammation is closely related to reactive astrocytes after stroke injury.

In the control group, a massive number of microglia (marked by Iba1) in the injury core at 17 dpi (*see,* Figure 5D, top) were observed; but in the NeuroD1 group, the microglia showed a significant reduction (*see,* Figure 5D, bottom) - supporting an interaction between microglia and astrocytes. Morphologically, microglia in the control group were mostly amoeboid type (*see,* 5E, top); but in the NeuroD1 group, microglia mainly showed ramified shapes with clear processes (*see,* Figure 5E, bottom; quantified in Figure 6D; *see also* Figure 6A-B for 7 dpi and 40 dpi signal, respectively).

Accompanying the glial morphological changes, the glia-secreted proteins such as chondroitin sulfate proteoglycans (CSPGs) and lipocalin-2 (LCN2) were significantly reduced in the NeuroD1 group compared to the control group (*see,* Figure 5F and Figure 5G; quantified in Figure 6E-F). CSPG is an important player during glial scar formation that inhibits axonal regeneration and LCN2 is highly expressed in reactive glial cells and associated with neuroinflammation.

To verify the immunostaining results, RT-PCR experiments at 17 dpi were run to quantitatively analyze the glial markers and cytokines in the control or NeuroD1-infected cortical tissues. It was first confirmed that NeuroD1 was indeed highly expressed in the NeuroD1-injected samples (*see,* Figure 5H). The GFAP transcriptional level increased by ~50-fold after stroke but significantly decreased after NeuroD1 treatment (*see,* Figure 5H). The neuroinflammation related factor LCN2 was drastically increased by ~1600-fold after stroke but decreased to only 15-fold in NeuroD1-treatment group (*see,* Figure 5G).

Similarly, the pro-inflammatory factors including interferon γ, TNFα, interleukin 1β (IL-1β), and interleukin 6 (IL-6) all showed an increase after stroke, but were downregulated after NeuroD1-treatment (*see,* Figure 5I). The unexpected reduction of neuroinflammation after NeuroD1-treatment suggests that converting reactive astrocytes into neurons has a much broader impact than simply generating new neurons.

### Example 3: Neuroregeneration Leads to Neuroprotection

Consistent with a reduction of neuroinflammation, the number of NeuN-positive neurons appeared to increase significantly in the NeuroD1-infected areas (*see,* Figure 7A). Surprisingly, in the stroke areas close to the injury core, not only were NeuroD1-GFP-labeled neurons detected but also many non-converted neurons that were neither GFP-positive (*see,* Figure 7A, arrow) nor NeuroD1-positive (*see,* Figure 7B, arrow).

Quantitative analysis revealed that ~40% of NeuN+ neurons in the peri-infarct areas were NeuroD1-positive and ~60% of neurons were NeuroD1-negative (*see,* Figure 7C; Control group NeuN+, 27.1 ± 8.1 / 0.1 mm²; NeuroD1 group: NeuroD1-/NeuN+, 64.9, ± 7.9 / 0.1 mm²; NeuroD1+/NeuN+, 39.9, ± 2.4 / 0.1 mm²; n = 3 mice per group).

Similar results were obtained when the GFP+/NeuN+ neurons in NeuroD1-NeuroD1-GFP infected neurons were quantified (*see,* Figure 8A). In addition, the non-converted neurons in the NeuroD1-treated areas also more than doubled the number of neurons in the control GFP-infected areas (*see,* Figure 7C), suggesting that NeuroD1-mediated glia-to-neuron conversion significantly enhances neuronal survival.

Overall, the NeuN+ neurons in the NeuroD1-infected areas more than tripled the ones in the control group. In accordance with a significant increase in the number of neurons after NeuroD1-treatment, immunostaining of neuronal dendritic markers including microtubule-associated protein 2 (Map2) and SMI32 showed clear dendritic morphology in the NeuroD1 group, but not in the GFP control group (*see,* Figure 7D and Figure 7E; Map2 quantified in Figure 8C; *see also* Figure 8B for Map2 staining at 40 dpi).

Similarly, using axonal marker SMI312 and NF200 along with myelination marker MBP, more axons in the stroke areas were observed (*see,* Figure 7F; quantified in Figure 8D) in the NeuroD1 group, which were associated with high level of myelination (*see,* Figure 7G; quantified in Figure 8E). To corroborate with the immunostaining results, RT-PCR experiments to examine the neuronal gene expression level were run. Neuronal genes including NeuN, Robo2, and Syn1 all showed a significant decrease after stroke, but were rescued by NeuroD1-treatment (*see,* Figure 7H).

Finally, the total number of NeuN-positive neurons within the stroke injured cortical areas (500 µm to 2500 µm from midline, injection site at 1500 µm from midline) from two weeks to two months after viral infection were quantified. The total number of NeuN-positive neurons in the control group after stroke was only ~20% of the non-stroke brains, indicating 80% neuronal loss in a severe focal stroke model. However, the number of neurons in the NeuroD1 group significantly increased over two month period, rescuing ~70% of the total neurons in the stroke areas (*see,* Figure 7I).

Such a significant increase in the number of cortical neurons after *in vivo* cell conversion, including both converted and protected neurons, forms a solid foundation for functional recovery

### Example 4: Reconstitution of Functional Neural Circuits After Stroke

One pivotal question regarding *in vivo* cell conversion is whether the glia-converted neurons can form functional neural circuits. To answer this question, the morphological structures in the stroke areas after NeuroD1-mediated cell conversion was examined. After stroke and viral injection, the ischemic-injured motor cortex at 7, 17, 40, and 60 dpi in both controls and NeuroD1-treated mice was examined (*see,* Figure 9A).

Over the time course of two months, a gradual cortical tissue loss in the group injected with GFP control virus was observed, but the NeuroD1-treated group showed significant tissue preservation (*see,* Figure 9A). The analysis of the motor cortical tissue revealed that the control group lost 70% of the injured tissue over a 2-month period, whereas NeuroD1-treated group preserved the majority of the injured tissue with only 20% tissue loss (*see*, Figure 9B; *see also* Figure 10A for serial sections).

Notably, after 60 days of NeuroD1 infection, clear cortical layer structures were observed in all treated animals (*see,* Figure 9A, 60 dpi, NeuroD1 image), suggesting that NeuroD1-treatment not only generates new neurons, but also can reconstitute/preserve cortical layers.

H&E (hematoxylin and eosin) staining at 60 dpi (*see,* Figure 10B) was also performed. In the control group, besides tissue damage, many neurons appeared shrunken with eosinophilic cytoplasm and pyknotic nuclei; while in the NeuroD1 group, neurons showed much healthier morphology. Moreover, in coronal sections, axon bundles in the striatum originating from the NeuroD1-converted neurons in the stroke areas were detected. To track where the NeuroD1-infected neurons in the stroke areas projected to after *in vivo* cell conversion, through sagittal section analysis it was determined that the cortical neurons converted by NeuroD1 could send axons to striatum, thalamus, and hypothalamus ipsilaterally (*see,* Figure 9C). Contralaterally, NeuroD1-converted neurons projected their axons across the midline through corpus callosum to the contralateral cortical regions (*see,* Figure 10C). Therefore, the NeuroD1-converted neurons can be integrated into global brain circuits.

Neuronal functions in the stroke areas after NeuroD1-mediated cell conversion were also investigated. Whole-cell patch-clamp recordings were performed on AAV-NeuroD1-GFP-infected neurons in the cortical slices at 2 months after viral injection (*see,* 9D). Injecting depolarizing currents into the neurons triggered repetitive action potentials (*see,* Figure 9D).

Robust spontaneous synaptic events, both excitatory and inhibitory, in the NeuroD1-expressing neurons after stroke (*see,* Figure 9F) were recorded - suggesting that these neurons have integrated into the brain circuits through synaptic connections with other neurons. Quantitative comparison between the control and NeuroD1 groups demonstrated that the frequency of both EPSCs and IPSCs in the NeuroD1 group was significantly higher than in the control group (*see,* Figure 9G).

To confirm that the recorded neurons were NeuroD1-converted rather than pre-existing neurons, biocytin was included in the recording pipettes and immunostaining was performed after electrophysiological recordings. Figure 9E demonstrates that the neuron recorded (biocytin positive) was indeed NeuroD1-positive. The biocytin immunostaining also illuminated the complex dendritic structures of the NeuroD1-converted neurons in the motor cortex (*see,* Figure 9E).

Clear dendritic spines in the biocytin-labeled neurons converted by NeuroD1 in the stroke areas were observed (*see,* Figure 12A), which was confirmed with Golgi staining (*see,* Figure 12B). Further immunostaining with glutamatergic synaptic marker VGluT1 (vesicular glutamate transporter 1) revealed that the synaptic density in the NeuroD1 group was significantly increased compared to the control group (*see,* Figure 12C-D).

In addition, GABAergic neurons were observed by immunostaining of parvalbumin (PV, a specific marker for a subtype of GABAergic neurons). Ischemic stroke caused a significant loss of PV neurons (*see,* Figure 12F), but in the NeuroD1-treated group many PV neurons in the stroke areas were detected (*see,* Figure 12E; quantified in Figure 12F). While PV neurons typically were not colocalized with NeuroD1-GFP cells, they were intermingled with NeuroD1-GFP expressing neurons *(see,* Figure 12E) - suggesting that GABAergic neurons were likely protected by NeuroD1-treatment.

Together, this data demonstrates that NeuroD1-mediated cell conversion can reconstitute functional neural circuits in the stroke areas through both regenerating and preserving excitatory and inhibitory neurons.

### Example 5: Rescue of Blood Vessels and Blood-Brain-Barrier After Cell Conversion

If NeuroD1-treatment can rescue brain tissue loss and form neural circuits in the stroke areas, can blood vessels and blood-brain-barrier be restored after cell conversion?

To answer this question, blood vessels and astrocytic endfeet that contact blood vessels to form blood-brain-barrier in the stroke areas were examined *(see,* Figures 11A-11H). In normal brains without stroke, the astrocytic endfeet marker aquaporin 4 (AQP4, water channel) wrapped around the blood vessels (labeled by Ly6C, endothelial cell and monocyte marker), displayed an intact blood-brain-barrier (BBB) *(see,* Figure 11A, top row). In contrast, one week after ET-1 (1-31)-induced focal stroke, significant disruption of both blood vessels and astrocytic endfeet were observed *(see,* Figure 11A, bottom row).

The AQP4-labeled astrocytic endfeet were detached from blood vessels and became co-localized with GFAP *(see,* Figure 11A, bottom row) - suggesting a mislocalization of AQP4 from the endfeet to other areas of astrocytes. After NeuroD1-treatment, a significant restoration of blood vessels together with re-association of AQP4-labeled astrocytic endfeet in the stroke areas was observed *(see,* Figure 11B-C; 17 dpi, 27 dps).

Co-immunostaining of AQP4 with GFAP revealed that in the control group after stroke, AQP4 was diffusely distributed throughout the astrocytic processes; but in the NeuroD1-treated group, AQP4 was largely concentrated at the endfeet wrapping around the blood vessels *(see,* Figure 11D). The re-alignment of astrocytic endfeet on blood vessels in the NeuroD1 group was also clearly illustrated through coimmunostaining of CD31 (blood vessel) and GFAP *(see,* Figures 11C-E).

Quantitative analysis showed a significant decrease of both AQP4 intensity *(see,* Figure 11F) and AQP4-covered areas *(see,* Figure 11G) in NeuroD1-treated group compared to the GFP control group. Even in NeuroD1 group, the AQP4 signal appeared more associated with blood vessels in the regions where NeuroD1 was highly expressed than the areas with less NeuroD1 signal *(see,* Figure 12G).

The blood vessels showed a significant increase in the NeuroD1 group compared to the control group *(see,* Figure 11H). To study the integrity of BBB (blood brain barrier), a biotin perfusion test was used to detect BBB leakage. In the peri-infarct area, biotin showed strong signal outside the blood vessel (labeled by Ly6C) in control group, indicating BBB leakage *(see,* Figure 12H).

In NeuroD1 group, biotin signal mainly remained inside the blood vessels *(see,* Figure 12H). Even after 2 months of viral injection, the difference of AQP4 and Ly6C signal was still very significant between control group and NeuroD1 group *(see,* Figure 12I). Taken together, this suggests that NeuroD1-mediated cell conversion promotes angiogenesis in the stroke areas and restores the BBB after stroke injury

### Example 6: Functional Rescue of Motor and Memory Deficits

In view of the significant level of neuroregeneration in the stroke areas after NeuroD1-treatment, experiments were performed to determine whether this treatment can rescue functional deficits caused by stroke. After preliminary studies on a variety of animal behavioral tests, detailed analyses on mouse forelimb functions were performed using three tests: food pellet retrieval, grid walking, and a cylinder test *(see,* Figure 13A, behavioral test paradigm).

For all the behavioral tests, ET-1 (1-31) was injected at two points on one cortical side (one at forelimb motor cortex and one at forelimb sensory cortex) to produce a severe unilateral stroke on the dominant side according to the food pellet retrieval test. To test for food pellet retrieval, mice were food-deprived before the test to increase their motivation for food. Before stroke, normal animals were trained to retrieve on average 5-6 pellets out of 8 total in 5 minutes *(see,* Figure 14A for the pellet retrieval device).

At 9 days after stroke, their pellet retrieval capability was severely impaired (dropping to around 1 pellet on average in 5 min) *(see,* 13B). The stroked animals were then randomly divided into two groups with similar deficits for injection of AAV9 expressing either GFP alone or NeuroD1-GFP to monitor their functional recovery over 2 months. At 10 days after viral injection (20 days post stroke), there was no significant difference between the two groups; but after 20 days of viral injection, the NeuroD1 group started to show improvement. By 60 days after viral injection, the NeuroD1 group reached ~4 pellets/5 min, whereas the GFP control group remained at <2 pellets/5 min *(see,* Figure 13B).

Similarly, for the grid walking test, normal animals before stroke had a low rate of foot fault, typically ~5% within 5 minutes of free walking on a grid, but the foot fault rate increased to >10% after stroke *(see,* Figure 13C). After viral injection, the NeuroD1 group showed consistent improvement after 20 and 40 days of treatment, with the foot fault rate decreasing to ~7%, whereas the GFP control group remained at a high foot fault rate >9% *(see,* Figure 13C).

A cylinder test that assesses the forelimb function when animals rise and touch the sidewall of a cylinder was also performed. Normal animals typically use both forelimbs to touch and push steadily against the sidewall and normal touching rate is ~85%. After a unilateral stroke in the forelimb motor cortex, the function of the contralateral forelimb was significantly weakened and the impaired limb often failed to touch the sidewall or dragged along the wall after briefly touching *(see,* Figure 13D, normal touching rate dropped to ~35%).

After 40-60 days of viral infection, the NeuroD1 group showed a significant recovery in touching the sidewall with both forelimbs (~65%), whereas the majority of the GFP control mice remained weak and unable to use the injured forelimb *(see,* Figure 13D). In all three tests, injection of PBS as a sham control for ET-1 (1-31) did not result in any behavioral deficits, and injection of ET-1 (1-31) without viral injection produced similar deficits as the GFP control viral injection.

For the grid walk and cylinder test, the non-stroke side forelimb did not show significant deficits compared to the control animals without stroke. For the food pellet retrieval test, only the forelimb preferentially used to take the food pellets during the training sessions was stroke injured and tested. After finishing the behavioral tests at around two months post viral injection, the mice were sacrificed and anatomical and immunocytochemical analysis were performed. Consistent with the behavioral deficits, the brains of the GFP control group displayed a remarkable cavity in the stroke areas, whereas the NeuroD1 group showed much smaller injury at the stroke site *(see,* Figure 14B).

Immunostaining results following behavioral tests also showed a large tissue loss in the control group but a better-preserved cortex in the NeuroD1 group *(see,* Figure 14C; quantified in Figure 14D). Therefore, using three different mouse behavioral models, it is demonstrated that NeuroD1 treatment not only regenerates new neurons and reduces brain tissue loss, but also rescues motor functional deficits induced by ischemic stroke.

### Broad Impact of NeuroD1-Mediated In vivo Cell Conversion

In the stroke areas following NeuroD1-mediated *in vivo* glia-to-neuron conversion, both glial morphology and function were significantly improved in NeuroD1-infected regions. The significant number of astrocytes after neural conversion is consistent with the proliferation capability of reactive astrocytes. The improvement in astroglial morphology, from hypertrophic toward normally branched in NeuroD1-expressing areas, suggests that reactive glial cells are exposed to less injury signals, the newly generated astrocytes are less reactive, or both.

The change of microglia morphology from an amoeboid shape to a more ramified shape in the stroke areas after NeuroD1-treatment is consistent with a reduction of neuroinflammation - confirmed by the reduction of inflammatory factors of TNFα and IL-1β. Previous studies have reported a significant upregulation of CSPG and LCN2 in the stroke areas. As demonstrated herein, NeuroD1-treatment can significantly reduce the level of CSPG and LCN2 in the stroke areas, which is consistent with a significant reduction of reactive glial cells after conversion.

Following neuroregeneration, NeuroD1-treatment results in the repair of blood vessels and restoration of BBB in the stroke areas. This type of repair after neuroregeneration is consistent with neurovascular interaction during brain development and neural repair. Notably, NeuroD1-mediated *in vivo* cell conversion has a broad impact on local neuron-glia circuitry, including the generation of a large number of new neurons, decreasing the same number of reactive astrocytes, reducing neuroinflammation, repairing blood vessels, and restoring the BBB. Such landscape change of the entire neuron-glia circuitry is unprecedented.

### Structural Support for Functional Rescue

*In vivo* glia-to-neuron conversion can also functionally rescue the motor deficits caused by ischemic stroke in rodent models. This functional rescue is based on structural changes including both cellular conversion and circuit rebuilding after NeuroD1-treatment. A mixture of NeuroD1-GFP-labeled neurons together with non-labeled neurons in the stroke areas after NeuroD1-treatment was observed. This suggests that the repaired brain circuitry is an integration of both newly generated neurons and old mature neurons.

While NeuroD1-converted neurons are mainly glutamatergic, a significant number of GABAergic neurons that are not labeled by NeuroD1-GFP were observed -suggesting that GABAergic neurons are among the neurons rescued by NeuroD1-treatment. The preservation of GABAergic neurons suggests that NeuroD1-treatment may keep an excitation-inhibition balance after *in vivo* cell conversion, which is important to achieve normal brain function. Functional recovery is also achieved since NeuroD1-treatment can rescue over 70% of neurons in stroke injured motor cortex.

### SEQUENCES

SEQ ID NO: 1 - Human NeuroD1 nucleic acid sequence encoding human NeuroD1 protein - 1071 nucleotides, including stop codon 
ATGACCAAATCGTACAGCGAGAGTGGGCTGATGGGCGAGCCTCAGCCCCAAGGTCCTCCAAGCT
GGACAGACGAGTGTCTCAGTTCTCAGGACGAGGAGCACGAGGCAGACAAGAAGGAGGACGACCT
CGAAGCCATGAACGCAGAGGAGGACTCACTGAGGAACGGGGGAGAGGAGGAGGACGAAGATGAG
GACCTGGAAGAGGAGGAAGAAGAGGAAGAGGAGGATGACGATCAAAAGCCCAAGAGACGCGGCC
CCAAAAAGAAGAAGATGACTAAGGCTCGCCTGGAGCGTTTTAAATTGAGACGCATGAAGGCTAA
CGCCCGGGAGCGGAACCGCATGCACGGACTGAACGCGGCGCTAGACAACCTGCGCAAGGTGGTG
CCTTGCTATTCTAAGACGCAGAAGCTGTCCAAAATCGAGACTCTGCGCTTGGCCAAGAACTACA
TCTGGGCTCTGTCGGAGATCCTGCGCTCAGGCAAAAGCCCAGACCTGGTCTCCTTCGTTCAGAC
GCTTTGCAAGGGCTTATCCCAACCCACCACCAACCTGGTTGCGGGCTGCCTGCAACTCAATCCT
CGGACTTTTCTGCCTGAGCAGAACCAGGACATGCCCCCCCACCTGCCGACGGCCAGCGCTTCCT
TCCCTGTACACCCCTACTCCTACCAGTCGCCTGGGCTGCCCAGTCCGCCTTACGGTACCATGGA
CAGCTCCCATGTCTTCCACGTTAAGCCTCCGCCGCACGCCTACAGCGCAGCGCTGGAGCCCTTC
TTTGAAAGCCCTCTGACTGATTGCACCAGCCCTTCCTTTGATGGACCCCTCAGCCCGCCGCTCA
GCATCAATGGCAACTTCTCTTTCAAACACGAACCGTCCGCCGAGTTTGAGAAAAATTATGCCTT
TACCATGCACTATCCTGCAGCGACACTGGCAGGGGCCCAAAGCCACGGATCAATCTTCTCAGGC
ACCGCTGCCCCTCGCTGCGAGATCCCCATAGACAATATTATGTCCTTCGATAGCCATTCACATC
ATGAGCGAGTCATGAGTGCCCAGCTCAATGCCATATTTCATGATTAG
SEQ ID NO:2 - Human NeuroD1 amino acid sequence - 356 amino acids - encoded by SEQ ID NO:1 
MTKSYSESGLMGEPQPQGPPSWTDECLSSQDEEHEADKKEDDLEAMNAEEDSLRNGGEEEDEDE
DLEEEEEEEEEDDDQKPKRRGPKKKKMTKARLERFKLRRMKANARERNRMHGLNAALDNLRKVV
PCYSKTQKLSKIETLRLAKNYIWALSEILRSGKSPDLVSFVQTLCKGLSQPTTNLVAGCLQLNP
RTFLPEQNQDMPPHLPTASASFPVHPYSYQSPGLPSPPYGTMDSSHVFHVKPPPHAYSAALEPF
FESPLTDCTSPSFDGPLSPPLSINGNFSFKHEPSAEFEKNYAFTMHYPAATLAGAQSHGSIFSG
TAAPRCEIPIDNIMSFDSHSHHERVMSAQLNAIFHD
SEQ ID NO:3 - Mouse NeuroD1 nucleic acid sequence encoding mouse NeuroD1 protein - 1074 nucleotides, including stop codon 
ATGACCAAATCATACAGCGAGAGCGGGCTGATGGGCGAGCCTCAGCCCCAAGGTCCCCCAAGCT
GGACAGATGAGTGTCTCAGTTCTCAGGACGAGGAACACGAGGCAGACAAGAAAGAGGACGAGCT
TGAAGCCATGAATGCAGAGGAGGACTCTCTGAGAAACGGGGGAGAGGAGGAGGAAGATGAG
GATCTAGAGGAAGAGGAGGAAGAAGAGGAGGAGGATCAAAAGCCCAAGAGACGGGGTC
CCAAAAAGAAAAAGATGACCAAGGCGCGCCTAGAACGTTTTAAATTAAGGCGCATGAAGGCCAA
CGCCCGCGAGCGGAACCGCATGCACGGGCTGAACGCGGCGCTGGACAACCTGCGCAAGGTGGTA
CCTTGCTACTCCAAGACCCAGAAACTGTCTAAAATAGAGACACTGCGCTTGGCCAAGAACTACA
TCTGGGCTCTGTCAGAGATCCTGCGCTCAGGCAAAAGCCCTGATCTGGTCTCCTTCGTACAGAC
GCTCTGCAAAGGTTTGTCCCAGCCCACTACCAATTTGGTCGCCGGCTGCCTGCAGCTCAACCCT
CGGACTTTCTTGCCTGAGCAGAACCCGGACATGCCCCCGCATCTGCCAACCGCCAGCGCTTCCT
TCCCGGTGCATCCCTACTCCTACCAGTCCCCTGGACTGCCCAGCCCGCCCTACGGCACCATGGA
CAGCTCCCACGTCTTCCACGTCAAGCCGCCGCCACACGCCTACAGCGCAGCTCTGGAGCCCTTC
TTTGAAAGCCCCCTAACTGACTGCACCAGCCCTTCCTTTGACGGACCCCTCAGCCCGCCGCTCA
GCATCAATGGCAACTTCTCTTTCAAACACGAACCATCCGCCGAGTTTGAAAAAAATTATGCCTT
TACCATGCACTACCCTGCAGCGACGCTGGCAGGGCCCCAAAGCCACGGATCAATCTTCTCTTCC
GGTGCCGCTGCCCCTCGCTGCGAGATCCCCATAGACAACATTATGTCTTTCGATAGCCATTCGC
ATCATGAGCGAGTCATGAGTGCCCAGCTTAATGCCATCTTTCACGATTAG
SEQ ID NO:4 - Mouse NeuroD1 amino acid sequence - 357 amino acids - encoded by SEQ ID NO:3 
MTKSYSESGLMGEPQPQGPPSWTDECLSSQDEEHEADKKEDELEAMNAEEDSLRNGGEEEEEDE
DLEEEEEEEEEEEDQKPKRRGPKKKKMTKARLERFKLRRMKANARERNRMHGLNAALDNLRKVV
PCYSKTQKLSKIETLRLAKNYIWALSEILRSGKSPDLVSFVQTLCKGLSQPTTNLVAGCLQLNP
RTFLPEQNPDMPPHLPTASASFPVHPYSYQSPGLPSPPYGTMDSSHVFHVKPPPHAYSAALEPF
FESPLTDCTSPSFDGPLSPPLSINGNFSFKHEPSAEFEKNYAFTMHYPAATLAGPQSHGSIFSS
GAAAPRCEIPIDNIMSFDSHSHHERVMSAQLNAIFHD
Mouse LCN2 promoter - SEQ ID NO:5 
GCAGTGTGGAGACACACCCACTTTCCCCAAGGGCTCCTGCTCCCCCAAGTGATCCCCTTATCCT
CCGTGCTAAGATGACACCGAGGTTGCAGTCCTTACCTTTGAAAGCAGCCACAAGGGCGTGGGGG
TGCACACCTTTAATCCCAGCACTCGGGAGGCAGAGGCAGGCAGATTTCTGAGTTCGAGACCAGC
CTGGTCTACAAAGTGAATTCCAGGACAGCCAGGGCTATACAGAGAAACCCTGTCTTGAAAAAAA
AAGAGAAAGAAAAAAGAAAAAAAAAAATGAAAGCAGCCACATCTAAGGACTACGTGGCACAGGA
GAGGGTGAGTCCCTGAGAGTTCAGCTGCTGCCCTGTCTGTTCCTGTAAATGGCAGTGGGGTCAT
GGGAAAGTGAAGGGGCTCAAGGTATTGGACACTTCCAGGATAATCTTTTGGACGCCTCACCCTG
TGCCAGGACCAAGGCTGAGCTTGGCAGGCTCAGAACAGGGTGTCCTGTTCTTCCCTGTCTAAAA
CATTCACTCTCAGCTTGCTCACCCTTCCCCAGACAAGGAAGCTGCACAGGGTCTGGTGTTCAGA
TGGCTTTGGCTTACAGCAGGTGTGGGTGTGGGGTAGGAGGCAGGGGGTAGGGGTGGGGGAAGCC
TGTACTATACTCACTATCCTGTTTCTGACCCTCTAGGACTCCTACAGGGTTATGGGAGTGGACA
GGCAGTCCAGATCTGAGCTGCTGACCCACAAGCAGTGCCCTGTGCCTGCCAGAATCCAAAGCCC
TGGGAATGTCCCTCTGGTCCCCCTCTGTCCCCTGCAGCCCTTCCTGTTGCTCAACCTTGCACAG
TTCCGACCTGGGGGAGAGAGGGACAGAAATCTTGCCAAGTATTTCAACAGAATGTACTGGCAAT
TACTTCATGGCTTCCTGGACTTGGTAAAGGATGGACTACCCCGCCCAACAGGGGGGCTGGCAGC
CAGGTAGGCCCATAAAAAGCCCGCTGGGGAGTCCTCCTCACTCTCTGCTCTTCCTCCTCCAGCA
CACATCAGACCTAGTAGCTGTGGAAACCA
Human GFAP promoter - SEQ ID NO:6 
GTCTGCAAGCAGACCTGGCAGCATTGGGCTGGCCGCCCCCCAGGGCCTCCTCTTCATGCCCAGT
GAATGACTCACCTTGGCACAGACACAATGTTCGGGGTGGGCACAGTGCCTGCTTCCCGCCGCAC
CCCAGCCCCCCTCAAATGCCTTCCGAGAAGCCCATTGAGTAGGGGGCTTGCATTGCACCCCAGC
CTGACAGCCTGGCATCTTGGGATAAAAGCAGCACAGCCCCCTAGGGGCTGCCCTTGCTGTGTGG
CGCCACCGGCGGTGGAGAACAAGGCTCTATTCAGCCTGTGCCCAGGAAAGGGGATCAGGGGATG
CCCAGGCATGGACAGTGGGTGGCAGGGGGGGAGAGGAGGGCTGTCTGCTTCCCAGAAGTCCAAG
GACACAAATGGGTGAGGGGACTGGGCAGGGTTCTGACCCTGTGGGACCAGAGTGGAGGGCGTAG
ATGGACCTGAAGTCTCCAGGGACAACAGGGCCCAGGTCTCAGGCTCCTAGTTGGGCCCAGTGGC
TCCAGCGTTTCCAAACCCATCCATCCCCAGAGGTTCTTCCCATCTCTCCAGGCTGATGTGTGGG
AACTCGAGGAAATAAATCTCCAGTGGGAGACGGAGGGGTGGCCAGGGAAACGGGGCGCTGCAGG
AATAAAGACGAGCCAGCACAGCCAGCTCATGCGTAACGGCTTTGTGGAGCTGTCAAGGCCTGGT
CTCTGGGAGAGAGGCACAGGGAGGCCAGACAAGGAAGGGGTGACCTGGAGGGACAGATCCAGGG
GCTAAAGTCCTGATAAGGCAAGAGAGTGCCGGCCCCCTCTTGCCCTATCAGGACCTCCACTGCC
 
ACATAGAGGCCATGATTGACCCTTAGACAAAGGGCTGGTGTCCAATCCCAGCCCCCAGCCCCAG
AACTCCAGGGAATGAATGGGCAGAGAGCAGGAATGTGGGACATCTGTGTTCAAGGGAAGGACTC
CAGGAGTCTGCTGGGAATGAGGCCTAGTAGGAAATGAGGTGGCCCTTGAGGGTACAGAACAGGT
TCATTCTTCGCCAAATTCCCAGCACCTTGCAGGCACTTACAGCTGAGTGAGATAATGCCTGGGT
TATGAAATCAAAAAGTTGGAAAGCAGGTCAGAGGTCATCTGGTACAGCCCTTCCTTCCCTTTTTT
TTTTTTTTTTTTTGTGAGACAAGGTCTCTCTCTGTTGCCCAGGCTGGAGTGGCGCAAACACAG
CTCACTGCAGCCTCAACCTACTGGGCTCAAGCAATCCTCCAGCCTCAGCCTCCCAAAGTGCTGG
GATTACAAGCATGAGCCACCCCACTCAGCCCTTTCCTTCCTTTTTAATTGATGCATAATAATTG
TAAGTATTCATCATGGTCCAACCAACCCTTTCTTGACCCACCTTCCTAGAGAGAGGGTCCTCTT
GATTCAGCGGTCAGGGCCCCAGACCCATGGTCTGGCTCCAGGTACCACCTGCCTCATGCAGGAG
TTGGCGTGCCCAGGAAGCTCTGCCTCTGGGCACAGTGACCTCAGTGGGGTGAGGGGAGCTCTCC
CCATAGCTGGGCTGCGGCCCAACCCCACCCCCTCAGGCTATGCCAGGGGGTGTTGCCAGGGGCA
CCCGGGCATCGCCAGTCTAGCCCACTCCTTCATAAAGCCCTCGCATCCCAGGAGCGAGCAGAGC
CAGAGCAT
Mouse Aldh1L1 promoter - SEQ ID NO:7 
AACTGAGAGTGGAGGGGCACAGAAGAGCCCAAGAGGCTCCTTAGGTTGTGTGGAGGGTACAATA
TGTTTGGGCTGAGCAACCCAGAGCCAGACTTTGTCTGGCTGGTAAGAGACAGAGGTGCCTGCTA
TCACAATCCAAGGGTCTGCTTGAGGCAGAGCCAGTGCAAAGGATGTGGTTAGAGCCAGCCTGGT
GTACTGAAGAGGGGCGAAGAGCTTGAGTAAGGAGTCTCAGCGGTGGTTTGAGAGGCAGGGTGGT
TAATGGAGTAGCTGCAGGGGAGAATCCTTGGGAGGGAGCCTGCAGGACAGAGCTTTGGTCAGGA
AGTGATGGGCATGTCACTGGACCCTGTATTGTCTCTGACTTTTCTCAAGTAGGACAATGACTCT
GCCCAGGGAGGGGGTCTGTGACAAGGTGGAAGGGCCAGAGGAGAACTTCTGAGAAGAAAACCAG
AGGCCGTGAAGAGGTGGGAAGGGCATGGGATTCAGAACCTCAGGCCCACCAGGACACAACCCCA
GGTCCACAGCAGATGGGTGACCTTGCATGTCTCAGTCACCAGCATTGTGCTCCTTGCTTATCAC
GCTTGGGTGAAGGAAATGACCCAAATAGCATAAAGCCTGAAGGCCGGGACTAGGCCAGCTAGGG
CTTGCCCTTCCCTTCCCAGCTGCACTTTCCATAGGTCCCACCTTCAGCAGATTAGACCCGCCTC
CTGCTTCCTGCCTCCTTGCCTCCTCACTCATGGGTCTATGCCCACCTCCAGTCTCGGGACTGAG
GCTCACTGAAGTCCCATCGAGGTCTGGTCTGGTGAATCAGCGGCTGGCTCTGGGCCCTGGGCGA
CCAGTTAGGTTCCGGGCATGCTAGGCAATGAACTCTACCCGGAATTGGGGGTGCGGGGAGGCGG
GGAGGTCTCCAACCCAGCCTTTTGAGGACGTGCCTGTCGCTGCACGGTGCTTTTTATAGACGAT
GGTGGCCCATTTTGCAGAAGGGAAAGCCGGAGCCCTCTGGGGAGCAAGGTCCCCGCAAATGGAC
 
GGATGACCTGAGCTTGGTTCTGCCAGTCCACTTCCCAAATCCCTCACCCCATTCTAGGGACTAG
GGAAAGATCTCCTGATTGGTCATATCTGGGGGCCTGGCCGGAGGGCCTCCTATGATTGGAGAGA
TCTAGGCTGGGCGGGCCCTAGAGCCCGCCTCTTCTCTGCCTGGAGGAGGAGCACTGACCCTAAC
CCTCTCTGCACAAGACCCGAGCTTGTGCGCCCTTCTGGGAGCTTGCTGCCCCTGTGCTGACTGC
TGACAGCTGACTGACGCTCGCAGCTAGCAGGTACTTCTGGGTTGCTAGCCCAGAGCCCTGGGCC
GGTGACCCTGTTTTCCCTACTTCCCGTCTTTGACCTTGGGTAAGTTTCTTTTTCTTTTGTTTTT
GAGAGAGGCACCCAGATCCTCTCCACTACAGGCAGCCGCTGAACCTTGGATCCTCAGCTCCTGC
CCTGGGAACTACAGTTCCTGCCCTTTTTTTCCCACCTTGAGGGAGGTTTTCCCTGAGTAGCTTC
GACTATCCTGGAACAAGCTTTGTAGACCAGCCTGGGTCTCCGGAGAGTTGGGATTAAAGGCGTG
CACCACCACC
Human NG2 promoter - SEQ ID NO:8 
CTCTGGTTTCAAGACCAATACTCATAACCCCCACATGGACCAGGCACCATCACACCTGAGCACT
GCACTTAGGGTCAAAGACCTGGCCCCACATCTCAGCAGCTATGTAGACTAGCTCCAGTCCCTTA
ATCTCTCTCAGCCTCAGTTTCTTCATCTGCAAAACAGGTCTCAGTTTCGTTGCAAAGTATGAAG
TGCTGGGCTGTTACTGGTCAAAGGGAAGAGCTGGGAAGAGGGTGCAAGGTGGGGTTGGGCTGGA
GATGGGCTGGAGCAGATAGATGGAGGGACCTGAATGGAGGAAGTAAACCAAGGCCCGGTAACAT
TGGGACTGGACAGAGAACACGCAGATCCTCTAGGCACCGGAAGCTAAGTAACATTGCCCTTTCT
CCTCCTGTTTGGGACTAGGCTGATGTTGCTGCCTGGAAGGGAGCCAGCAGAAGGGCCCCAGCCT
GAAGCTGTTAGGTAGAAGCCAAATCCAGGGCCAGATTTCCAGGAGGCAGCCTCGGGAAGTTGAA
ACACCCGGATTCAGGGGTCAGGAGGCCTGGGCTTCTGGCACCAAACGGCCAGGGACCTACTTTC
CACCTGGAGTCTTGTAAGAGCCACTTTCAGCTTGAGCTGCACTTTCGTCCTCCATGAAATGGGG
GAGGGGATGCTCCTCACCCACCTTGCAAGGTTATTTTGAGGCAAATGTCATGGCGGGACTGAGA
ATTCTTCTGCCCTGCGAGGAAATCCAGACATCTCTCCCTTACAGACAGGGAGACTGAGGTGAGG
CCCTTCCAGGCAGAGAAGGTCACTGTTGCAGCCATGGGCAGTGCCCCACAGGACCTCGGGTGGT
GCCTCTGGAGTCTGGAGAAGTTCCTAGGGGACCTCCGAGGCAAAGCAGCCCAAAAGCCGCCTGT
GAGGGTGGCTGGTGTCTGTCCTTCCTCCTAAGGCTGGAGTGTGCCTGTGGAGGGGTCTCCTGAA
CTCCCGCAAAGGCAGAAAGGAGGGAAGTAGGGGCTGGGACAGTTCATGCCTCCTCCCTGAGGGG
GTCTCCCGGGCTCGGCTCTTGGGGCCAGAGTTCAGGGTGTCTGGGCCTCTCTATGACTTTGTTC
TAAGTCTTTAGGGTGGGGCTGGGGTCTGGCCCAGCTGCAAGGGCCCCCTCACCCCTGCCCCAGA
GAGGAACAGCCCCGCACGGGCCCTTTAAGAAGGTTGAGGGTGGGGGCAGGTGGGGGAGTCCAAG
CCTGAAACCCGAGCGGGCGCGCGGGTCTGCGCCTGCCCCGCCCCCGGAGTTAAGTGCGCGGACA
 
CCCGGAGCCGGCCCGCGCCCAGGAGCAGAGCCGCGCTCGCTCCACTCAGCTCCCAGCTCCCAGG
ACTCCGCTGGCTCCTCGCAAGTCCTGCCGCCCAGCCCGCCGGG
CAG::NeuroD1-IRES-GFP - SEQ ID NO:9

GATCCGGCCATTAGCCATATTATTCATTGGTTATATAGCATAAATCAATATTGGCTATTGGCCA
TTGCATACGTTGTATCCATATCATAATATGTACATTTATATTGGCTCATGTCCAACATTACCGC
CATGTTGACATTGATTATTGACTAGTTATTAATAGTAATCAATTACGGGGTCATTAGTTCATAG
CCCATATATGGAGTTCCGCGTTACATAACTTACGGTAAATGGCCCGCCTGGCTGACCGCCCAAC
GACCCCCGCCCATTGACGTCAATAATGACGTATGTTCCCATAGTAACGCCAATAGGGACTTTCC
ATTGACGTCAATGGGTGGAGTATTTACGGTAAACTGCCCACTTGGCAGTACATCAAGTGTATCA
TATGCCAAGTACGCCCCCTATTGACGTCAATGACGGTAAATGGCCCGCCTGGCATTATGCCCAG
TACATGACCTTATGGGACTTTCCTACTTGGCAGTACATCTACGTATTAGTCATCGCTATTACCA
TGGTGATGCGGTTTTGGCAGTACATCAATGGGCGTGGATAGCGGTTTGACTCACGGGGATTTCC
AAGTCTCCACCCCATTGACGTCAATGGGAGTTTGTTTTGGCACCAAAATCAACGGGACTTTCCA
AAATGTCGTAACAACTCCGCCCCATTGACGCAAATGGGCGGTAGGCATGTACGGTGGGAGGTCT
ATATAAGCAGAGCTCAATAAAAGAGCCCACAACCCCTCACTCGGGGCGCCAGTCCTCCGATTGA
CTGAGTCGCCCGGGTACCCGTATTCCCAATAAAGCCTCTTGCTGTTTGCATCCGAATCGTGGTC
TCGCTGTTCCTTGGGAGGGTCTCCTCTGAGTGATTGACTACCCACGACGGGGGTCTTTCATTTG
GGGGCTCGTCCGGGATTTGGAGACCCCTGCCCAGGGACCACCGACCCACCACCGGGAGGTAAGC
TGGCCAGCAACTTATCTGTGTCTGTCCGATTGTCTAGTGTCTATGTTTGATGTTATGCGCCTGC
GTCTGTACTAGTTAGCTAACTAGCTCTGTATCTGGCGGACCCGTGGTGGAACTGACGAGTTCTG
AACACCCGGCCGCAACCCTGGGAGACGTCCCAGGGACTTTGGGGGCCGTTTTTGTGGCCCGACC
TGAGGAAGGGAGTCGATGTGGAATCCGACCCCGTCAGGATATGTGGTTCTGGTAGGAGACGAGA
ACCTAAAACAGTTCCCGCCTCCGTCTGAATTTTTGCTTTCGGTTTGGAACCGAAGCCGCGCGTC
TTGTCTGCTGCAGCGCTGCAGCATCGTTCTGTGTTGTCTCTGTCTGACTGTGTTTCTGTATTTG
TCTGAAAATTAGGGCCAGACTGTTACCACTCCCTTAAGTTTGACCTTAGGTCACTGGAAAGATG
TCGAGCGGATCGCTCACAACCAGTCGGTAGATGTCAAGAAGAGACGTTGGGTTACCTTCTGCTC
TGCAGAATGGCCAACCTTTAACGTCGGATGGCCGCGAGACGGCACCTTTAACCGAGACCTCATC
ACCCAGGTTAAGATCAAGGTCTTTTCACCTGGCCCGCATGGACACCCAGACCAGGTCCCCTACA
TCGTGACCTGGGAAGCCTTGGCTTTTGACCCCCCTCCCTGGGTCAAGCCCTTTGTACACCCTAA
GCCTCCGCCTCCTCTTCCTCCATCCGCCCCGTCTCTCCCCCTTGAACCTCCTCGTTCGACCCCG
CCTCGATCCTCCCTTTATCCAGCCCTCACTCCTTCTCTAGGCGCCGGAATTCGATGTCGACATT

GATTATTGACTAGTTATTAATAGTAATCAATTACGGGGTCATTAGTTCATAGCCCATATATGGA
GTTCCGCGTTACATAACTTACGGTAAATGGCCCGCCTGGCTGACCGCCCAACGACCCCCGCCCA
TTGACGTCAATAATGACGTATGTTCCCATAGTAACGCCAATAGGGACTTTCCATTGACGTCAAT
GGGTGGACTATTTACGGTAAACTGCCCACTTGGCAGTACATCAAGTGTATCATATGCCAAGTAC
GCCCCCTATTGACGTCAATGACGGTAAATGGCCCGCCTGGCATTATGCCCAGTACATGACCTTA
TGGGACTTTCCTACTTGGCAGTACATCTACGTATTAGTCATCGCTATTACCATGGGTCGAGGTG
AGCCCCACGTTCTGCTTCACTCTCCCCATCTCCCCCCCCTCCCCACCCCCAATTTTGTATTTAT
TTATTTTTTAATTATTTTGTGCAGCGATGGGGGCGGGGGGGGGGGGGGCGCGCGCCAGGCGGGG
CGGGGCGGGGCGAGGGGCGGGGCGGGGCGAGGCGGAGAGGTGCGGCGGCAGCCAATCAGAGCGG
CGCGCTCCGAAAGTTTCCTTTTATGGCGAGGCGGCGGCGGCGGCCCTATAAAAAGCGAAGC
GCGCGGCGGGCGGGAGTCGCTGCGTTGCCTTCGCCCCGTGCCCCGCTCCGCGCCGCCTCGCGCC
GCCCGCCCCGGCTCTGACTGACCGCGTTACTCCCACAGGTGAGCGGGCGGGACGGCCCTTCTCC
TCCGGGCTGTAATTAGCGCTTGGTTTAATGACGGCTCGTTTCTTTTCTGTGGCTGCGTGAAAGC
CTTAAAGGGCTCCGGGAGGGCCCTTTGTGCGGGGGGGAGCGGCTCGGGGGGTGCGTGCGTGTGT
GTGTGCGTGGGGAGCGCCGCGTGCGGCCCGCGCTGCCCGGCGGCTGTGAGCGCTGCGGGCGCGG
CGCGGGGCTTTGTGCGCTCCGCGTGTGCGCGAGGGGAGCGCGGCCGGGGGCGGTGCCCCGCGGT
GCGGGGGGGCTGCGAGGGGAACAAAGGCTGCGTGCGGGGTGTGTGCGTGGGGGGGTGAGCAGGG
GGTGTGGGCGCGGCGGTCGGGCTGTAACCCCCCCCTGCACCCCCCTCCCCGAGTTGCTGAGCAC
GGCCCGGCTTCGGGTGCGGGGCTCCGTGCGGGGCGTGGCGCGGGGCTCGCCGTGCCGGGCGGGG
GGTGGCGGCAGGTGGGGGTGCCGGGCGGGGCGGGGCCGCCTCGGGCCGGGGAGGGCTCGGGGGA
GGGGCGCGGCGGCCCCGGAGCGCCGGCGGCTGTCGAGGCGCGGCGAGCCGCAGCCATTGCCTTT
TATGGTAATCGTGCGAGAGGGCGCAGGGACTTCCTTTGTCCCAAATCTGGCGGAGCCGAAATCT
GGGAGGCGCCGCCGCACCCCCTCTAGCGGGCGCGGGCGAAGCGGTGCGGCGCCGGCAGGAAGGA
AATGGGCGGGGAGGGCCTTCGTGCGTCGCCGCGCCGCCGTCCCCTTCTCCATCTCCAGCCTCGG
GGCTGCCGCAGGGGGACGGCTGCCTTCGGGGGGGACGGGGCAGGGCGGGGTTCGGCTTCTGGCG
TGTGACCGGCGGCTCTAGAGCCTCTGCTAACCATGTTCATGCCTTCTTCTTTTTCCTACAGCTC
CTGGGCAACGTGCTGGTTGTTGTGCTGTCTCATCATTTTGGCAAAGAATTCGCTAGCGGATCCG
GCCGCCTCGGCCACCGGTCGCCACCATCGCCACCATGACCAAATCATACAGCGAGAGCGGGCTG
ATGGGCGAGCCTCAGCCCCAAGGTCCCCCAAGCTGGACAGATGAGTGTCTCAGTTCTCAGGACG
AGGAACACGAGGCAGACAAGAAAGAGGACGAGCTTGAAGCCATGAATGCAGAGGAGGACTCTCT
GAGAAACGGGGGAGAGGAGGAGGAGGAAGATGAGGATCTAGAGGAAGAGGAGGAAGAAGAAGAG
GAGGAGGAGGATCAAAAGCCCAAGAGACGGGGTCCCAAAAAGAAAAAGATGACCAAGGCGCGCC

TAGAACGTTTTAAATTAAGGCGCATGAAGGCCAACGCCCGCGAGCGGAACCGCATGCACGGGCT
GAACGCGGCGCTGGACAACCTGCGCAAGGTGGTACCTTGCTACTCCAAGACCCAGAAACTGTCT
AAAATAGAGACACTGCGCTTGGCCAAGAACTACATCTGGGCTCTGTCAGAGATCCTGCGCTCAG
GCAAAAGCCCTGATCTGGTCTCCTTCGTACAGACGCTCTGCAAAGGTTTGTCCCAGCCCACTAC
CAATTTGGTCGCCGGCTGCCTGCAGCTCAACCCTCGGACTTTCTTGCCTGAGCAGAACCCGGAC
ATGCCCCCGCATCTGCCAACCGCCAGCGCTTCCTTCCCGGTGCATCCCTACTCCTACCAGTCCC
CTGGACTGCCCAGCCCGCCCTACGGCACCATGGACAGCTCCCACGTCTTCCACGTCAAGCCGCC
GCCACACGCCTACAGCGCAGCTCTGGAGCCCTTCTTTGAAAGCCCCCTAACTGACTGCACCAGC
CCTTCCTTTGACGGACCCCTCAGCCCGCCGCTCAGCATCAATGGCAACTTCTCTTTCAAACACG
AACCATCCGCCGAGTTTGAAAAAAATTATGCCTTTACCATGCACTACCCTGCAGCGACGCTGGC
AGGGCCCCAAAGCCACGGATCAATCTTCTCTTCCGGTGCCGCTGCCCCTCGCTGCGAGATCCCC
ATAGACAACATTATGTCTTTCGATAGCCATTCGCATCATGAGCGAGTCATGAGTGCCCAGCTTA
ATGCCATCTTTCACGATTAGGTTTAAACGCGGCCGCGCCCCTCTCCCTCCCCCCCCCCTAACGT
TACTGGCCGAAGCCGCTTGGAATAAGGCCGGTGTGCGTTTGTCTATATGTTATTTTCCACCATA
TTGCCGTCTTTTGGCAATGTGAGGGCCCGGAAACCTGGCCCTGTCTTCTTGACGAGCATTCCTA
GGGGTCTTTCCCCTCTCGCCAAAGGAATGCAAGGTCTGTTGAATGTCGTGAAGGAAGCAGTTCC
TCTGGAAGCTTCTTGAAGACAAACAACGTCTGTAGCGACCCTTTGCAGGCAGCGGAACCCCCCA
CCTGGCGACAGGTGCCTCTGCGGCCAAAAGCCACGTGTATAAGATACACCTGCAAAGGCGGCAC
AACCCCAGTGCCACGTTGTGAGTTGGATAGTTGTGGAAAGAGTCAAATGGCTCTCCTCAAGCGT
ATTCAACAAGGGGCTGAAGGATGCCCAGAAGGTACCCCATTGTATGGGATCTGATCTGGGGCCT
CGGTGCACATGCTTTACATGTGTTTAGTCGAGGTTAAAAAAACGTCTAGGCCCCCCGAACCACG
GGGACGTGGTTTTCCTTTGAAAAACACGATGATAATATGGCCACAACCATGGTGAGCAAGGGCG
AGGAGCTGTTCACCGGGGTGGTGCCCATCCTGGTCGAGCTGGACGGCGACGTAAACGGCCACAA
GTTCAGCGTGTCCGGCGAGGGCGAGGGCGATGCCACCTACGGCAAGCTGACCCTGAAGTTCATC
TGCACCACCGGCAAGCTGCCCGTGCCCTGGCCCACCCTCGTGACCACCCTGACCTACGGCGTGC
AGTGCTTCAGCCGCTACCCCGACCACATGAAGCAGCACGACTTCTTCAAGTCCGCCATGCCCGA
AGGCTACGTCCAGGAGCGCACCATCTTCTTCAAGGACGACGGCAACTACAAGACCCGCGCCGAG
GTGAAGTTCGAGGGCGACACCCTGGTGAACCGCATCGAGCTGAAGGGCATCGACTTCAAGGAGG
ACGGCAACATCCTGGGGCACAAGCTGGAGTACAACTACAACAGCCACAACGTCTATATCATGGC
CGACAAGCAGAAGAACGGCATCAAGGTGAACTTCAAGATCCGCCACAACATCGAGGACGGCAGC
GTGCAGCTCGCCGACCACTACCAGCAGAACACCCCCATCGGCGACGGCCCCGTGCTGCTGCCCG
ACAACCACTACCTGAGCACCCAGTCCGCCCTGAGCAAAGACCCCAACGAGAAGCGCGATCACAT

GGTCCTGCTGGAGTTCGTGACCGCCGCCGGGATCACTCTCGGCATGGACGAGCTGTACAAGTAA
GTCGACAATCAACCTCTGGATTACAAAATTTGTGAAAGATTGACTGGTATTCTTAACTATGTTG
CTCCTTTTACGCTATGTGGATACGCTGCTTTAATGCCTTTGTATCATGCTATTGCTTCCCGTAT
GGCTTTCATTTTCTCCTCCTTGTATAAATCCTGGTTGCTGTCTCTTTATGAGGAGTTGTGGCCC
GTTGTCAGGCAACGTGGCGTGGTGTGCACTGTGTTTGCTGACGCAACCCCCACTGGTTGGGGCA
TTGCCACCACCTGTCAGCTCCTTTCCGGGACTTTCGCTTTCCCCCTCCCTATTGCCACGGCGGA
ACTCATCGCCGCCTGCCTTGCCCGCTGCTGGACAGGGGCTCGGCTGTTGGGCACTGACAATTCC
GTGGTGTTGTCGGGGAAGCTGACGTCCTTTCCATGGCTGCTCGCCTGTGTTGCCACCTGGATTC
TGCGCGGGACGTCCTTCTGCTACGTCCCTTCGGCCCTCAATCCAGCGGACCTTCCTTCCCGCGG
CCTGCTGCCGGCTCTGCGGCCTCTTCCGCGTCTTCGCCTTCGCCCTCAGACGAGTCGGATCTCC
CTTTGGGCCGCCTCCCCGCCTGGAATTCGAGCTCGAGCTTGTTAACATCGATAAAATAAAAGAT
TTTATTTAGTCTCCAGAAAAAGGGGGGAATGAAAGACCCCACCTGTAGGTTTGGCAAGCTAGCT
TAAGTAACGCCATTTTGCAAGGCATGGAAAAATACATAACTGAGAATAGAGAAGTTCAGATCAA
GGTCAGGAACAGATGGAACAGCTGAATATGGGCCAAACAGGATATCTGTGGTAAGCAGTTCCTG
CCCCGGCTCAGGGCCAAGAACAGATGGAACAGCTGAATATGGGCCAAACAGGATATCTGTGGTA
AGCAGTTCCTGCCCCGGCTCAGGGCCAAGAACAGATGGTCCCCAGATGCGGTCCAGCCCTCAGC
AGTTTCTAGAGAACCATCAGATGTTTCCAGGGTGCCCCAAGGACCTGAAATGACCCTGTGCCTT
ATTTGAACTAACCAATCAGTTCGCTTCTCGCTTCTGTTCGCGCGCTTCTGCTCCCCGAGCTCAA
TAAAAGAGCCCACAACCCCTCACTCGGGGCGCCAGTCCTCCGATTGACTGAGTCGCCCGGGTAC
CCGTGTATCCAATAAACCCTCTTGCAGTTGCATCCGACTTGTGGTCTCGCTGTTCCTTGGGAGG
GTCTCCTCTGAGTGATTGACTACCCGTCAGCGGGGGTCTTTCATTTCCGACTTGTGGTCTCGCT
GCCTTGGGAGGGTCTCCTCTGAGTGATTGACTACCCGTCAGCGGGGGTCTTCACATGCAGCATG
TATCAAAATTAATTTGGTTTTTTTTCTTAAGTATTTACATTAAATGGCCATAGTTGCATTAATG
AATCGGCCAACGCGCGGGGAGAGGCGGTTTGCGTATTGGCGCTCTTCCGCTTCCTCGCTCACTG
ACTCGCTGCGCTCGGTCGTTCGGCTGCGGCGAGCGGTATCAGCTCACTCAAAGGCGGTAATACG
GTTATCCACAGAATCAGGGGATAACGCAGGAAAGAACATGTGAGCAAAAGGCCAGCAAAAGGCC
AGGAACCGTAAAAAGGCCGCGTTGCTGGCGTTTTTCCATAGGCTCCGCCCCCCTGACGAGCATC
ACAAAAATCGACGCTCAAGTCAGAGGTGGCGAAACCCGACAGGACTATAAAGATACCAGGCGTT
TCCCCCTGGAAGCTCCCTCGTGCGCTCTCCTGTTCCGACCCTGCCGCTTACCGGATACCTGTCC
GCCTTTCTCCCTTCGGGAAGCGTGGCGCTTTCTCATAGCTCACGCTGTAGGTATCTCAGTTCGG
TGTAGGTCGTTCGCTCCAAGCTGGGCTGTGTGCACGAACCCCCCGTTCAGCCCGACCGCTGCGC
CTTATCCGGTAACTATCGTCTTGAGTCCAACCCGGTAAGACACGACTTATCGCCACTGGCAGCA

GCCACTGGTAACAGGATTAGCAGAGCGAGGTATGTAGGCGGTGCTACAGAGTTCTTGAAGTGGT
GGCCTAACTACGGCTACACTAGAAGGACAGTATTTGGTATCTGCGCTCTGCTGAAGCCAGTTAC
CTTCGGAAAAAGAGTTGGTAGCTCTTGATCCGGCAAACAAACCACCGCTGGTAGCGGTGGTTTT
TTTGTTTGCAAGCAGCAGATTACGCGCAGAAAAAAAGGATCTCAAGAAGATCCTTTGATCTTTT
CTACGGGGTCTGACGCTCAGTGGAACGAAAACTCACGTTAAGGGATTTTGGTCATGAGATTATC
AAAAAGGATCTTCACCTAGATCCTTTTAAATTAAAAATGAAGTTTGCGGCCGGCCGCAAATCAA
TCTAAAGTATATATGAGTAAACTTGGTCTGACAGTTACCAATGCTTAATCAGTGAGGCACCTAT
CTCAGCGATCTGTCTATTTCGTTCATCCATAGTTGCCTGACTCCCCGTCGTGTAGATAACTACG
ATACGGGAGGGCTTACCATCTGGCCCCAGTGCTGCAATGATACCGCGAGACCCACGCTCACCGG
CTCCAGATTTATCAGCAATAAACCAGCCAGCCGGAAGGGCCGAGCGCAGAAGTGGTCCTGCAAC
TTTATCCGCCTCCATCCAGTCTATTAATTGTTGCCGGGAAGCTAGAGTAAGTAGTTCGCCAGTT
AATAGTTTGCGCAACGTTGTTGCCATTGCTACAGGCATCGTGGTGTCACGCTCGTCGTTTGGTA
TGGCTTCATTCAGCTCCGGTTCCCAACGATCAAGGCGAGTTACATGATCCCCCATGTTGTGCAA
AAAAGCGGTTAGCTCCTTCGGTCCTCCGATCGTTGTCAGAAGTAAGTTGGCCGCAGTGTTATCA
CTCATGGTTATGGCAGCACTGCATAATTCTCTTACTGTCATGCCATCCGTAAGATGCTTTTCTG
TGACTGGTGAGTACTCAACCAAGTCATTCTGAGAATAGTGTATGCGGCGACCGAGTTGCTCTTG
CCCGGCGTCAACACGGGATAATACCGCGCCACATAGCAGAACTTTAAAAGTGCTCATCATTGGA
AAACGTTCTTCGGGGCGAAAACTCTCAAGGATCTTACCGCTGTTGAGATCCAGTTCGATGTAAC
CCACTCGTGCACCCAACTGATCTTCAGCATCTTTTACTTTCACCAGCGTTTCTGGGTGAGCAAA
AACAGGAAGGCAAAATGCCGCAAAAAAGGGAATAAGGGCGACACGGAAATGTTGAATACTCATA
CTCTTCCTTTTTCAAT

## Claims

1. A recombinant adeno-associated virus (AAV) expression vector comprising a human GFAP promoter sequence operably linked to a nucleic acid encoding NeuroD1 for use in a method for decreasing neuroinflammation after a stroke in a subject in need thereof, wherein the decrease in neuroinflammation comprises a downregulation in at least one pro-inflammatory factor selected from the group consisting of interferon y, TNFα, interleukin 1β, and interleukin 6, and wherein the AAV expression vector is administered to the central nervous system of the subject.

2. The recombinant AAV expression vector for use of claim 1, wherein the decrease in neuroinflammation comprises a downregulation in interferon y, TNFα, interleukin 1β, and interleukin 6.

3. The recombinant AAV expression vector for use of claim 1, wherein the nucleic acid encoding NeuroD1 comprises:
(a) a nucleic acid sequence that is at least 80% identical to SEQ ID NO: 1 or SEQ ID NO: 3, or a complement thereof, or a fragment thereof; or
(b) a nucleic acid sequence encoding a protein which has 79% or greater identity to SEQ ID NO: 2 or SEQ ID NO: 4, or a functional fragment thereof.

4. The recombinant AAV expression vector for use of any one of claims 1-3, wherein the human GFAP promoter sequence comprises SEQ ID NO: 6.

5. The recombinant AAV expression vector for use of any one of claims 1-4, wherein the AAV expression vector is an AAV9 vector.

6. The recombinant AAV expression vector for use of any one of claims 1-5, wherein the stroke is an ischemic stroke.

7. The recombinant AAV expression vector for use of any one of claims 1-5, wherein the stroke is a hemorrhagic stroke.

8. The recombinant AAV expression vector for use of any one of claims 1-7, wherein the decrease in neuroinflammation is assessed by an assay selected from the group consisting of an electrophysiology assay, a blood flow assay, a tissue structure assay, a function assay, and a combination of any two or more thereof.

9. The recombinant AAV expression vector for use of any one of claims 1-7, wherein the decrease in neuroinflammation is assessed via an assay of blood flow selected from the group consisting of Near Infrared Spectroscopy and fMRI, or an assay of tissue structure selected from the group consisting of MRI, CAT scan, PET scan, and ultrasound.

10. The recombinant AAV expression vector for use of any one of claims 1-9, wherein the AAV expression vector is administered to the subject by injection into the central nervous system of the subject.

11. The recombinant AAV expression vector for use of claim 10, wherein the injection is a stereotactic injection.

12. The recombinant AAV expression vector for use of any one of claims 1-11, wherein the AAV expression vector is administered to the subject at a concentration of 10¹⁰ to 10¹⁴ AAV particles/ml.

## Patentansprüche

1. Rekombinanter Expressionsvektor eines Adeno-assoziierten Virus (AAV), umfassend eine humane GFAP-Promotorsequenz, die funktionsfähig mit einer Nukleinsäure verbunden ist, die für NeuroD1 kodiert, zur Verwendung in einem Verfahren zum Verringern von Neuroinflammation nach einem Schlaganfall bei einem Subjekt, das dies benötigt, wobei die Verringerung der Neuroinflammation eine Herunterregulierung von mindestens einem pro-inflammatorischen Faktor umfasst, der aus der Gruppe ausgewählt ist, die aus Interferon γ, TNFα, Interleukin 1β und Interleukin 6 besteht, und wobei der AAV-Expressionsvektor an das Zentralnervensystem des Subjekts verabreicht wird.

2. Rekombinanter AAV-Expressionsvektor zur Verwendung nach Anspruch 1, wobei die Verringerung der Neuroinflammation eine Herunterregulierung von Interferon γ, TNFα, Interleukin 1β und Interleukin 6 umfasst.

3. Rekombinanter AAV-Expressionsvektor zur Verwendung nach Anspruch 1, wobei die Nukleinsäure, die für NeuroD1 kodiert, Folgendes umfasst:
(a) eine Nukleinsäuresequenz, die zu mindestens 80 % mit SEQ ID NO:1 oder SEQ ID NO:3 identisch ist, oder ein Komplement davon oder ein Fragment davon; oder
(b) eine Nukleinsäuresequenz, die ein Protein kodiert, das eine Identität von 79 % oder mehr mit SEQ ID NO:2 oder SEQ ID NO:4 aufweist, oder ein funktionelles Fragment davon.

4. Rekombinanter AAV-Expressionsvektor zur Verwendung nach einem der Ansprüche 1-3, wobei die humane GFAP-Promotorsequenz SEQ ID NO:6 umfasst.

5. Rekombinanter AAV-Expressionsvektor zur Verwendung nach einem der Ansprüche 1-4, wobei der AAV-Expressionsvektor ein AAV9-Vektor ist.

6. Rekombinanter AAV-Expressionsvektor zur Verwendung nach einem der Ansprüche 1-5, wobei der Schlaganfall ein ischämischer Schlaganfall ist.

7. Rekombinanter AAV-Expressionsvektor zur Verwendung nach einem der Ansprüche 1-5, wobei der Schlaganfall ein hämorrhagischer Schlaganfall ist.

8. Rekombinanter AAV-Expressionsvektor zur Verwendung nach einem der Ansprüche 1-7, wobei die Verringerung der Neuroinflammation durch einen Assay beurteilt wird, der aus der Gruppe ausgewählt ist, die aus einem elektrophysiologischen Assay, einem Blutflussassay, einem Gewebestrukturassay, einem Funktionsassay und einer Kombination von beliebigen zwei oder mehr davon besteht.

9. Rekombinanter AAV-Expressionsvektor zur Verwendung nach einem der Ansprüche 1-7, wobei die Verringerung der Neuroinflammation über einen Assay des Blutflusses, ausgewählt aus der Gruppe bestehend aus Nahinfrarotspektroskopie und fMRT, oder einen Assay der Gewebestruktur, ausgewählt aus der Gruppe bestehend aus MRT, CAT-Scan, PET-Scan und Ultraschall, beurteilt wird.

10. Rekombinanter AAV-Expressionsvektor zur Verwendung nach einem der Ansprüche 1-9, wobei der AAV-Expressionsvektor dem Subjekt durch Injektion in das Zentralnervensystem des Subjekts verabreicht wird.

11. Rekombinanter AAV-Expressionsvektor zur Verwendung nach Anspruch 10, wobei die Injektion eine stereotaktische Injektion ist.

12. Rekombinanter AAV-Expressionsvektor zur Verwendung nach einem der Ansprüche 1-11, wobei der AAV-Expressionsvektor dem Subjekt in einer Konzentration von 10¹⁰ bis 10¹⁴ AAV-Partikeln/ml verabreicht wird.

## Revendications

1. Vecteur d'expression de virus adéno-associé (AAV) recombinant comprenant une séquence du promoteur GFAP humain liée de manière opérationnelle à un acide nucléique codant pour NeuroD1 destiné à être utilisé dans un procédé de diminution de la neuroinflammation après un accident vasculaire cérébral chez un sujet en ayant besoin, ladite diminution de la neuroinflammation comprenant une régulation à la baisse d'au moins un facteur pro-inflammatoire choisi dans le groupe constitué de l'interféron y, du TNFα, de l'interleukine 1β et de l'interleukine 6, et ledit vecteur d'expression d'AAV étant administré au système nerveux central du sujet.

2. Vecteur d'expression d'AAV recombinant destiné à être utilisé selon la revendication 1, dans lequel la diminution de la neuroinflammation comprend une régulation à la baisse de l'interféron y, du TNFα, de l'interleukine 1β et de l'interleukine 6.

3. Vecteur d'expression d'AAV recombinant destiné à être utilisé selon la revendication 1, dans lequel l'acide nucléique codant pour NeuroD1 comprend :
(a) une séquence d'acide nucléique identique à au moins 80 % à SEQ ID N° : 1 ou SEQ ID N° : 3, ou un complément de celle-ci, ou un fragment de celle-ci ; ou
(b) une séquence d'acide nucléique codant pour une protéine comportant une identité de 79 % ou plus avec SEQ ID N° : 2 ou SEQ ID N° : 4, ou un fragment fonctionnel de celle-ci.

4. Vecteur d'expression d'AAV recombinant destiné à être utilisé selon l'une quelconque des revendications 1 à 3, dans lequel la séquence du promoteur GFAP humain comprend SEQ ID N° : 6.

5. Vecteur d'expression d'AAV recombinant destiné à être utilisé selon l'une quelconque des revendications 1 à 4, ledit vecteur d'expression d'AAV étant un vecteur AAV9.

6. Vecteur d'expression d'AAV recombinant destiné à être utilisé selon l'une quelconque des revendications 1 à 5, dans lequel l'accident vasculaire cérébral est un accident vasculaire cérébral ischémique.

7. Vecteur d'expression d'AAV recombinant destiné à être utilisé selon l'une quelconque des revendications 1 à 5, dans lequel l'accident vasculaire cérébral est un accident vasculaire cérébral hémorragique.

8. Vecteur d'expression d'AAV recombinant destiné à être utilisé selon l'une quelconque des revendications 1 à 7, dans lequel la diminution de la neuroinflammation est évaluée par un dosage choisi dans le groupe constitué par un dosage électrophysiologique, un dosage de flux sanguin, un dosage de structure tissulaire, un dosage de fonction et une combinaison de deux quelconques ou plus de ceux-ci.

9. Vecteur d'expression d'AAV recombinant destiné à être utilisé selon l'une quelconque des revendications 1 à 7, dans lequel la diminution de la neuroinflammation est évaluée par l'intermédiaire d'un dosage de flux sanguin choisi dans le groupe constitué de la spectroscopie dans le proche infrarouge et de l'IRMf, ou d'un dosage de structure tissulaire choisi dans le groupe constitué de l'IRM, de la tomodensitométrie, de la TEP et des ultrasons.

10. Vecteur d'expression d'AAV recombinant destiné à être utilisé selon l'une quelconque des revendications 1 à 9, ledit vecteur d'expression d'AAV étant administré au sujet par injection dans le système nerveux central du sujet.

11. Vecteur d'expression d'AAV recombinant destiné à être utilisé selon la revendication 10, dans lequel l'injection est une injection stéréotaxique.

12. Vecteur d'expression d'AAV recombinant destiné à être utilisé selon l'une quelconque des revendications 1 à 11, ledit vecteur d'expression d'AAV étant administré au sujet à une concentration de 10¹⁰ à 10¹⁴ particules d'AAV/ml.
